# EUROPEAN PATENT SPECIFICATION

(11) **EP 3 153 155 B1**
(45) Date of publication and mention of the grant of the patent: **03.03.2021**
(21) Application number: 16191732.3
(22) Date of filing: 30.09.2016
(51) Int. Cl.: A61K 9/00, A61K 9/06, A61K 47/36, A61K 31/00

(54) **REFILLABLE DRUG DELIVERY DEVICES AND METHODS OF USE THEREOF**
NACHFÜLLBARE WIRKSTOFFFREISETZUNGSVORRICHTUNGEN UND VERFAHREN ZUR VERWENDUNG DAVON
DISPOSITIFS RECHARGEABLES D'ADMINISTRATION DE MÉDICAMENTS ET LEURS PROCÉDÉS D'UTILISATION

(30) Priority: 08.10.2015 US 201514878578
(43) Date of publication of application: 12.04.2017
(73) Proprietor: President and Fellows of Harvard College, Cambridge, MA 02138 (US)
(72) Inventor: BRUDNO, Yevgeny, Somerville, MA 02143 (US); KEARNEY, Cathal J., Boston, MA 02130 (US); SILVA, Eduardo, Davis, CA 95618 (US); AIZENBERG, Michael, Cambridge, MA 02138 (US); KWEE, Brian, Cambridge, MA 02138 (US); DESAI, Rajiv, San Diego, CA 92131 (US); JOSHI, Neel S., Somerville, MA 02143 (US); MOONEY, David J., Sudbury, MA 01776 (US)
(74) Representative: Lock, Graham James

(56) References cited:
- WO-A1-2012/049624
- WO-A1-2012/156918
- WO-A1-2014/205126
- US-A1- 2009 192 583
- HAIFA AL-DUBAI ET AL: "Biocompatible medical implant materials with binding sites for a biodegradable drug-delivery system", NANOTECHNOLOGY, SCIENCE AND APPLICATIONS, 1 October 2011 (2011-10-01), page 87, XP055300987, DOI: 10.2147/NSA.S23605
- GUO HUA ET AL: "Functional alginate nanoparticles for efficient intracellular release of doxorubicin and hepatoma carcinoma cell targeting therapy", INTERNATIONAL JOURNAL OF PHARMACEUTICS, ELSEVIER, AMSTERDAM, NL, vol. 451, no. 1, 22 April 2013 (2013-04-22), pages 1-11, XP028556769, ISSN: 0378-5173, DOI: 10.1016/J.IJPHARM.2013.04.025

## Description

### BACKGROUND OF THE INVENTION

Drug-eluting polymer systems have proven useful in a variety of clinical settings, including prevention of restenosis with stenting, cancer treatment and enhancing wound healing. See, *e.g.,* Simard T, et al., (2014) The Canadian journal of cardiology 30(1): 34-45; Wessely R, (2010) Nature Reviews Cardiology 7(4):194-203; Iwamoto T, (2013) Biological & pharmaceutical bulletin 36(5):715-718; Attanasio S & Snell J, (2009) Cardiology Rev 17(3):115-120; Freedman S & Isner J, (2001) Journal of molecular and cellular cardiology 33(3):379-393; and Losordo D & Dimmeler S, (2004) Circulation 109(21):2487-2491. These systems benefit from tunable drug release kinetics, days or even weeks of continuous drug release, and local delivery which together provide spatiotemporal control over drug availability and can diminish drug toxicity. See Kearney C & Mooney D (2013) Nature materials 12(11):1004-1017. However, existing drug-eluting systems have a finite depot of drug and become unneeded when spent and, in the case of non-degrading systems, may need surgical removal.

For many therapeutic applications, an invasive procedure is needed to inject or implant a drug-eluting device, and these devices cannot be refilled or replaced without another invasive surgery.

Haifa Al-Dubai et al., (2011) Nanotechnology, Science and Applications 4: 87-94; describes a medical implant device comprising a PEG polymer coating, to which chitosan antibodies are anchored, and drug-containing chitosan nanoparticles, which can bind to the coating. Upon binding to the coating, the chitosan nanoparticles are degraded by enzymes in the surrounding body fluids to release the drug.

WO 2014/2015126 A1 discloses the selective delivery of a therapeutic/diagnostic agent to an organ or tissue by implant of a biocompatible solid support linked to a first binding agent and administration of a second binding agent linked to the therapeutic/diagnostic agent. The therapeutic/diagnostic agent, *e.g.,* radionuclide, accumulates at the targeted organ or tissue, so that the agent can be detected, *e.g.,* for imaging purposes.

Guo Hua et al., (2013) International Journal of Pharmaceutics 451(1): 1-11, describes a pH-sensitive system for the delivery of chemotherapy drugs into hepatoma cells. The system comprises nanoparticles of alginate conjugated to the hepatocyte-targeting ligand glycrrhetinic acid and alginate conjugated to doxorubicin (via a hydrazine linker). The nanoparticles are internalized by hepatocytes which allow for delivery of the chemotherapeutic drug, doxorubicin, to hepatoma cells.

US 2009/0192583 A1 discloses a stent comprising a layered coating and at least one therapeutic agent disposed within the coating, whereby the stent is placed at a treatment site and therapeutic agent is released from the coating in an ordered sequence and over a selected period of time at the treatment site.
There is currently no non-invasive technique to refill these systems once their payload is exhausted. Thus, there exists an onging and unmet need for a non-invasive method to refill a localized drug delivery device.

### SUMMARY OF THE INVENTION

The present invention is based, at least in part, on the development of a drug delivery system which permits refilling of a drug delivery device *in vivo* in a miminally invasive manner, by modifying the drug delivery device with molecular targets capable of recognizing and binding drug refills circulating in the body. The drug delivery system features a dual functionality drug refill, which not only permits a direct targeted delivery of a pharmaceutical composition from the drug refill to the drug delivery device, but also masks the potential toxicity of a pharmaceutical composition, such as a chemotherapeutic, until the pharmaceutical composition is delivered to the drug delivery device. The pharmaceutical composition will only be unmasked upon delivery into the drug delivery device where the target is separated from the pharmaceutical composition, thus eliminating any side effects or toxicity associated with the pharmaceutical composition at any undesired sites. Release of the pharmaceutical composition from the drug delivery device can be achieved in a controlled manner. Unlike the existing drug delivery systems which typically mediate delivery of a pharmaceutical composition within minutes, the drug delivery systems of the present invention provide a more sustained and controlled release of a pharmaceutical composition over a time scale of days, weeks, months or years.

Accordingly, in one aspect, the present invention provides, a system comprising a drug delivery device and a drug refill, wherein the drug delivery device comprises a carrier and a target recognition moiety and is suitable for implantation in a desirable location within a subject; wherein the drug refill comprises a pharmaceutical composition and a target; wherein the pharmaceutical composition is attached to the target via a cleavable linker capable of being cleaved within the subject; wherein the target and the target recognition moiety form a two-component binding pair; wherein the drug refill is mobile until the target on the drug refill binds to the target recognition moiety on the drug delivery device; wherein upon binding of the target to the target recognition moiety, the drug refill delivers the pharmaceutical composition directly to the drug delivery device, thereby refilling the drug delivery device; and wherein the pharmaceutical composition is released at the desirable location within the subject in a controlled manner over a time scale of days, weeks, months, or years, upon cleavage of the cleavable linker.

In some embodiments, the pharmaceutical composition comprises a small molecule or a biologic. In some embodiments, a biologic is selected from a group consisting of an antibody, a vaccine, a blood, or blood component, an allergenic, a gene therapy, a recombinant therapeutic protein, and a cell therapy. In some embodiments, a biologic may be composed of sugars, proteins, or nucleic acids or complex combinations of these substances, or may be living cells or tissues. In other embodiments, a biologic can be isolated from natural sources such as human, animal, or microorganism.

In some embodiments, the pharmaceutical composition has undesired toxicity and wherein the drug refill masks the toxicity of the pharmaceutical composition. In some embodiments, the drug refill masks the toxicity of the pharmaceutical composition by preventing the pharmaceutical composition from crossing the cell membrane. In other embodiments, the drug refill masks the toxicity of the pharmaceutical composition by preventing the pharmaceutical composition from binding to the biological target of the pharmaceutical composition. In some embodiments, at least 10%, 20%, 30%, 40%, 50%, 55%, 60%, 65%, 70%, 75%, 80%, 85%, 90%, 95% or 100% of the toxicity of the pharmaceutical composition is masked by the drug refill.

In some embodiments, the pharmaceutical composition is unmasked after delivery into the drug delivery device. In other embodiments, the pharmaceutical composition is unmasked by separating it from the target. In some embodiments, the target is separated from the pharmaceutical composition by cleaving a bond between the pharmaceutical composition and the cleavable linker, a bond within the cleavable linker, or a bond between the pharmaceutical composition and the target. In other embodiments, the bond between the pharmaceutical composition and the cleavable linker, the bond within the cleavable linker, or the bond between the pharmaceutical composition and the target is cleaved by enzyme degradation, hydrolysis or reduction of the bond. In some embodiments, the bond between the pharmaceutical composition and the cleavable linker comprises a bond selected from the group consisting of a hydrazine bond, an acetal bond, a ketal bond, an oxime bond, an imine bond, and an aminal bond.

In some embodiments, the pharmaceutical composition comprises a drug selected from the group consisting of an anti-cancer drug, a drug that promotes wound healing, a drug that promotes vascularization, a drug that treats or prevents infection, a drug that prevents restenosis, a drug that reduces macular degeneration, a drug that prevents immunological rejection, a drug that prevents thrombosis, and a drug that treats inflammation. In other embodiments, the anti-cancer drug comprises doxorubicin.

In some embodiments, the carrier comprises a polymer, a protein, a synthetic hydrogel, a biological hydrogel, an organogel, a ceramic, a composite, a metal, a wood, or a glass material. In other embodiments, the hydrogel is selected from the group consisting of collagen, alginate, polysaccharide, hyaluronic acid (HA), polyethylene glycol (PEG), poly(glycolide) (PGA), poly(L-lactide) (PLA), poly(lactide-co-glycolide) (PLGA), and poly lactic-coglycolic acid. In certain embodiments, the drug delivery device comprises a hydrogel. In some embodiments, the hydrogel comprises an alginate hydrogel.

In some embodiments, the desired location is a tissue within a subject or at a site away from the tissue. In other embodiments, the desired location is an organ within a subject or at a site away from the organ. In some embodiments, the desired location is an implant, a prothestic, or any tissue or device that can be introduced into the body or on the surface of the body.

In some embodiments, delivery of the drug refill to the drug delivery device allows for the pharmaceutical composition to be released in a controlled manner from the drug delivery device to the desired location within a subject over a time scale of days, weeks, months or years. In other embodiments, the pharmaceutical composition is released by cleaving a bond between the pharmaceutical composition and the cleavable linker, a bond within the cleavable linker, or a bond between the pharmaceutical composition and the target. In other embodiments, the bond between the pharmaceutical composition and the cleavable linker, the bond within the cleavable linker, or the bond between the pharmaceutical composition and the target is cleaved by enzyme degradation, hydrolysis or reduction of the bond. In yet another embodiment, the pharmaceutical composition is not released from the drug refill to the desired location within a subject. In some embodiments, the bond between the pharmaceutical composition and the cleavable linker comprises a bond selected from the group consisting of a hydrazine bond, an acetal bond, a ketal bond, an oxime bond, an imine bond, and an aminal bond.

In some embodiments, the target comprises a bioorthogonal functional group and the target recognition moiety comprises a complementary functional group, wherein the bioorthogonal functional group is capable of chemically reacting with the complementary functional group to form a covalent bond. In some embodiments, the bioorthogonal functional group is capable of reacting by click chemistry with the complementary functional group. In some embodiments, the bioorthogonal functional group comprises an alkyne and the complementary functional group comprises an azide, or the bioorthogonal functional group comprises an azide and the complementary functional group comprises an alkyne. In other embodiments, the alkyne comprises a cyclooctyne. In yet another embodiment, the cyclooctyne comprises dibenzocyclooctyne (DBCO). In some embodiments, the bioorthogonal functional group comprises an alkene and the complementary functional group comprises a tetrazine (Tz), or the bioorthogonal functional group comprises a tetrazine (Tz) and the complementary functional group comprises an alkene. In other embodiments, the alkene comprises a cyclooctene. In yet another embodiment, the cyclooctene comprises transcyclooctene (TCO).

In some embodiments, the target on the drug refill and the target recognition moiety on the drug delivery device comprise a nucleic acid. In some embodiments, the target comprises a nucleic acid sequence that is complementary to the nucleic acid sequence in the target recognition moiety. In other embodiments, the nucleic acid comprises DNA, RNA, modified DNA, or modified RNA.

In some embodiments, the target comprises biotin and the target recognition moiety comprises avidin or streptavidin. In other embodiments, the target comprises avidin or streptavidin and the target recognition moiety comprises biotin.

In some embodiments, the drug delivery system comprises at least two drug delivery devices. In some embodiments, the drug delivery devices are located at the same desired location within a subject. In other embodiments, the drug delivery devices are located at different desired locations within a subject. In some embodiments, the drug delivery system comprises at least two drug refills. In some embodiments, each drug refill comprises a different pharmaceutical composition and a different target. In other embodiments, each drug delivery device comprises a different target recognition moiety. In another embodiment, each drug refill binds to a different drug delivery device.

One aspect of the present invention provides a system comprising a drug delivery device and a drug refill, wherein the drug delivery device comprises a carrier and a target recognition moiety and is suitable for implantation in a desirable location within a subject; wherein the drug refill comprises a pharmaceutical composition and a target; wherein the pharmaceutical composition is attached to the target via a cleavable linker, wherein the pharmaceutical composition has undesired toxicity and the drug refill masks the toxicity of the pharmaceutical composition, wherein the target and the target recognition moiety form a two-component binding pair; wherein the drug refill is mobile until the target on the drug refill binds to the target recognition moiety on the drug delivery device, and wherein upon binding of the target to the target recognition moiety, the drug refill delivers the pharmaceutical composition directly to the drug delivery device, wherein the pharmaceutical composition is released in a controlled manner from the drug delivery device to the desirable location within the subject.

In some embodiments, the pharmaceutical composition comprises a small molecule or a biologic. In some embodiments, a biologic is selected from a group consisting of an antibody, a vaccine, a blood, or blood component, an allergenic, a gene therapy, a recombinant therapeutic protein, and a cell therapy. In some embodiments, a biologic may be composed of sugars, proteins, or nucleic acids or complex combinations of these substances, or may be living cells or tissues. In other embodiments, a biologic can be isolated from natural sources such as human, animal, or microorganism.

In some embodiments, the pharmaceutical composition has undesired toxicity and wherein the drug refill masks the toxicity of the pharmaceutical composition. In some embodiments, the drug refill masks the toxicity of the pharmaceutical composition by preventing the pharmaceutical composition from crossing the cell membrane. In other embodiments, the drug refill masks the toxicity of the pharmaceutical composition by preventing the pharmaceutical composition from binding to the biological target of the pharmaceutical composition. In some embodiments, at least 10%, 20%, 30%, 40%, 50%, 55%, 60%, 65%, 70%, 75%, 80%, 85%, 90%, 95% or 100% of the toxicity of the pharmaceutical composition is masked by the drug refill.

In some embodiments, the pharmaceutical composition is unmasked after delivery into the drug delivery device. In other embodiments, the pharmaceutical composition is unmasked by separating it from the target. In some embodiments, the target is separated from the pharmaceutical composition by cleaving a bond between the pharmaceutical composition and the cleavable linker, a bond within the cleavable linker, or a bond between the pharmaceutical composition and the target. In other embodiments, the bond between the pharmaceutical composition and the cleavable linker, the bond within the cleavable linker, or the bond between the pharmaceutical composition and the target is cleaved by enzyme degradation, hydrolysis or reduction of the bond. In some embodiments, the bond between the pharmaceutical composition and the cleavable linker comprises a bond selected from the group consisting of a hydrazine bond, an acetal bond, a ketal bond, an oxime bond, an imine bond, and an aminal bond.

In some embodiments, the pharmaceutical composition comprises a drug selected from the group consisting of an anti-cancer drug, a drug that promotes wound healing, a drug that promotes vascularization, a drug that treats or prevents infection, a drug that prevents restenosis, a drug that reduces macular degeneration, a drug that prevents immunological rejection, a drug that prevents thrombosis, and a drug that treats inflammation. In other embodiments, the anti-cancer drug comprises doxorubicin.

In some embodiments, the drug delivery device comprises a carrier selected from a group consisting of a polymer, a protein, a synthetic hydrogel, a biological hydrogel, an organogel, a ceramic, a composite, a metal, a wood, or a glass material. In other embodiments, the hydrogel is selected from the group consisting of collagen, alginate, polysaccharide, hyaluronic acid (HA), polyethylene glycol (PEG), poly(glycolide) (PGA), poly(L-lactide) (PLA), poly(lactide-co-glycolide) (PLGA), and poly lactic-coglycolic acid. In certain embodiments, the drug delivery device comprises a hydrogel. In some embodiments, the hydrogel comprises an alginate hydrogel.

In some embodiments, the desired location is a tissue within a subject or at a site away from the tissue. In other embodiments, the desired location is an organ within a subject or at a site away from the organ. In some embodiments, the desired location is an implant, a prothestic, or any tissue or device that can be introduced into the body or on the surface of the body.

In some embodiments, delivery of drug refill to the drug delivery device allows for the pharmaceutical composition to be released in a controlled manner from the drug delivery device to the desired location within a subject over a time scale of days, weeks, months or years. In other embodiments, the pharmaceutical composition is released by cleaving a bond between the pharmaceutical composition and the cleavable linker, a bond within the cleavable linker, or a bond between the pharmaceutical composition and the target. In other embodiments, the bond between the pharmaceutical composition and the cleavable linker, the bond within the cleavable linker, or the bond between the pharmaceutical composition and the target is cleaved by enzyme degradation, hydrolysis or reduction of the bond. In yet another embodiment, the pharmaceutical composition is not released from the drug refill to the desired location within a subject. In some embodiments, the bond between the pharmaceutical composition and the cleavable linker comprises a bond selected from the group consisting of a hydrazine bond, an acetal bond, a ketal bond, an oxime bond, an imine bond, and an aminal bond.

In some embodiments, the target comprises a bioorthogonal functional group and the target recognition moiety comprises a complementary functional group, wherein the bioorthogonal functional group is capable of chemically reacting with the complementary functional group to form a covalent bond. In some embodiments, the bioorthogonal functional group is capable of reacting by click chemistry with the complementary functional group. In some embodiments, the bioorthogonal functional group comprises an alkyne and the complementary functional group comprises an azide, or the bioorthogonal functional group comprises an azide and the complementary functional group comprises an alkyne. In other embodiments, the alkyne comprises a cyclooctyne. In yet another embodiment, the cyclooctyne comprises dibenzocyclooctyne (DBCO). In some embodiments, the bioorthogonal functional group comprises an alkene and the complementary functional group comprises a tetrazine (Tz), or the bioorthogonal functional group comprises a tetrazine (Tz) and the complementary functional group comprises an alkene. In other embodiments, the alkene comprises a cyclooctene. In yet another embodiment, the cyclooctene comprises transcyclooctene (TCO).

In some embodiments, the target on the drug refill and the target recognition moiety on the drug delivery device comprise a nucleic acid. In some embodiments, the target comprises a nucleic acid sequence that is complementary to the nucleic acid sequence in the target recognition moiety. In other embodiments, the nucleic acid comprises DNA, RNA, modified DNA, or modified RNA.

In some embodiments, the target comprises biotin and the target recognition moiety comprises avidin or streptavidin. In other embodiments, the target comprises avidin or streptavidin and the target recognition moiety comprises biotin.

In some embodiments, the drug delivery system comprises at least two drug delivery devices. In some embodiments, the drug delivery devices are located at the same desired location within a subject. In other embodiments, the drug delivery devices are located at different desired locations within a subject. In some embodiments, the drug delivery system comprises at least two drug refills. In some embodiments, each drug refill comprises a different pharmaceutical composition and a different target. In other embodiments, each drug delivery device comprises a different target recognition moiety. In another embodiment, each drug refill binds to a different drug delivery device.

One aspect of the present invention provides a stationary drug delivery device comprising a pharmaceutical composition, a target recognition moiety and a carrier, wherein the target recognition moiety is capable of binding to a target on a drug refill, wherein the drug refill comprises a pharmaceutical composition and a target, wherein the pharmaceutical composition is attached to the target via a cleavable linker capable of being cleaved within a subject, wherein the pharmaceutical composition is released at a desirable location within the subject in a controlled manner over a time scale of days, weeks, months, or years, upon cleavage of the cleavable linker, and wherein the drug delivery device is suitable for implantation in a desired location within a subject.

In some embodiments, the carrier of the drug delivery device comprises a polymer, a protein, a synthetic hydrogel, a biological hydrogel, an organogel, a ceramic, a composite, a metal, a wood, or a glass material. In other embodiments, the hydrogel is selected from the group consisting of collagen, alginate, polysaccharide, hyaluronic acid (HA), polyethylene glycol (PEG), poly(glycolide) (PGA), poly(L-lactide) (PLA), poly(lactide-co-glycolide) (PLGA), and poly lactic-coglycolic acid. In certain embodiments, the drug delivery device comprises a hydrogel. In some embodiments, the hydrogel comprises an alginate hydrogel.

In some embodiments, the desired location is a tissue within a subject or at a site away from the tissue. In other embodiments, the desired location is an organ within a subject or at a site away from the organ. In some embodiments, the desired location is an implant, a prothestic, or any tissue or device that can be introduced into the body or on the surface of the body.

Another aspect of the present invention provides a drug refill comprising a pharmaceutical composition and a target, wherein the pharmaceutical composition is attached to the target via a cleavable linker, wherein the pharmaceutical composition has undesired toxicity and the drug refill masks the toxicity of the pharmaceutical composition, wherein the target is capable of binding to a target recognition moiety on a drug delivery device, wherein the drug refill is mobile until the target binds to the target recognition moiety on a drug delivery device, wherein upon binding of the target to the target recognition moiety, the drug refill delivers the pharmaceutical composition to the drug delivery device, the pharmaceutical composition is unmasked after delivery into the drug delivery device, and the pharmaceutical composition is released at a desirable location within the subject in a controlled manner over a time scale of days, weeks, months, or years, upon cleavage of the cleavable linker.

In some embodiments, the pharmaceutical composition comprises a small molecule or a biologic. In some embodiments, a biologic is selected from a group consisting of an antibody, a vaccine, a blood, or blood component, an allergenic, a gene therapy, a recombinant therapeutic protein, and a cell therapy. In some embodiments, a biologic may be composed of sugars, proteins, or nucleic acids or complex combinations of these substances, or may be living cells or tissues. In other embodiments, a biologic can be isolated from natural sources such as human, animal, or microorganism.

In some embodiments, the drug refill masks the toxicity of the pharmaceutical composition by preventing the pharmaceutical composition from crossing the cell membrane: In other embodiments, the drug refill masks the toxicity of the pharmaceutical composition by preventing the pharmaceutical composition from binding to the biological target of the pharmaceutical composition. In some embodiments, at least 10%, 20%, 30%, 40%, 50%, 55%, 60%, 65%, 70%, 75%, 80%, 85%, 90%, 95% or 100% of the toxicity of the pharmaceutical composition is masked by the drug refill.

In some embodiments, the pharmaceutical composition is unmasked after delivery into the drug delivery device. In other embodiments, the pharmaceutical composition is unmasked by separating it from the target. In some embodiments, the target is separated from the pharmaceutical composition by cleaving a bond between the pharmaceutical composition and the cleavable linker, a bond within the cleavable linker, or a bond between the pharmaceutical composition and the target. In other embodiments, the bond between the pharmaceutical composition and the cleavable linker, the bond within the cleavable linker, or the bond between the pharmaceutical composition and the target is cleaved by enzyme degradation, hydrolysis or reduction of the bond. In some embodiments, the bond between the pharmaceutical composition and the cleavable linker comprises a bond selected from the group consisting of a hydrazine bond, an acetal bond, a ketal bond, an oxime bond, an imine bond, and an aminal bond.

In some embodiments, the pharmaceutical composition comprises a drug selected from the group consisting of an anti-cancer drug, a drug that promotes wound healing, a drug that promotes vascularization, a drug that treats or prevents infection, a drug that prevents restenosis, a drug that reduces macular degeneration, a drug that prevents immunological rejection, a drug that prevents thrombosis, and a drug that treats inflammation. In other embodiments, the anti-cancer drug comprises doxorubicin.
In some embodiments, the pharmaceutical composition is released by cleaving a bond between the pharmaceutical composition and the cleavable linker, a bond within the cleavable linker, or a bond between the pharmaceutical composition and the target. In other embodiments, the bond between the pharmaceutical composition and the cleavable linker, the bond within the cleavable linker, or the bond between the pharmaceutical composition and the target is cleaved by enzyme degradation, hydrolysis or reduction of the bond. In yet another embodiment, the pharmaceutical composition is not released from the drug refill to the desired location within a subject. In some embodiments, the bond between the pharmaceutical composition and the cleavable linker comprises a bond selected from the group consisting of a hydrazine bond, a hydrazone bond, an acetal bond, a ketal bond, an oxime bond, an imine bond, and an aminal bond.

In some embodiments, the target comprises a bioorthogonal functional group and the target recognition moiety comprises a complementary functional group, wherein the bioorthogonal functional group is capable of chemically reacting with the complementary functional group to form a covalent bond. In some embodiments, the bioorthogonal functional group is capable of reacting by click chemistry with the complementary functional group. In some embodiments, the bioorthogonal functional group comprises an alkyne and the complementary functional group comprises an azide, or the bioorthogonal functional group comprises an azide and the complementary functional group comprises an alkyne. In other embodiments, the alkyne comprises a cyclooctyne. In yet another embodiment, the cyclooctyne comprises dibenzocyclooctyne (DBCO). In some embodiments, the bioorthogonal functional group comprises an alkene and the complementary functional group comprises a tetrazine (Tz), or the bioorthogonal functional group comprises a tetrazine (Tz) and the complementary functional group comprises an alkene. In other embodiments, the alkene comprises a cyclooctene. In yet another embodiment, the cyclooctene comprises transcyclooctene (TCO).

In some embodiments, the target on the drug refill and the target recognition moiety on the drug delivery device comprise a nucleic acid. In some embodiments, the target comprises a nucleic acid sequence that is complementary to the nucleic acid sequence in the target recognition moiety. In other embodiments, the nucleic acid comprises DNA, RNA, modified DNA, or modified RNA.

In some embodiments, the target comprises biotin and the target recognition moiety comprises avidin or streptavidin. In other embodiments, the target comprises avidin or streptavidin and the target recognition moiety comprises biotin.

In some embodiments, the drug delivery system comprises at least two drug delivery devices. In some embodiments, the drug delivery devices are located at the same desired location within a subject. In other embodiments, the drug delivery devices are located at different desired locations within a subject. In some embodiments, the drug delivery system comprises at least two drug refills. In some embodiments, each drug refill comprises a different pharmaceutical composition and a different target. In other embodiments, each drug delivery device comprises a different target recognition moiety. In another embodiment, each drug refill binds to a different drug delivery device.

In one aspect, the present invention provides a kit for drug delivery comprising a drug delivery device and a drug refill, wherein the drug delivery device comprises a carrier and a target recognition moiety and is suitable for implantation in a desired location within a subject; wherein the drug refill comprises a pharmaceutical composition and a target, wherein the pharmaceutical composition is attached to the target directly or via a cleavable linker, wherein the pharmaceutical composition has undesired toxicity and the drug refill masks the toxicity of the pharmaceutical composition, wherein the target and the target recognition moiety form a two-component binding pair; wherein the drug refill is mobile until the target on the drug refill binds to the target recognition moiety on the drug delivery device, and wherein upon binding of the target to the target recognition moiety, the drug refill delivers the pharmaceutical composition to the drug delivery device, the pharmaceutical composition is unmasked after delivery into the drug delivery device, and the pharmaceutical composition is released at a desirable location within the subject in a controlled manner over a time scale of days, weeks, months, or years, upon cleavage of the cleavable linker.

In some embodiments, the pharmaceutical composition comprises a small molecule or a biologic. In some embodiments, a biologic is selected from a group consisting of an antibody, a vaccine, a blood, or blood component, an allergenic, a gene therapy, a recombinant therapeutic protein, and a cell therapy. In some embodiments, a biologic may be composed of sugars, proteins, or nucleic acids or complex combinations of these substances, or may be living cells or tissues. In other embodiments, a biologic can be isolated from natural sources such as human, animal, or microorganism.

In some embodiments, the drug refill masks the toxicity of the pharmaceutical composition by preventing the pharmaceutical composition from crossing the cell membrane. In other embodiments, the drug refill masks the toxicity of the pharmaceutical composition by preventing the pharmaceutical composition from binding to the biological target of the pharmaceutical composition. In some embodiments, at least 10%, 20%, 30%, 40%, 50%, 55%, 60%, 65%, 70%, 75%, 80%, 85%, 90%, 95% or 100% of the toxicity of the pharmaceutical composition is masked by the drug refill.

In some embodiments, the pharmaceutical composition is unmasked after delivery into the drug delivery device. In other embodiments, the pharmaceutical composition is unmasked by separating it from the target. In some embodiments, the target is separated from the pharmaceutical composition by cleaving a bond between the pharmaceutical composition and the cleavable linker, a bond within the cleavable linker, or a bond between the pharmaceutical composition and the target. In other embodiments, the bond between the pharmaceutical composition and the cleavable linker, the bond within the cleavable linker, or the bond between the pharmaceutical composition and the target is cleaved by enzyme degradation, hydrolysis or reduction of the bond. In some embodiments, the bond between the pharmaceutical composition and the cleavable linker comprises a bond selected from the group consisting of a hydrazine bond, an acetal bond, a ketal bond, an oxime bond, an imine bond, and an aminal bond.

In some embodiments, the pharmaceutical composition comprises a drug selected from the group consisting of an anti-cancer drug, a drug that promotes wound healing, a drug that promotes vascularization, a drug that treats or prevents infection, a drug that prevents restenosis, a drug that reduces macular degeneration, a drug that prevents immunological rejection, a drug that prevents thrombosis, and a drug that treats inflammation. In other embodiments, the anti-cancer drug comprises doxorubicin.

In some embodiments, the drug delivery device comprises a carrier selected from a group consisting of a polymer, a protein, a synthetic hydrogel, a biological hydrogel, an organogel, a ceramic, a composite, a metal, a wood, or a glass material. In other embodiments, the hydrogel is selected from the group consisting of collagen, alginate, polysaccharide, hyaluronic acid (HA), polyethylene glycol (PEG), poly(glycolide) (PGA), poly(L-lactide) (PLA), poly(lactide-co-glycolide) (PLGA), and poly lactic-coglycolic acid. In certain embodiments, the drug delivery device comprises a hydrogel. In some embodiments, the hydrogel comprises an alginate hydrogel.

In some embodiments, the desired location is a tissue within a subject or at a site away from the tissue. In other embodiments, the desired location is an organ within a subject or at a site away from the organ. In some embodiments, the desired location is an implant, a prothestic, or any tissue or device that can be introduced into the body or on the surface of the body.

In some embodiments, the pharmaceutical composition is released by cleaving a bond between the pharmaceutical composition and the cleavable linker, a bond within the cleavable linker, or a bond between the pharmaceutical composition and the target. In other embodiments, the bond between the pharmaceutical composition and the cleavable linker, the bond within the cleavable linker, or the bond between the pharmaceutical composition and the target is cleaved by enzyme degradation, hydrolysis or reduction of the bond. In yet another embodiment, the pharmaceutical composition is not released from the drug refill to the desired location within a subject. In some embodiments, the bond between the pharmaceutical composition and the cleavable linker comprises a bond selected from the group consisting of a hydrazine bond, an acetal bond, a ketal bond, an oxime bond, an imine bond, and an aminal bond.

In some embodiments, the target comprises a bioorthogonal functional group and the target recognition moiety comprises a complementary functional group, wherein the bioorthogonal functional group is capable of chemically reacting with the complementary functional group to form a covalent bond. In some embodiments, the bioorthogonal functional group is capable of reacting by click chemistry with the complementary functional group. In some embodiments, the bioorthogonal functional group comprises an alkyne and the complementary functional group comprises an azide, or the bioorthogonal functional group comprises an azide and the complementary functional group comprises an alkyne. In other embodiments, the alkyne comprises a cyclooctyne. In yet another embodiment, the cyclooctyne comprises dibenzocyclooctyne (DBCO). In some embodiments, the bioorthogonal functional group comprises an alkene and the complementary functional group comprises a tetrazine (Tz), or the bioorthogonal functional group comprises a tetrazine (Tz) and the complementary functional group comprises an alkene. In other embodiments, the alkene comprises a cyclooctene. In yet another embodiment, the cyclooctene comprises transcyclooctene (TCO).

In some embodiments, the target on the drug refill and the target recognition moiety on the drug delivery device comprise a nucleic acid. In some embodiments, the target comprises a nucleic acid sequence that is complementary to the nucleic acid sequence in the target recognition moiety. In other embodiments, the nucleic acid comprises DNA, RNA, modified DNA, or modified RNA.

In some embodiments, the target comprises biotin and the target recognition moiety comprises avidin or streptavidin. In other embodiments, the target comprises avidin or streptavidin and the target recognition moiety comprises biotin.

In some embodiments, the drug delivery system comprises at least two drug delivery devices. In some embodiments, the drug delivery devices are located at the same desired location within a subject. In other embodiments, the drug delivery devices are located at different desired locations within a subject. In some embodiments, the drug delivery system comprises at least two drug refills. In some embodiments, each drug refill comprises a different pharmaceutical composition and a different target. In other embodiments, each drug delivery device comprises a different target recognition moiety. In another embodiment, each drug refill binds to a different drug delivery device.

In another aspect, the present invention provides the system as described herein for use in maintaining or reducing the size of a tumor in a subject in need thereof, wherein: i) the drug delivery device of the present invention is administered to a desired location within the subject, wherein the pharmaceutical composition comprises an anti-cancer drug; ii) the drug refill of the present invention is subsequently administered to the subject; iii) the target on the drug refill is allowed to bind to the target recognition moiety on the drug delivery device, to thereby deliver the pharmaceutical composition directly to the drug delivery device; iv) the pharmaceutical composition is allowed to be released from the drug delivery device to the desired location within the subject; v) optionally, steps ii)-iv); are repeated to thereby maintain or reduce the size of the tumor in the subject.

In some embodiment, the desired location is a tumor site or a site away from the tumor site within the subject. In some embodiments, the drug refill is administered orally, buccally, sublingually, rectally, intravenously, intra-arterially, intraosseously, intra-muscularly, intracerebrally, intracerebroventricularly, intrathecally, subcutaneously, intraperitoneally, intraocularly, intranasally, transdermally, epidurally, intracranially, percutaneously, intravaginaly, intrauterineally, intravitreally, transmucosally, or via injection, via aerosol-based delivery, or via implantation. In some embodiments, the anti-cancer drug comprises doxorubicin. In other embodiments, the tumor comprises a solid tumor or a hematological tumor.

In some embodiments, the system for use of the present invention comprises at least two drug delivery devices. In some embodiments, the drug delivery devices are located at the same desired location within a subject. In other embodiments, the drug delivery devices are located at different desired locations within a subject. In some embodiments, the drug delivery system comprises at least two drug refills. In some embodiments, each drug refill comprises a different pharmaceutical composition and a different target. In other embodiments, each drug delivery device comprises a different target recognition moiety. In another embodiment, each drug refill binds to a different drug delivery device.

Described herein is a method of refilling a drug delivery device *in vivo,* comprising the steps of i) administering the drug delivery device to a subject, wherein the drug delivery device comprises a target recognition moiety, wherein the drug delivery device is suitable for implantation in a desired location within a subject; and ii) subsequently administering to the subject a drug refill comprising a pharmaceutical composition and a target, wherein the target and the target recognition moiety form a two-component binding pair; wherein the drug refill is mobile until the target on the drug refill binds to the target recognition moiety on the drug delivery device, and wherein upon binding of the target to the target recognition moiety, the drug refill delivers the pharmaceutical composition directly to the drug delivery device; thereby refilling the drug delivery device.

The drug delivery device may be implanted in a desired location within a subject. The desired location may be a tissue within a subject or at a site away from the tissue. The desired location may be an organ within a subject or at a site away from the organ. The desired location may be an implant, a prothestic, or any tissue or device that can be introduced into the body or on the surface of the body.

Described herein is a method of reducing cancer progression in a subject in need thereof, comprising the steps of: i) administering the drug delivery device of the present invetion to a desired location within the subject, wherein the pharmaceutical composition comprises an anti-cancer drug; ii) subsequently administering the drug refill of the present invention to the subject orally, intraperitoneally, intravenously, or intra-arterially; iii) allowing the target on the drug refill to bind to the target recognition moiety on the drug delivery device, thereby delivering the pharmaceutical composition directly to the drug delivery device; iv) allowing the pharmaceutical composition to be released from the drug delivery device to the desired location within the subject; v) optionally, repeating steps ii-iv); thereby reducing cancer progression in the subject.

The desired location may be a tumor site or a site away from the tumor site within the subject. The drug refill may be administered orally, buccally, sublingually, rectally, intravenously, intra-arterially, intraosseously, intra-muscularly, intracerebrally, intracerebroventricularly, intrathecally, subcutaneously, intraperitoneally, intraocularly, intranasally, transdermally, epidurally, intracranially, percutaneously, intravaginaly, intrauterineally, intravitreally, transmucosally, or via injection, via aerosol-based delivery, or via implantation. The anti-cancer drug may comprise doxorubicin.

The tumor comprises a solid tumor or a hematological tumor.

Described herein is a method of preventing tumor recurrence in a subject in need thereof, comprising the steps of: i) administering the drug delivery device of the present invetion to a desired location within the subject, wherein the pharmaceutical composition comprises an anti-cancer drug; ii) subsequently administering the drug refill of the present invention to the subject orally, intraperitoneally, intravenously, or intra-arterially; iii) allowing the target on the drug refill to bind to the target recognition moiety on the drug delivery device, thereby delivering the pharmaceutical composition directly to the drug delivery device; iv) allowing the pharmaceutical composition to be released from the drug delivery device to the desired location within the subject; v) optionally, repeating steps ii-iv); thereby preventing tumor recurrence in the subject.

The desired location may be a tumor site or a site away from the tumor site within the subject. The drug refill may be administered orally, buccally, sublingually, rectally, intravenously, intra-arterially, intraosseously, intra-muscularly, intracerebrally, intracerebroventricularly, intrathecally, subcutaneously, intraperitoneally, intraocularly, intranasally, transdermally, epidurally, intracranially, percutaneously, intravaginaly, intrauterineally, intravitreally, transmucosally, or via injection, via aerosol-based delivery, or via implantation. In some embodiments, the anti-cancer drug comprises doxorubicin.

The tumor may comprise a solid tumor or a hematological tumor.

Described herein is a method of promoting wound healing in a subject, comprising the steps of: i) administering the drug delivery device of the present invention to a desired location within the subject, wherein the pharmaceutical composition promotes angiogenesis and/or maturation or remodeling of an existing blood vessel; ii) subsequently administering the drug refill of the present invention to the subject; iii) allowing the target on the drug refill to bind to the target recognition moiety on the drug delivery device, thereby delivering the pharmaceutical composition directly to the drug delivery device; iv) allowing the pharmaceutical composition to be released from the drug delivery device to the desired location within the subject; v) optionally, repeating steps ii-iv); thereby promoting wound healing in the subject.

The drug refill may be administered orally, buccally, sublingually, rectally, intravenously, intra-arterially, intraosseously, intra-muscularly, intracerebrally, intracerebroventricularly, intrathecally, subcutaneously, intraperitoneally, intraocularly, intranasally, transdermally, epidurally, intracranially, percutaneously, intravaginaly, intrauterineally, intravitreally, transmucosally, or via injection, via aerosol-based delivery, or via implantation.

Described herein is a method of reducing or controlling inflammation in a subject in need thereof, comprising the steps of: i) administering the drug delivery device of the present invention to a desired location within the subject, wherein the pharmaceutical composition comprises an anti-inflammatory agent; ii) subsequently administering the drug refill of the present invention to the subject; iii) allowing the target on the drug refill to bind to the target recognition moiety on the drug delivery device, thereby delivering the pharmaceutical composition directly to the drug delivery device; iv) allowing the pharmaceutical composition to be released from the drug delivery device to the desired location within the subject; v) optionally, repeating steps ii-iv); thereby reducing or controlling inflammation in the subject.

The drug refill may be administered orally, buccally, sublingually, rectally, intravenously, intra-arterially, intraosseously, intra-muscularly, intracerebrally, intracerebroventricularly, intrathecally, subcutaneously, intraperitoneally, intraocularly, intranasally, transdermally, epidurally, intracranially, percutaneously, intravaginaly, intrauterineally, intravitreally, transmucosally, or via injection, via aerosol-based delivery, or via implantation.

In one aspect, the present invention provides the system as described herein for use in treating an eye disease in a subject in need thereof, wherein: i) the drug delivery device of the present invention is administered to a desired location within the subject, wherein the pharmaceutical composition treats said eye disease; ii) the drug refill of the present invention is subsequently administered to the subject; iii) the target on the drug refill is allowed to bind to the target recognition moiety on the drug delivery device to thereby deliver the pharmaceutical composition directly to the drug delivery device; iv) the pharmaceutical composition is allowed to be released from the drug delivery device to the desired location within the subject; v) optionally, steps ii)-iv) are repeated to thereby treating the eye disease in the subject.

In some embodiments, the desired location is the eye of the subject. In other embodiments, the drug refill is administered orally, buccally, sublingually, rectally, intravenously, intra-arterially, intraosseously, intra-muscularly, intracerebrally, intracerebroventricularly, intrathecally, subcutaneously, intraperitoneally, intraocularly, intranasally, transdermally, epidurally, intracranially, percutaneously, intravaginaly, intrauterineally, intravitreally, transmucosally, or via injection, via aerosol-based delivery, or via implantation.

Described herein is a method of treating arrhythmia in a subject in need thereof, comprising the steps of: i) administering the drug delivery device of the present invention to a desired location within the subject, wherein the pharmaceutical composition treats said arrhythmia; ii) subsequently administering the drug refill of the present invention to the subject; iii) allowing the target on the drug refill to bind to the target recognition moiety on the drug delivery device, thereby delivering the pharmaceutical composition directly to the drug delivery device; iv) allowing the pharmaceutical composition to be released from the drug delivery device to the desired location within the subject; v) optionally, repeating steps ii-iv); thereby treating arrhythmia in the subject.

The desired location may be the heart of the subject. The drug refill may be administered orally, buccally, sublingually, rectally, intravenously, intra-arterially, intraosseously, intra-muscularly, intracerebrally, intracerebroventricularly, intrathecally, subcutaneously, intraperitoneally, intraocularly, intranasally, transdermally, epidurally, intracranially, percutaneously, intravaginaly, intrauterineally, intravitreally, transmucosally, or via injection, via aerosol-based delivery, or via implantation.

Described herein is a method of evaluating patient medication adherence comprising administering a drug delivery device to a subject in need thereof, where the drug delivery device comprises a target recognition moiety and is suitable for implantation in a desired location within a subject; subsequently administering a drug refill comprising a pharmaceutical composition and a target to the subject, wherein the target comprises a detectable label linked thereto, wherein the target and the target recognition moiety form a two-component binding pair, and wherein the drug refill travels to and binds to the drug delivery device; detecting said label on said drug delivery device; and comparing the level of said label on said drug delivery device to the level of said label on said drug delivery device prior to administration of said drug refill; thereby evaluating patient medication adherence.

The present invention is illustrated by the following drawings and detailed description, which do not limit the scope of the invention described in the claims.

### BRIEF DESCRIPTION OF THE DRAWINGS

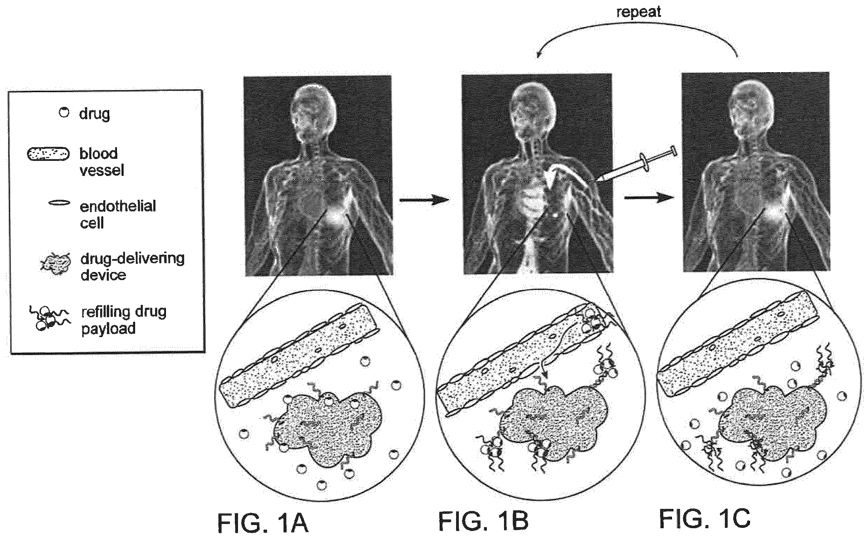

Figure 1A is a schematic for intravenous refilling of drug-delivering devices with therapeutic drugs payloads. A drug-delivering device is implanted into a target tissue site and releases active drug (circles) in a controlled, localized manner. Figure 1B is a schematic showing that intravenously infused drug payload homes to the device site and refills polymer with a fresh depot of drug. Figure 1C is a schematic that shows that IV infused drug cargo binds to gel, and is released over time. IV-mediated drug refilling can be repeated with the same or a different drug payload multiple times.

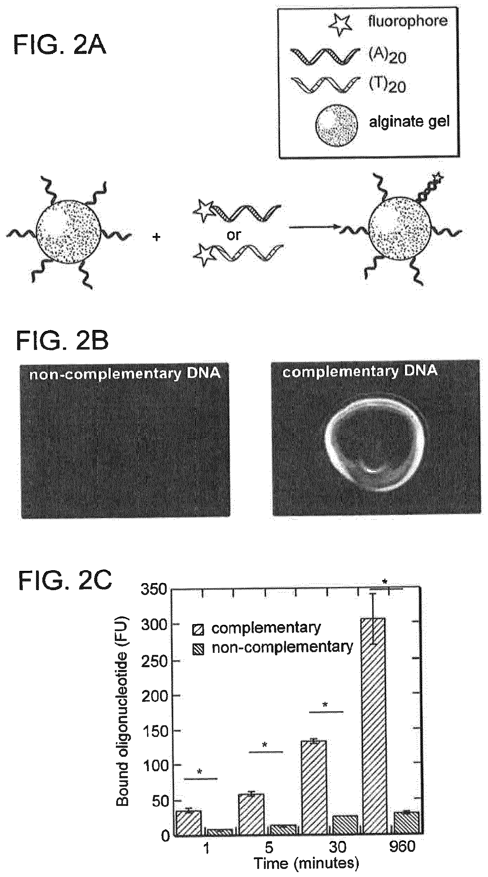

Figure 2A is a schematic showing DNA-conjugated calcium alginate gels incubated for various time periods with fluorescently-labeled complementary or non-complementary DNA. Figure 2A discloses "(T)₂₀" as SEQ ID NO: 1 and "(A)₂₀" as SEQ ID NO: 2. Figure 2B is a set of fluorescent images after 30 minutes of incubation. Figure 2C is a graph showing quantitation of retained fluorescence on beads after variable incubation times and washing away unbound DNA. Values represent mean and S.E.M. * represents p<.05 by Student's t-test. DNA-conjugated calcium-alginate gels retain oligonucleotide-binding properties.

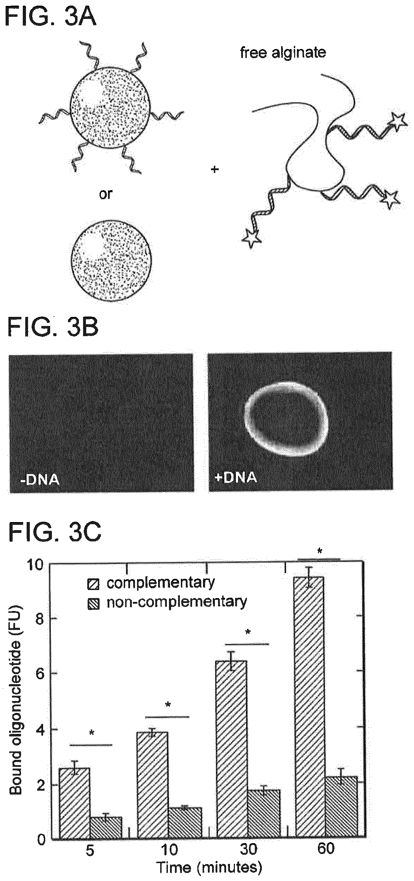

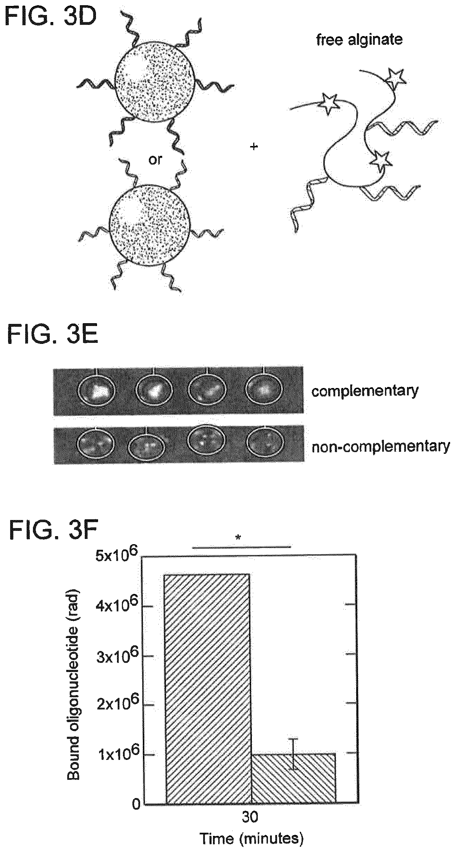

Figure 3A is a schematic showing calcium alginate gels comprised of polymer conjugated to DNA or unconjugated alginate that were incubated for various time periods with free alginate conjugated to fluorescently-labeled complementary DNA. Figure 3B is a set of fluorescent images after 1 hour. Figure 3C is a graph quantifying retained fluorescence on gels after incubation for different periods of time and washing away unbound alginate. Figure 3D is a schematic showing calcium alginate gels comprised of polymer conjugated to complementary or non-complementary DNA that were incubated for 30 mins with free alginate strands conjugated to DNA and near-IR fluorescent tags. Figure 3E is a set of epi-fluorescence images of gels after washing away unbound alginate. Figure 3F is a graph quantifying retained fluorescence on gels after washing away unbound alginate. Values represent mean and S.E.M. * represents p<.05 by Student's t-test. DNA-conjugated alginate gels selectively bind free alginate strands conjugated to complementary DNA.

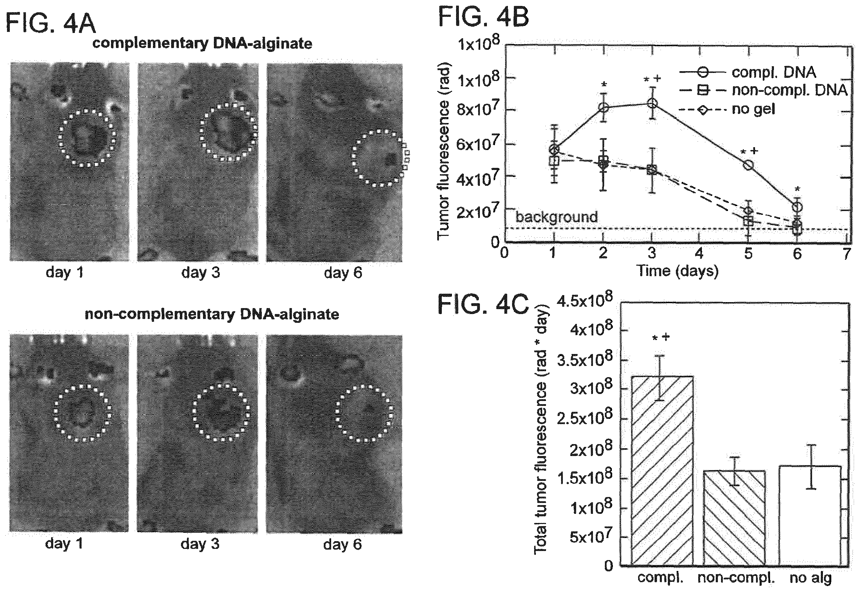

Figure 4A is a set of images of fluorescence in a tumor (dotted circles indicate tumor location). C57/B6 mice bearing B16/F10 melanoma tumors were injected intra-tumorally with alginate carrying complementary DNA, non-complementary DNA or no alginate. Homing to the tumor was tested through IV injection of a fluorescently-labeled alginate bearing DNA. Figure 4B is a graph quantifying the fluorescence in the tumor. Figure 4C is a graph showing the alginate residence at the tumor site, quantified by integrating area under the curve over the 6-day period for each experimental group. Values represent mean and S.E.M. (*) represents p<.05 vs. non-complementary and (⁺) p<.05 vs. no alginate controls by Student's t-test. DNA mediated homing of alginate polymers to intra-tumor gels.

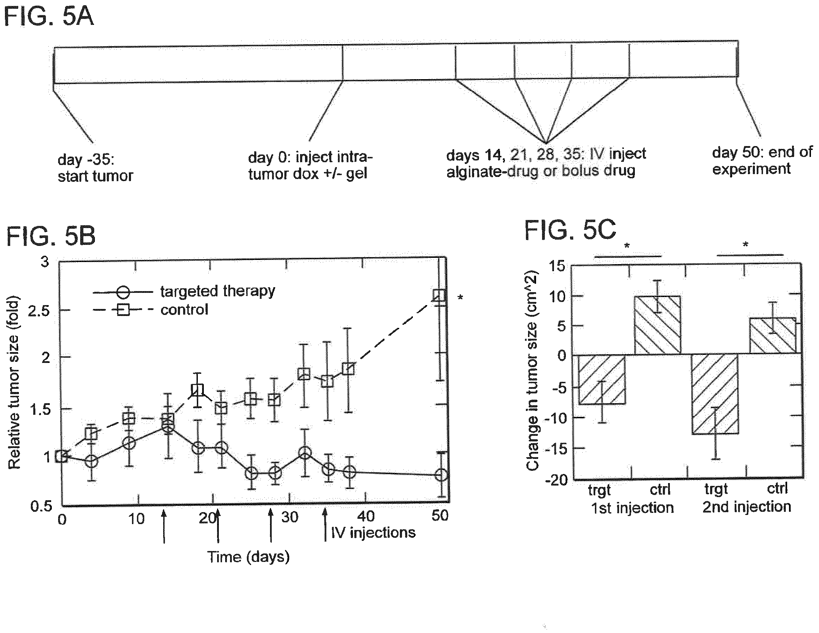

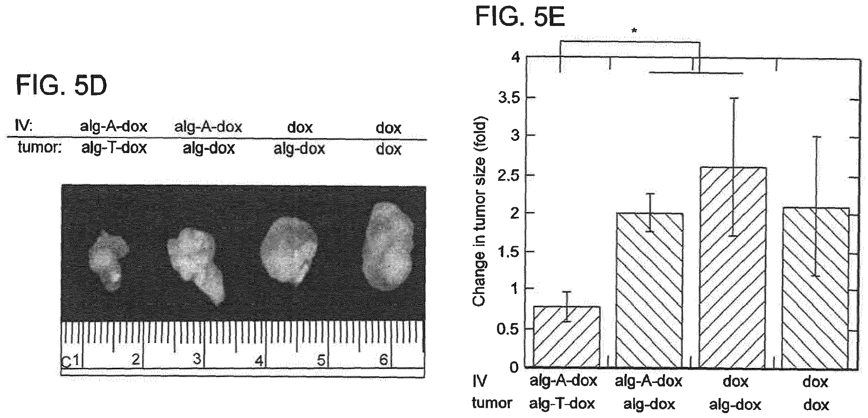

Figure 5A is a schematic showing the timeframe of experimental design. J:Nu mice were injected with human MDA-MB-231 breast cancer cells at day -35. Tumors were subsequently injected at day 0 with gels conjugated to DNA, control gels, or PBS with 80 µg doxorubicin. At 2, 3, 4, and 5 weeks after gel injection, animals were submitted to IV injections of doxorubicin conjugated to alginate and DNA or bolus doxorubicin controls. Figure 5B is a graph showing the tumor sizes as they were monitored over 7 weeks after gel injection for targeted and control groups. Figure 5C is a graph showing the 3-day change in tumor size after each IV injection. Figure 5D is a photograph of median-sized tumors. Figure 5E is a graph tumor growth seven weeks after intra-tumor injection in fully targeted alg-DNA-dox system or control systems with an alg-DNA-dox system incapable of DNA-mediated homing, bolus IV injections or bolus IV and intra-tumor injections. Tumor sizes were normalized to size at day 0. Values represent mean and S.E.M. N>5. * denotes statistical significance by Student's t-test. Drug refilling system led to arrest in tumor growth in vivo.

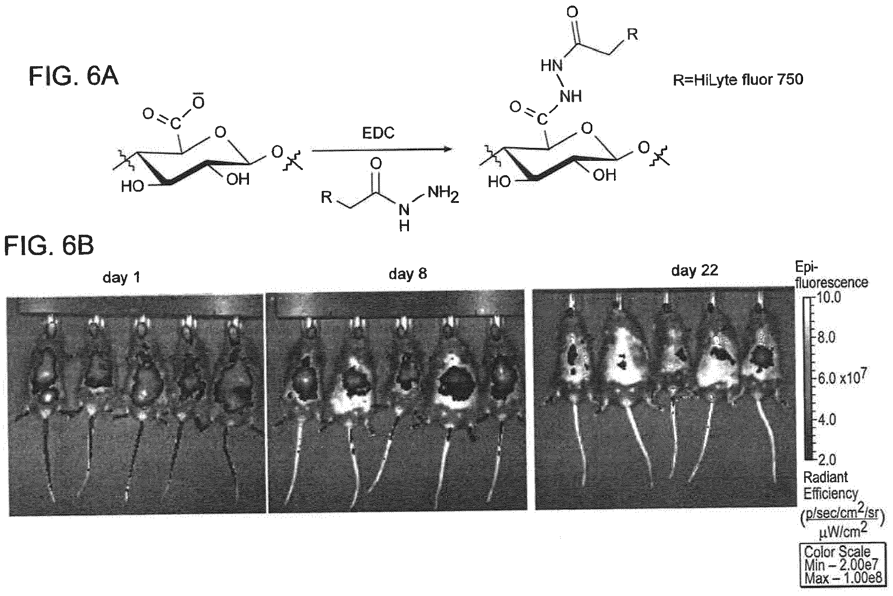

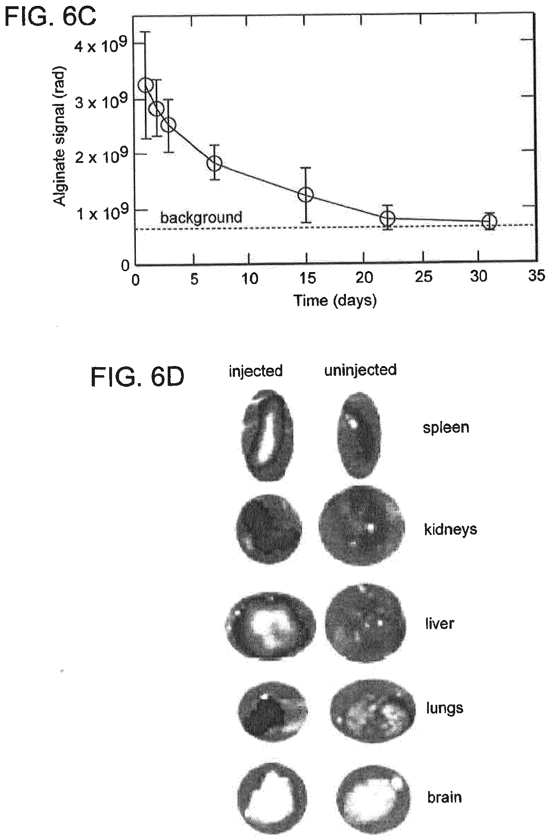

Figure 6A is a schematic for near-IR dye modification of alginate. Figure 6B is a set of fluorescence images of mice injected retro-orbitally with near-IR-dye-labeled (745 excitation, 820 emission) alginate. Images were taken on days 1, 8 and 22 post-injection. Figure 6C is a graph quantifying in vivo epi-fluorescence over four weeks in the blood by measuring snout fluorescence. Values represent mean and standard deviation. N=5. Figure 6D is a set of fluorescence images of internal organ two days after injection. These images illustrate the fate of IV-injected alginate in vivo.

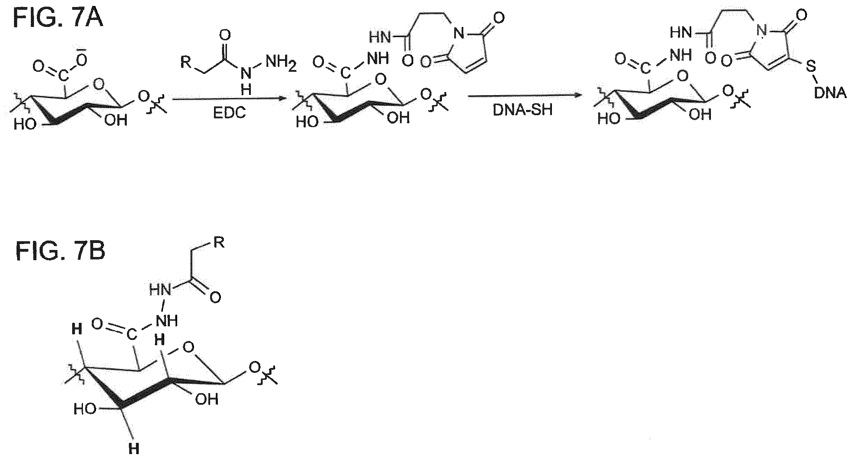

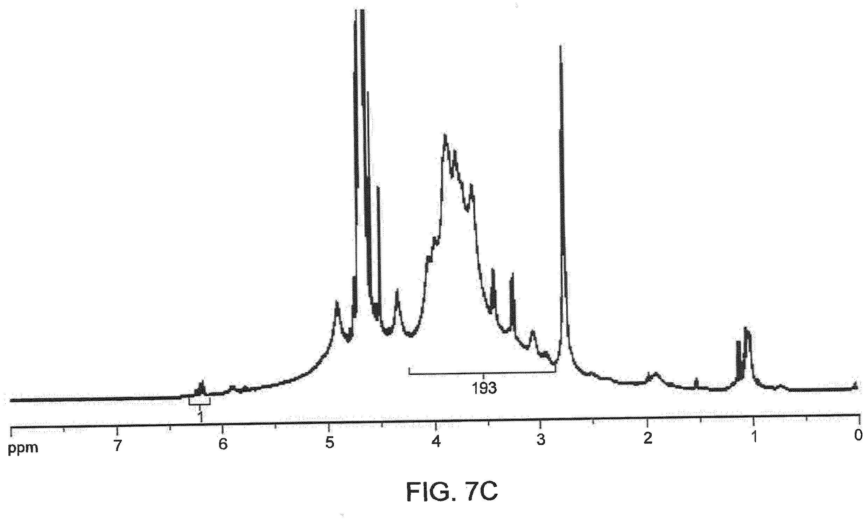

Figure 7A is schematic of conjugation of N-beta-Maleimidopropionic acid hydrazide-TFA (BMPH) and DNA to alginate. Figure 7B is a schematic of an alginate monomer (-1,250 monomers / strand) modified with BMPH, red protons designate those with absorptions 3-4.4 ppm, blue protons designate those with absorptions 6-6.56. Figure 7C is an nuclear magnetic resonance (NMR) spectrum of BMPH-modified alginate.

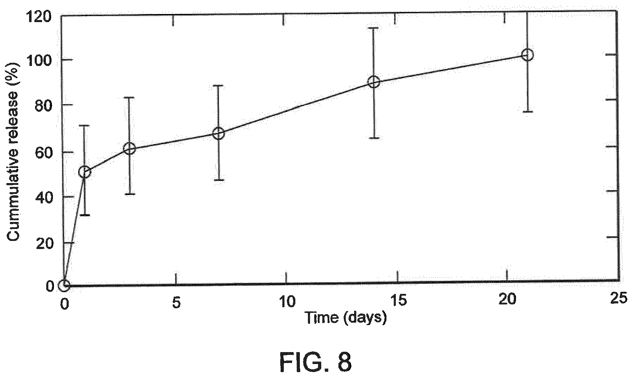

Figure 8 shows the release of doxorubicin from 2% calcium-crosslinked alginate gels over 21 days. Values represent mean and standard deviation. N=4.

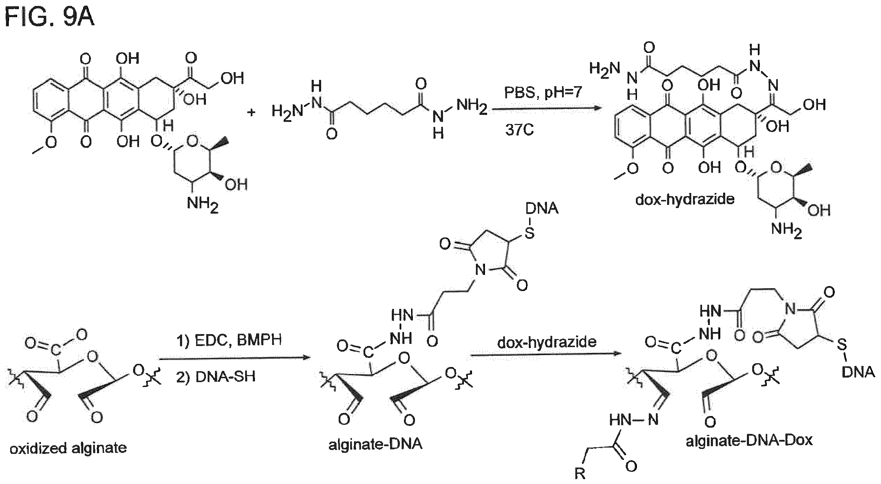

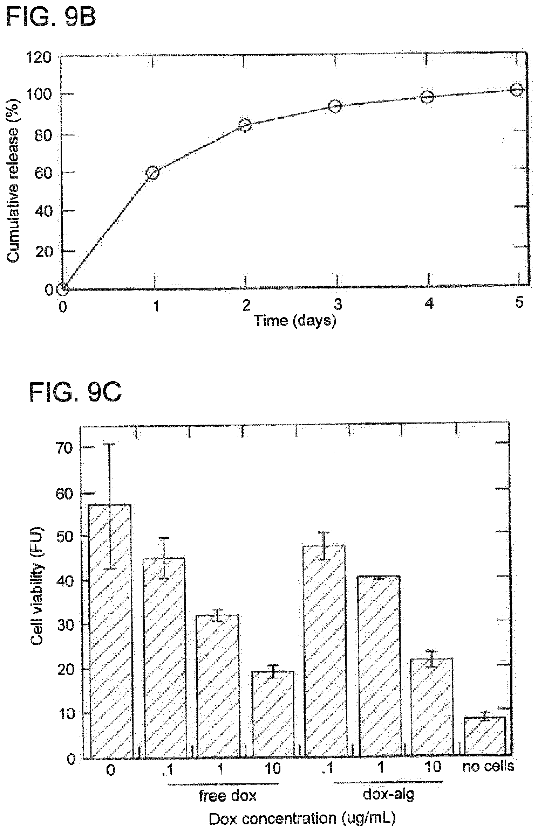

Figure 9A is a schematic of reactions performed on doxorubicin and alginate to bind doxorubicin to alginate through a hydrolyzable hydrazone linker. Figure 9B is a graph showing the release profile of hydrazone-linked doxorubicin from oxidized alginate. Figure 9C is a graph showing cell toxicity of free doxorubicin (dox) and of hydrazone-linked doxorubicin-alginate (dox-alg) against breast cancer MDA-MB-231 cells. Values represent mean and standard deviation. N=3. Doxorubicin loading and release from oxidized alginate is depicted in this figures.

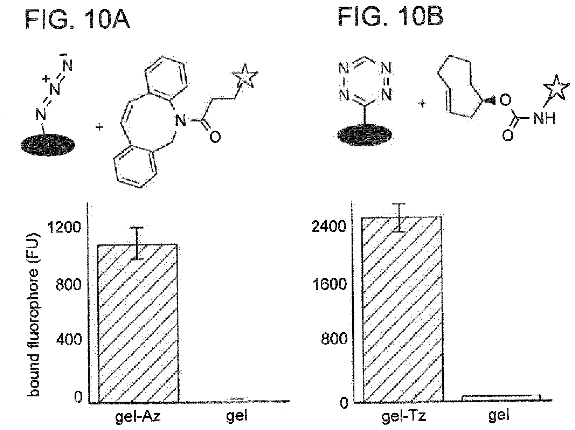

Figure 10A is a schematic and graph depicting click chemistry-mediated gel targeting. Azide-conjugated or control, calcium-crosslinked alginate gels were incubated with fluorophore carrying DBCO for 4 hours at 37 °C. Retained fluorescence was quantified. Figure 10B is a schematic and graph depicting click chemistry-mediated gel targeting. Tetrazine-conjugated or control, calcium-crosslinked alginate gels were incubated with fluorophore carrying *trans*-cyclooctene for 4 hours at 37 °C. Retained fluorescence was quantified.

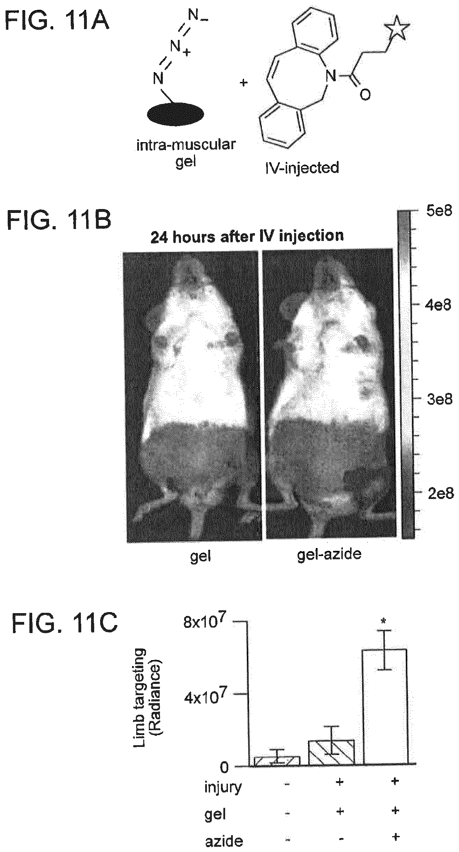

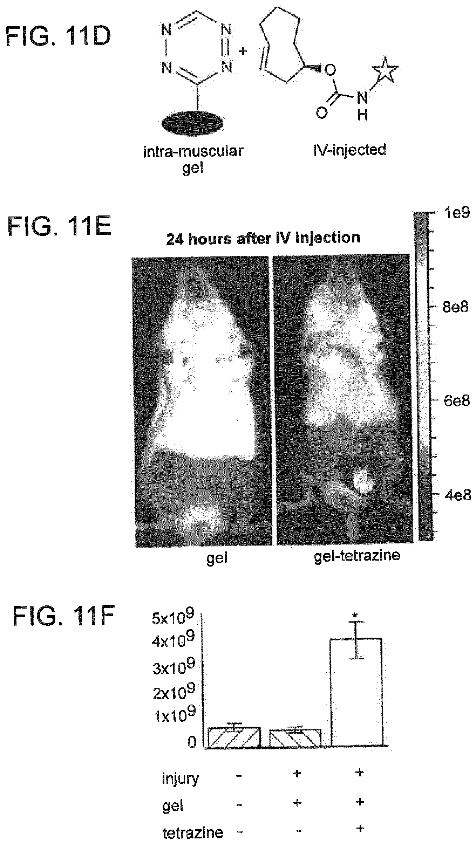

Figure 11A is a reaction scheme for alginate gels conjugated to azide or unconjugated, reacting with fluorescently labeled DBCO. Figure 11B is a set of images showing targeting of the IV-injected fluorescently labeled DBCO to the intra-muscular gel in the hindlimb of a hindlimb injury mouse model. Figure 11C is a graph quantifying the amount of fluorescence observed. Figure 11D is a reaction scheme for alginate gels conjugated to tetrazine or unconjugated, reacting with fluorescently labeled TCO. Figure 11E is a set of fluorescence images showing targeting of the IV-injected fluorescently labeled TCO to the intra-muscular gel in the hindlimb of a hindlimb injury mouse model. Figure 11F is a graph quantifying the amount of fluorescence observed. Values represent mean and S.E.M. n=3. * represents p<.01 vs. both controls by Student's t-test. Click chemistry mediated targeting of a drug surrogate to a hindlimb injury model.

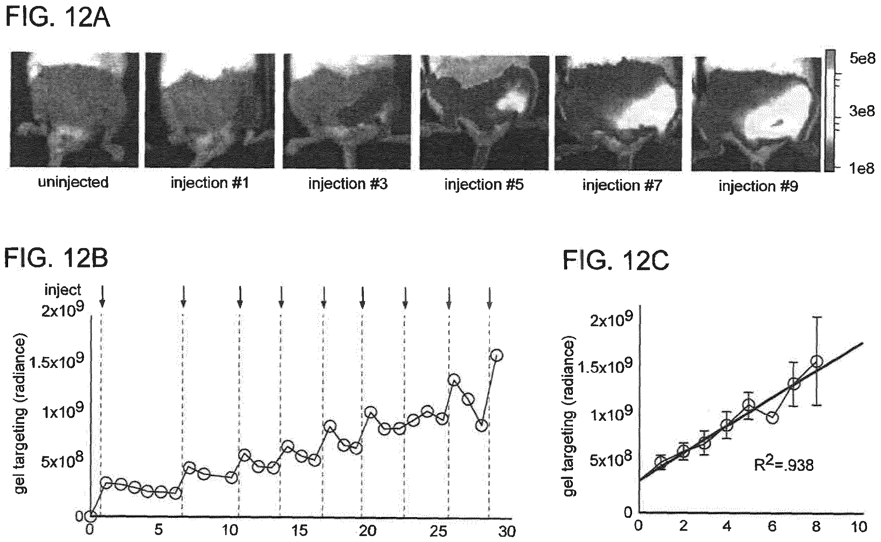

Figure 12A is a set of fluorescence images showing fluorescently-labeled DBCO that was repeatedly administered to mice over the course of a one month. Figure 12B is a graph quantifying limb fluorescence 24 hours after repeat injections (arrows) of targeting fluorescent DBCO. Figure 12C is a graph quantifying fluorescence 24 hours after fluorophore administration, showing a roughly linear increase in fluorescence with each injection. Repeat targeting of fluorescent drug surrogates to intra-muscular gel was achieved.

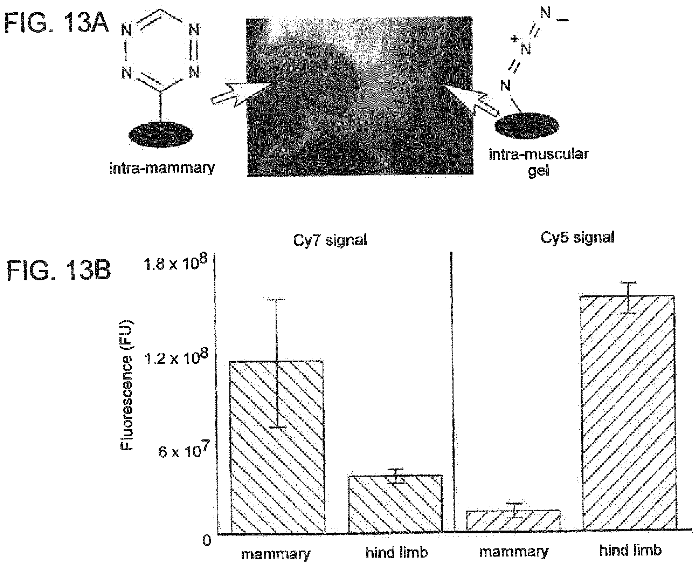

Figure 13A is an image plus schematic showing fluorescence in a mouse 1) in which alginate-Tz gel was implanted intra-muscularly in the left hind limb and alginate-Az was implanted into the right mammary pad of the same mouse, and 2) where a mixture of Cy5-TCO and Cy7-DBCO was injected IV, and 3) with the mouse imaged 48 hours later with fluorescence at both injection sites. Figure 13B is a set of graphs quantifying the fluorescence at each injection site. Spatial separation and specific targeting of two different small molecules was achieved using this system.

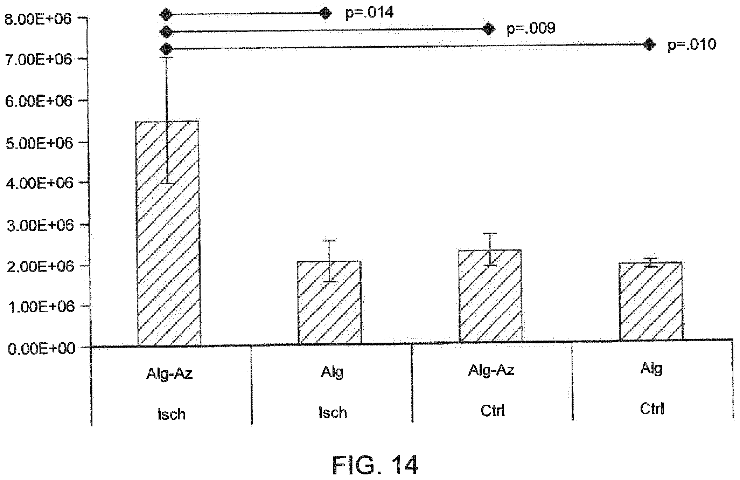

Figure 14 is a graph showing the targeting of oral delivery of fluorescently labeled DBCO to an alginate-azide gel implanted in mouse models of hind limb ischemia. Fluorescence (y-axis) at the site of alginate injection on mouse models of hind limb ischemia (Isch) and at a control site on the contralateral limb (Ctrl) is shown.

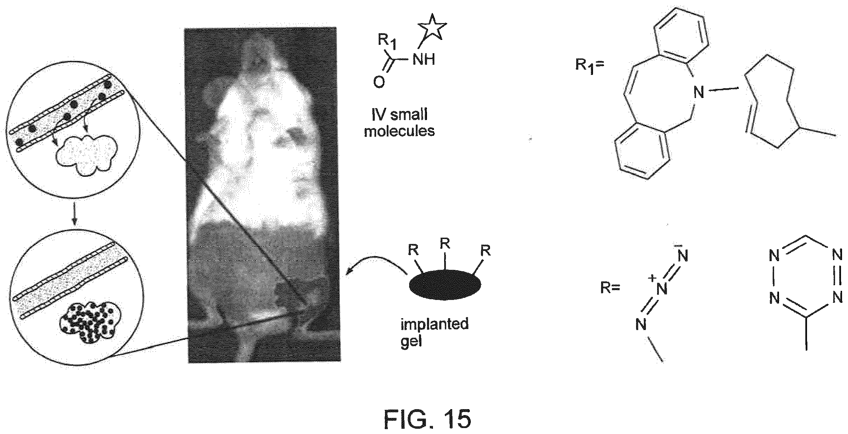

Figure 15 is an image and schematics illustrating the targeting of small molecules (e.g., via IV or oral) specifically to an implanted gel at a specific site in a body. The targeting can be mediated by click chemistry reactions.

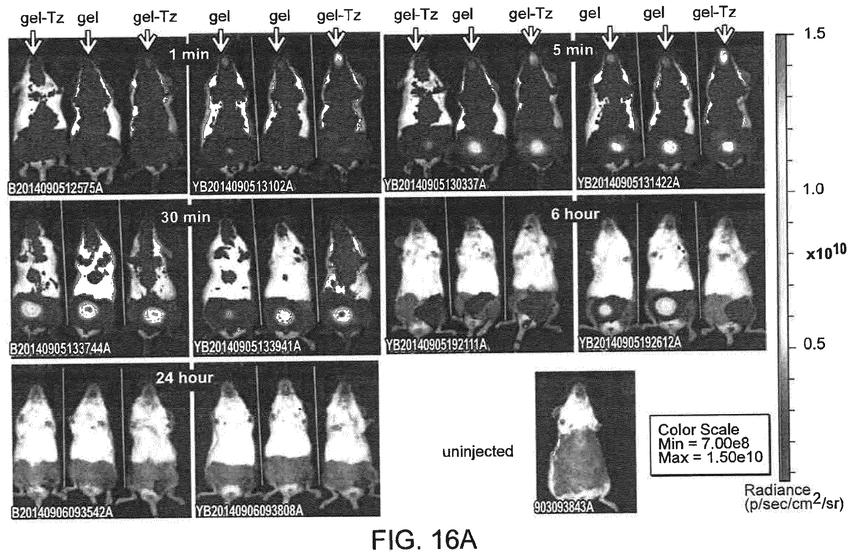

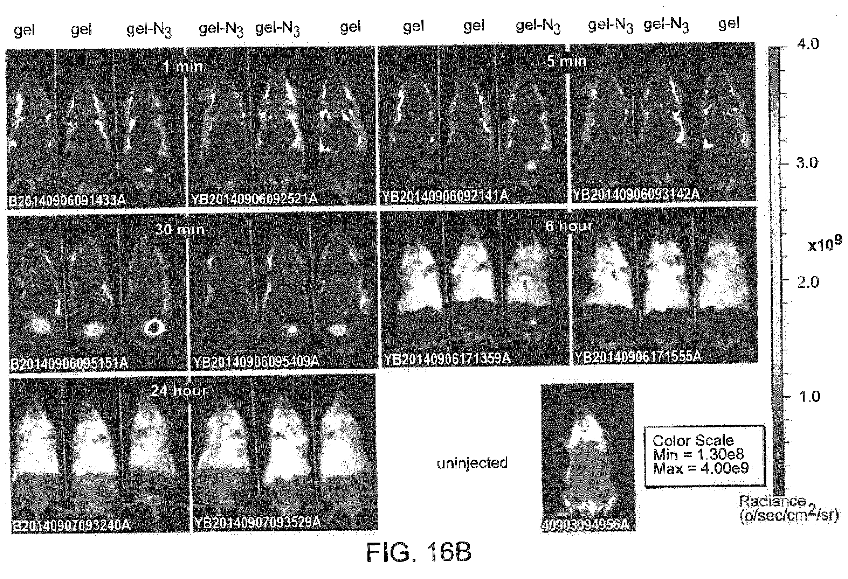

Figure 16A is a panel of live animal images of mice subjected to hind-limb ischemia and injected with unconjugated alginate gels or conjugated to tetrazine. 24 hours after surgery, fluorescent-TCO was injected IV. Animals were imaged at 1, 5, and 30 minutes, 6 hours, and 24 hours following TCO injection. Figure 16B is a panel of live cell images of mice subjected to hind-limb ischemia and injected with unconjugated alginate gels or conjugated to azide. 24 hours after surgery, fluorescent-DBCO was injected IV. Animals were imaged at 1, 5 and 30 minutes, 6 hours and 24 hours following DBCO injection. Images show mice (n=3 for each o the 2 groups) labeled with the alginate gel received intramuscularly at the different time points.

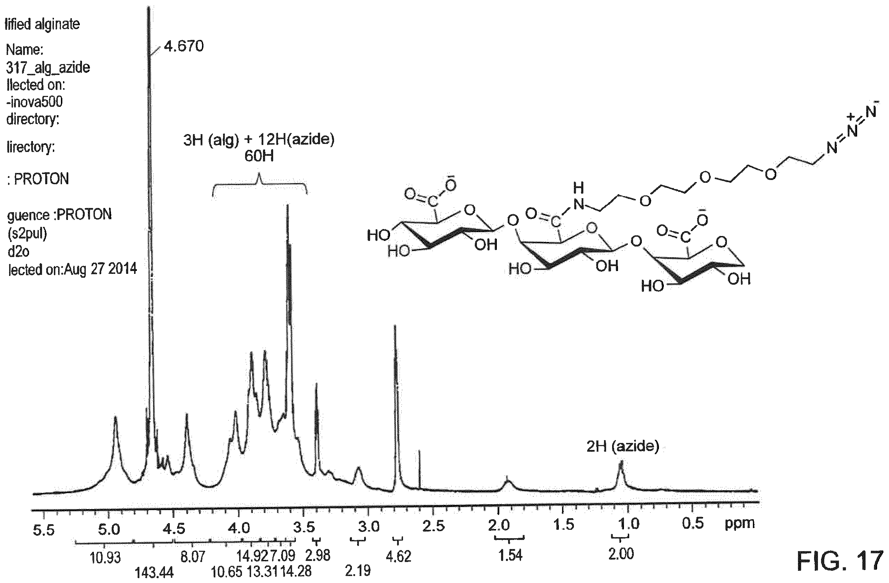

Figure 17 is a ¹H NMR spectrum of azide-modified alginate.

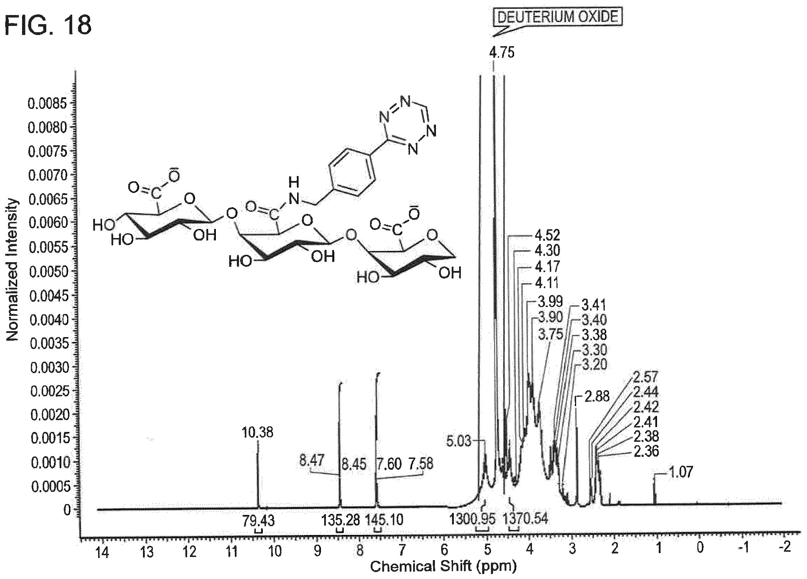

Figure 18 is a ¹H NMR spectrum of tetrazine-modified alginate.

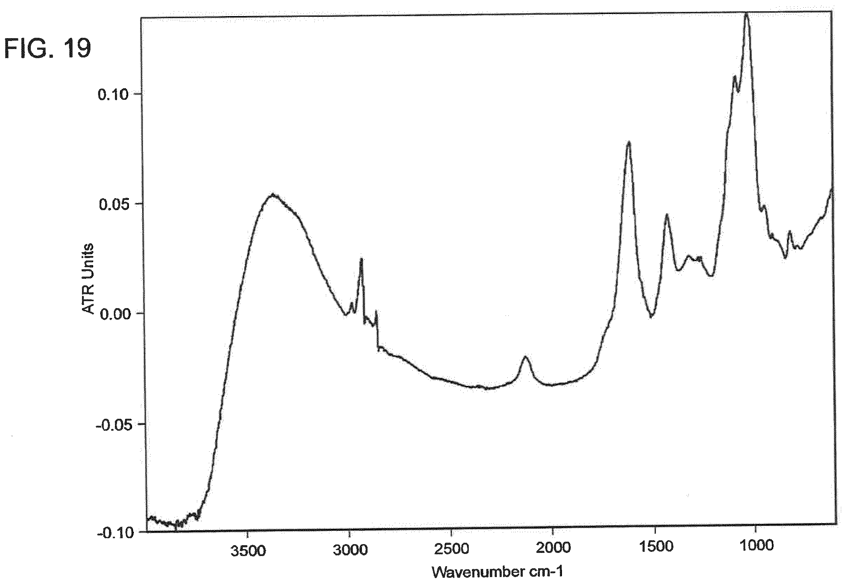

Figure 19 is an attenuated total reflectance (ATR)/infrared (IR) (ATIR) spectrum of azide-conjugated alginate.

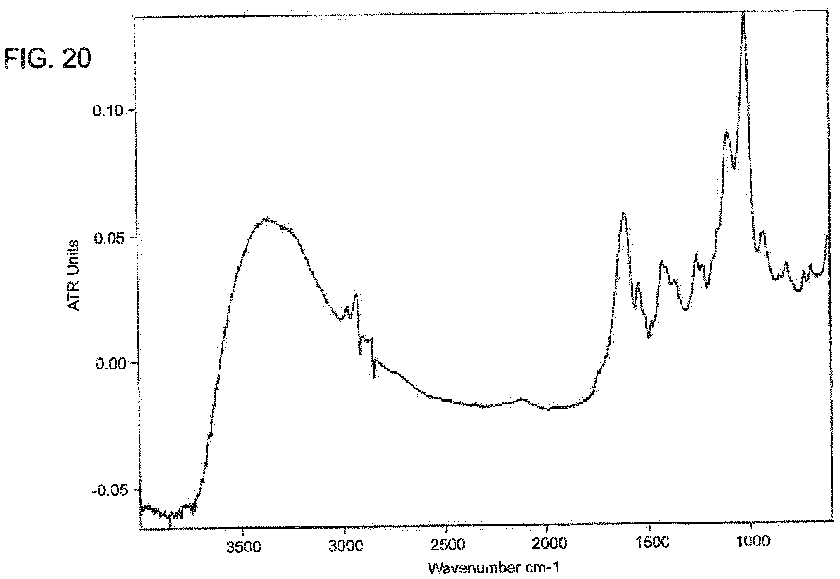

Figure 20 is an ATIR spectrum of azide-conjugated alginate reacted with 100 equivalents of DBCO-Cy7.

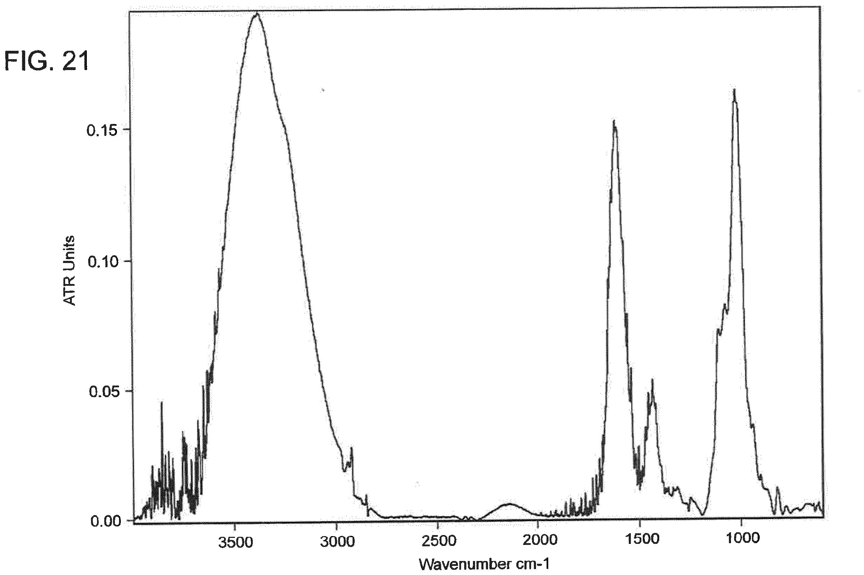

Figure 21 is an ATIR spectrum of tetrazine-conjugated alginate.

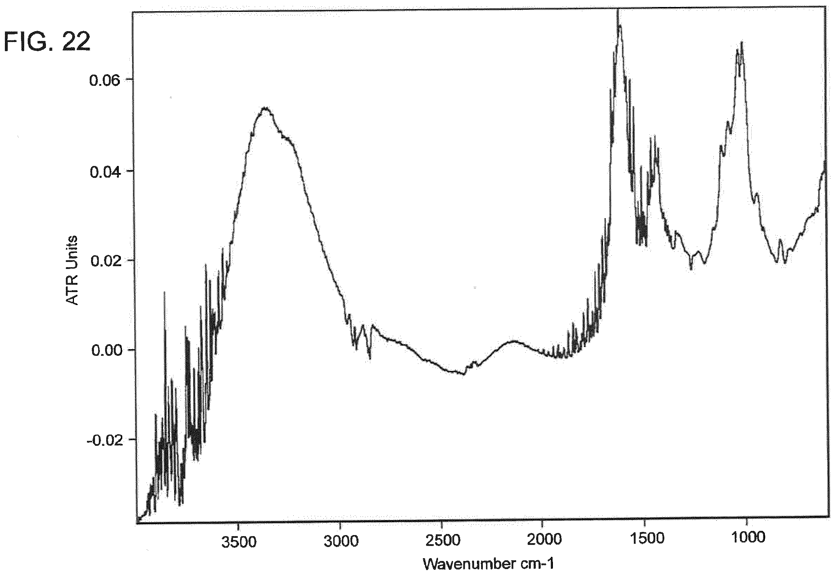

Figure 22 is an ATIR spectrum of tetrazine-conjugated alginate reacted with 100 equivalents of TCO-Cy5.

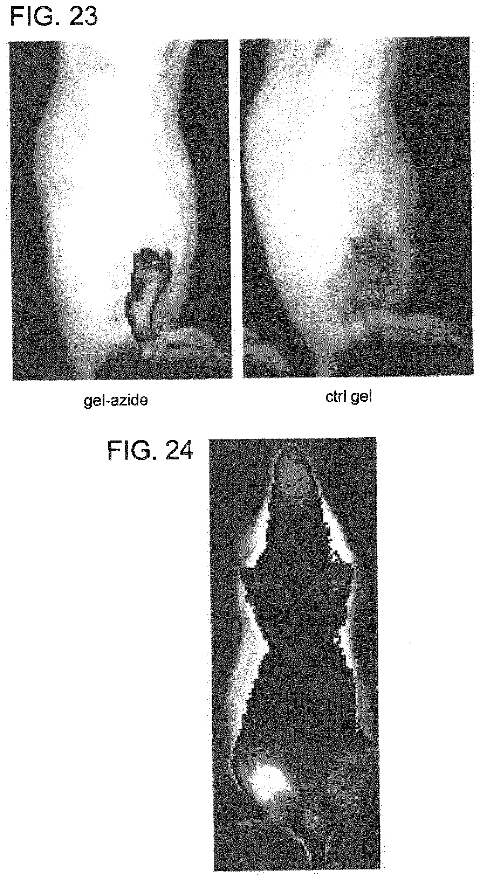

Figure 23 is a set of images showing targeting of the IV-injected fluorescently labeled DBCO to an intraosseous gel modified with azide or unmodified control gel in the femur of a mouse.
Figure 24 is an image showing targeting of the IV-injected fluorescently labeled DBCO to a gel modified with azide, injected into the right knee joint of a mouse.

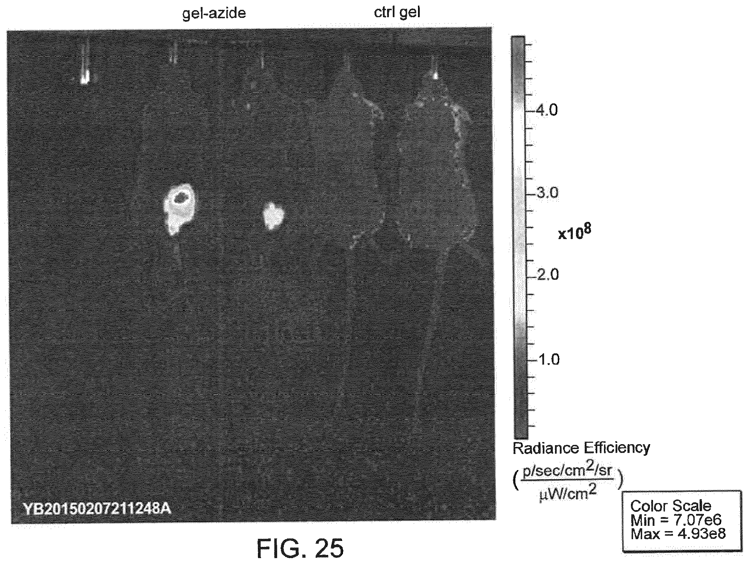

Figure 25 is an image showing targeting of the IV-injected fluorescently labeled DBCO to a gel modified with azide or control gel, injected into the tumor of a mouse. Imaged 24 hours after IV injection.

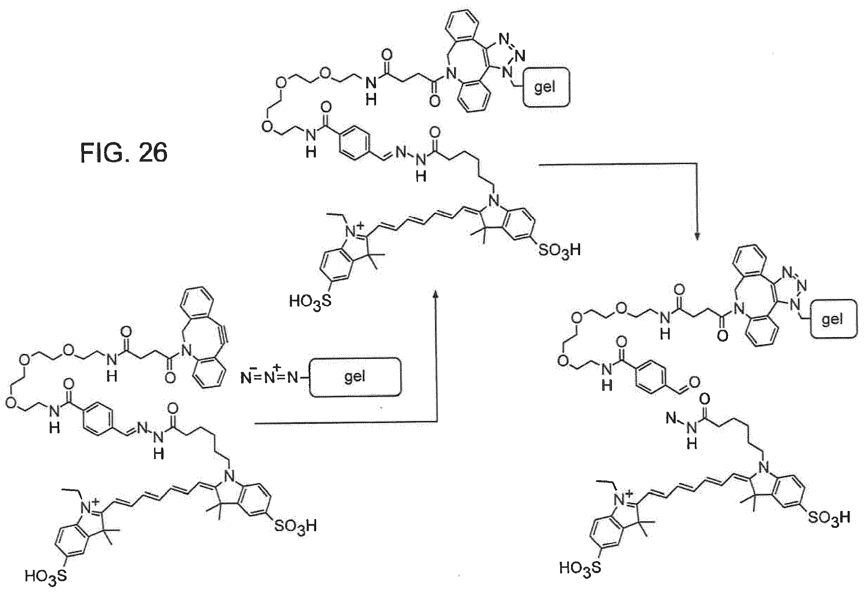

Figure 26 shows the capture of a small molecule (Cy7 linked to DBCO through a hydrolysable hydrazone linker) by an azide-modified gel and the subsequent release of a small molecule through hydrolysis after the capture.

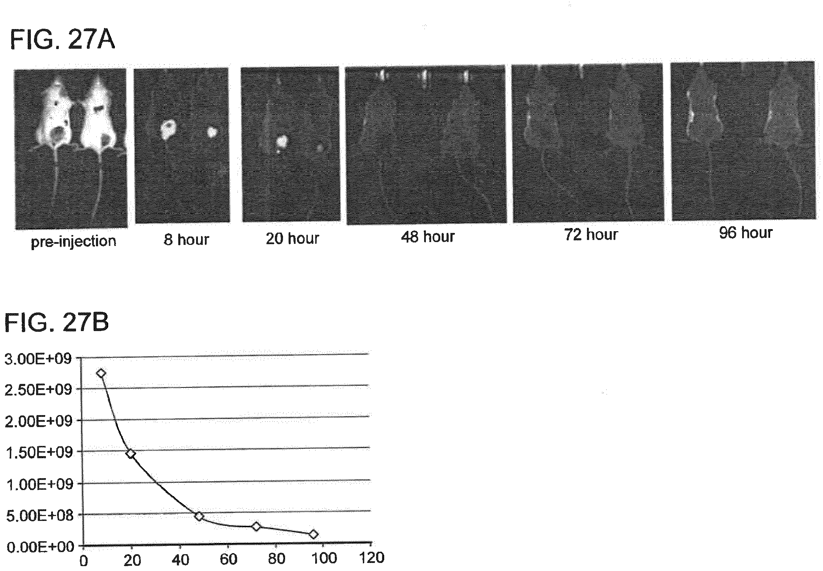

Figure 27A is a series of images showing targeting of the IV-injected small molecule by a gel modified with azide injected into the tumor of a mouse. The small molecule is subsequently released, leading to loss of the signal. Figure 27B is a line graph showing the quantification of the small molecule at the tumor site over time (hours) demonstrating release of the small molecule.

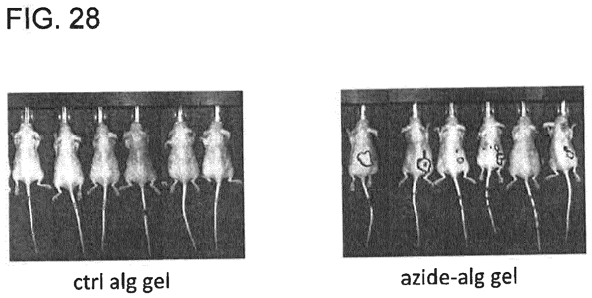

Figure 28 is an image showing targeting of the IV-injected fluorescently labeled DBCO to a gel modified with azide or control gel, injected into the tumor of a mouse. Imaged 24 hours after IV injection.

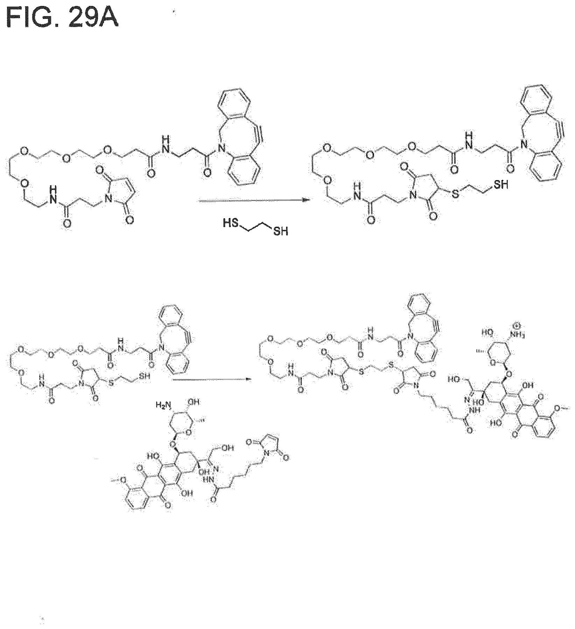

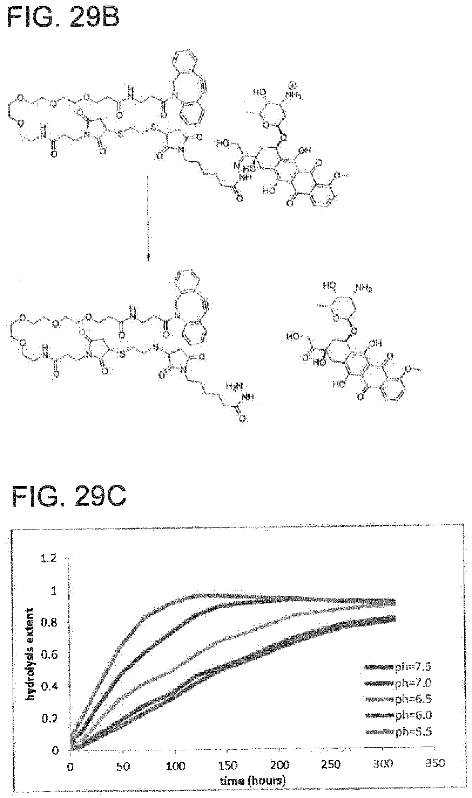

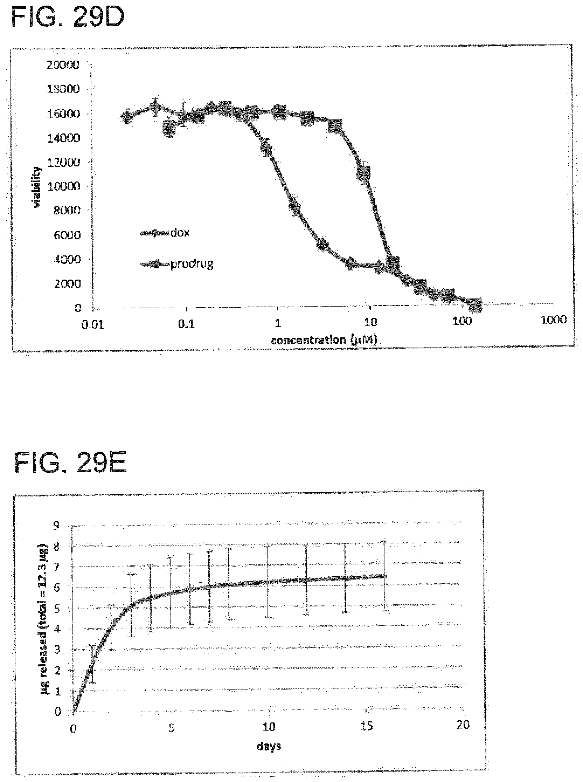

Figures 29A-E depict the synthesis and characterization of the DBCO-doxorubicin prodrug. Figure 29A is a schematic of reactions for synthesis of doxorubicin prodrug. Figure 29B is a schematic of reactions for hydrolysis of doxorubicin prodrug into active doxorubicin. Figure 29C is a gragh depicting the kinetics of hydrolysis of the DBCO-doxorubicin prodrug into active doxorubicin at different pH. Figure 29D is a graph depicting cell toxicity of free doxorubicin (dox) and doxorubicin prodrug (prodrug) against cancer cells. Figure 29E is a graph depicting the release profile of doxorubicin from azide-modified gels.

### DETAILED DESCRIPTION OF THE INVENTION

The present invention is based, at least in part, on the development of a drug delivery system which permits refilling of a drug delivery device *in vivo* in a minimally invasive manner, by modifying the drug delivery device with molecular targets capable of recognizing and binding drug refills circulating in the body. The drug delivery system features a dual functionality drug refill, which not only permits a direct targeted delivery of a pharmaceutical composition from the drug refill to the drug delivery device, but also masks the potential toxicity of a pharmaceutical composition, such as a chemotherapeutic, until the pharmaceutical composition is delivered to the drug delivery device. The pharmaceutical composition will only be unmasked upon delivery into the drug delivery device where the target is separated from the pharmaceutical composition, thus eliminating any side effects or toxicity associated with the pharmaceutical composition at any undesired sites. Release of the pharmaceutical composition from the drug delivery device can be achieved in a controlled manner. Unlike the existing drug delivery systems which typically mediate delivery of a pharmaceutical composition within minutes, the drug delivery systems of the present invention provide a more sustained and controlled release of a pharmaceutical composition over a time scale of days, weeks, months or years.

For example, an injectable drug delivery device is manufactured to include a target recognition moiety that binds to the target on a drug refill infused into the blood (Fig. 1A). Drug refills infused into the blood of a patient extravasate into the desired location and are bound by the drug delivery device (Fig. 1B). The drug delivery system allows a direct delivery of drugs from the drug refill to the drug delivery device. Subsequently, drugs can be released from the drug delivery device to the desired location in the subject, allowing a sustained release of drug at the desire location (Fig. 1C).

Thus, the invention provides, in one embodiment, a drug delivery system comprising a drug delivery device and a drug refill and, in another embodiment, the system for use in (a) maintaining or reducing the size of a tumor, or (b) treating an eye disease. Other embodiments of the invention include a stationary drug delivery device comprising a pharmaceutical composition, a target recognition moiety and a carrier; a drug refill comprising a pharmaceutical composition and a target; and a kit for drug delivery comprising a drug delivery device and a drug refill.

### I. Definition

In order that the present invention may be more readily understood, certain term are first defined.

Unless otherwise defined herein, scientific and technical terms used in connection with the present invention shall have the meanings that are commonly understood by those of ordinary skill in the art. The meaning and scope of the terms should be clear, however, in the event of any latent ambiguity, definitions provided herein take precedent over any dictionary or extrinsic definition.

The use of the terms "a" and "an" and "the" and similar referents in the context of describing the invention (especially in the context of the following claims) are to be construed to cover both the singular and the plural (*i.e.,* one or more), unless otherwise indicated herein or clearly contradicted by context. The terms "comprising, "having," "including," and "containing" are to be construed as open-ended terms (*i.e.,* meaning "including, but not limited to") unless otherwise noted. Recitation of ranges of values herein are merely intended to serve as a shorthand method of referring individually to each separate value recited or falling within the range, unless otherwise indicated herein, and each separate value is incorporated into the specification as if it were individually recited. Ranges provided herein are understood to be shorthand for all of the values within the range. For example, a range of 1 to 50 is understood to include any number, combination of numbers, from the group consisting of 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, or 50 or sub-ranges from the group consisting of 10-40, 20-50, 5-35, etc.

As described herein, the term "drug delivery device" generally refers to a device for delivering, or transporting a pharmaceutical compound in the body as needed to safely achieve its desired therapeutic effect.

As used herein, the term "drug refill" generally refers to a composition which delivers a pharmaceutical composition to a drug delivery device, and in doing so, replenishes or replaces the pharmaceutical composition that has been used up in the drug delivery device. A drug refill of the present invention comprises a pharmaceutical composition and a target. The drug refill has a dual functionality, which not only permits a direct targeted delivery of a pharmaceutical composition from the drug refill to the drug delivery device, but also masks the potential toxicity of a pharmaceutical composition, such as a chemotherapeutic, until the pharmaceutical composition is delivered to the drug delivery device.

As used herein, the term "pharmaceutical composition" generally refers to a medication with a therapeutic effect when administered to a subject. The pharmaceutical composition may comprise a small molecule or a biologic, *e.g.,* an antibody, a vaccine, a blood, or blood component, an allergenic, a gene therapy, a recombinant therapeutic protein, and living cells or somatic cells used in cell therapy. In some embodiments, the pharmaceutical composition is attached to the target directly. In other embodiments, the pharmaceutical composition is attached to the target via a cleavable linker.

As used herein, the term "small molecule" generally refers to a low molecular weight organic compound that may help regulate a biological process. Suitable small molecules can be drugs, such as an anti-cancer drug, a drug that promotes wound healing, a drug that promotes vascularization, a drug that treats or prevents infection, a drug that prevents restenosis, a drug that reduces macular degeneration, a drug that prevents immunological rejection, a drug that prevents thrombosis, and a drug that treats inflammation. Exemplary drugs are described in detail below.

As used herein, the term "biologic" generally refers to any medicinal product manufactured in, extracted from, or semisyntehsized from biological sources which is different from chemically synthesized pharmaceuticals. Exemplary biologies used in the present invention may include, but not limited to antibodies, vaccines, blood, or blood components, allergenics, somatic cells, gene therapies, tissues, recombinant therapeutic protein, and living cells used in cell therapy. Biologics can be composed of sugars, proteins, or nucleic acids or complex combinations of these substances, or may be living cells or tissues, and they can be isolated from natural sources such as human, animal, or microorganism.

As used herein, the term "attach" refers to formation of a bond, or a plurality of bonds between one composition and another. In some embodiments, the bond may be formed directly between the pharmaceutical composition and the target. Suitable bonds may include, but not limited to, covalent bonds, non-covalent bonds, or ionic bonds. In other embodiments, the bond may be formed between the pharmaceutical composition and the target via a linker. Suitable linkers may include cleavable linkers or non-cleavable linkers. Exemplary cleavable linkers are described in detail below.

As used herein, the term "target" generally refers to a molecule that is a target to be recognitzed by a target recognition moiety. Examples of such a target may include, but not limited to, inorganic chemical species or organic chemical species such as low-molecular-weight compounds, high-molecular-weight compounds, and substances from living organisms. Other examples of a target include sugars, lipids, peptides, proteins, nucleic acids, or any bioothogonal functional groups. Examples of bioothogonal functional species are described in detail below.

As used herein, the term "target recognition moiety" refers to a molecule that is capable of binding selectively to a given target. The target recognition moiety may include, but not limited to, inorganic chemical species or organic chemical species such as low-molecular-weight compounds, high-molecular-weight compounds, and substances from living organisms. More examples of a target recognition moiety include sugars, lipids, peptides, proteins, nucleic acids, or any complementary bioothogonal functional groups.

As used herein, the term "bioorthogonal" or "bioorthogonal functional group" refer to a functional group or chemical reaction that can occur inside a living cell, tissue, or organism without interfering with native biological or biochemical processes. A bioorthogonal functional group or reaction is not toxic to cells. In some embodiments, the target on the drug refill comprises a bioorthonal functional group and the target recognition moiety on the drug delivery device comprises a complementary functional group, where the bioorthogonal functional group is capable of chemically reacting with the complementary functional group to form a covalent bond.

### II. Refillable Drug Delivery Systems

In one aspect, the present invention features a refillable drug delivery system. The system of the invention comprises a drug delivery device and a drug refill, wherein the drug delivery device comprises a carrier and a target recognition moiety, and the drug refill comprises a pharmaceutical composition and a target. The pharmaceutical composition is attached to the target via a cleavable linker capable of being cleaved within the subject. The drug delivery device is suitable for implantation in a desirable location within a subject. Recognition between the drug delivery device and the drug refill is facilitated by the target and the target recognition moiety, wherein the target and the target recognition moiety form a two-component binding pair. The drug refill is mobile until the target on the drug refill binds to the target recognition moiety on the drug delivery device. Upon binding of the target to the target recognition moiety, the drug refill delivers the pharmaceutical composition directly to the drug delivery device, thereby refilling the drug delivery device. The pharmaceutical composition is released at the desirable location within the subject in a controlled manner over a time scale of days, weeks, months, or years, upon cleavage of the cleavable linker.

In another aspect, the present invention provides a system comprising a drug delivery device and a drug refill, wherein the drug delivery device comprises a target recognition moiety and is suitable for implantation in a desirable location within a subject; wherein the drug refill comprises a pharmaceutical composition and a target; wherein the pharmaceutical composition is attached to the target via a cleavable linker capable of being cleaved within the subject, wherein the pharmaceutical composition has undesired toxicity and the drug refill masks the toxicity of the pharmaceutical composition, wherein the target and the target recognition moiety form a two-component binding pair; wherein the drug refill is mobile until the target on the drug refill binds to the target recognition moiety on the drug delivery device, wherein upon binding of the target to the target recognition moiety, the drug refill delivers the pharmaceutical composition directly to the drug delivery device, and wherein the pharmaceutical composition is released in a controlled manner from the drug delivery device to the desirable location within the subject.

In another aspect, the present invention features a stationary drug delivery device comprising a pharmaceutical composition, a target recognition moiety and a carrier, wherein the target recognition moiety is capable of binding to a target on a drug refill, wherein the drug refill comprises a pharmaceutical composition and a target, wherein the pharmaceutical composition is attached to the target via a cleavable linker capable of being cleaved within a subject, wherein the pharmaceutical composition is released at a desirable location within the subject in a controller manner over a time scale of days, weeks, months, or years, upon cleavage of the cleavable linker, and wherein the drug delivery device is suitable for implantation in a desired location within a subject.

In yet another aspect, the present invention provides a drug refill comprising a pharmaceutical composition and a target, wherein the pharmaceutical composition is attached to the target via a cleavable linker capable of being cleaved within a subject, wherein the pharmaceutical composition has undesired toxicity and the drug refill masks the toxicity of the pharmaceutical composition, wherein the target is capable of binding to a target recognition moiety on a drug delivery device, wherein the drug refill is mobile until the target binds to the target recognition moiety on a drug delivery device, wherein upon binding of the target to the target recognition moiety, the drug refill delivers the pharmaceutical composition to the drug delivery device, the pharmaceutical composition is unmasked after delivery into the drug delivery device and the pharmaceutical composition is released at a desirable location within the subject in a controlled manner over a time scale of days, weeks, months, or years, upon cleavage of the cleavable linker.

### A. Drug Delivery Devices

The drug delivery devices of the present invention comprise a carrier and a target recognition moiety. The target recognition moiety is attached directly to the drug delivery device, *i.e.,* there is no intermediate moiety, such as a receptor, a linker or a ligand, between the device and the target recognition moiety. For this reason, unlike intermediate binding moieties that could cause non-specific interactions, there is no non-specific binding associated with the drug delivery systems described herein. In this manner, the pharmaceutical compositions, *e.g.,* small molecules or biologics, are delivered based on the direct interaction between the target on the drug refill and the target recognition moiety on the drug delivery device.

The drug delivery devices of the present invention can be implanted or injected to a desired location within a subject. In some embodiments, the desired location is a tissue within a subject or at a site away from the tissue. In other embodiments, the desired location is an organ within a subject or at a site away from the organ. The physiological state of the tissue or the organ may be normal or abberant. In some embodiments, the desired location is an implant, a prothestic, or any tissue or device that can be introduced into the body or on the surface of the body. Drug delivery devices are introduced into or onto a bodily tissue using a variety of known methods and tools, *e.g.,* tweezers, graspers, hypodermic needle, endoscopic manipulator, endo- or trans-vascular- catheter, stereotaxic needle, snake device, organ-surface-crawling robot (U.S. Patent Publication No. 20050154376; Ota et al., 2006, Innovations 1:227-231), minimally invasive surgical devices, surgical implantation tools, and transdermal patches. Devices can also be assembled in place, for example by sequentially injecting or inserting matrix materials.

In some embodiments, the drug delivery devices comprise a carrier, *e.g.,* a polymer, a protein, a hydrogel (*e.g.,* a synthetic hydrogel or a biological hydrogel), an organogel, a ceramic, a composite, a metal, a wood, or a glass material. In one embodiment, the drug delivery devices of the invention do not comprise a protein. In some embodiments, the drug delivery devices of the invention do not comprise a living cell, *e.g.,* a cancer cell, or other biological entity.

The drug delivery devices of the present invention may comprise a pharmaceutical composition. In other embodiments, the drug delivery devices are implanted or injected to a desired location within the subject without a pharmaceutical composition. For example, a drug delivery device comprising a polymeric material and a target recognition moiety may be implanted or injected to a desired location within the subject. Subsequently, if infection occurs, an antibiotic will be delivered into the drug delivery device.

The pharmaceutical compositions may also be added to the drug delivery devices before implantation using known methods including surface absorption, physical immobilization, *e.g.,* using a phase change to entrap the substance in the hydrogel material. For example, a pharmaceutical composition, such as a biologic, *e.g.,* a polypeptide, *e.g.,* growth factor, is mixed with the hydrogel material while it is in an aqueous or liquid phase, and after a change in environmental conditions (*e.g.,* pH, temperature, ion concentration), the liquid gels or solidifies thereby entrapping the pharmaceutical composition. Alternatively, covalent coupling, *e.g.,* using alkylating or acylating agents, is used to provide a stable, long term presentation of a pharmaceutical composition on the hydrogel in a defined conformation. Exemplary reagents for covalent coupling of pharmaceutical compositions, such as peptide/proteins, are provided in the table below.

**Methods to covalently couple peptides/proteins to polymers**

| Functional Group of Polymer | Coupling reagents and cross-linker | Reacting groups on proteins/peptides |
|---|---|---|
| -OH | Cyanogen bromide (CNBr) | -NH₂ |
| | Cyanuric chloride | |
| | 4-(4,6-Dimethoxy-1,3,5-triazin-2-yl)-4-methyl-morpholinium chloride (DMT-MM) | |
| -NH₂ | Diisocyanate compounds | -NH₂ |
| | Diisothoncyanate compounds | -OH |
| | Glutaraldehyde | |
| | Succinic anhydride | |
| -NH₂ | Nitrous Acid | -NH₂ |
| | Hydrazine + nitrous acid | -SH |
| | | -Ph-OH |
| -NH₂ | Carbodiimide compounds (e.g., EDC, | -COOH |
| | DCC)[a] | |
| | DMT-MM | |
| Azide | Copper catalyst | -alkyne |
| Azide | None | -DBCO |
| Tetrazine | none | -TCO |
| -NH₂ | Sortase enzyme | peptide |
| -O-NH₂ | Pyridoxamine | N-terminus |
| -COOH | Thionyl chloride | -NH₂ |
| | N-hydroxysuccinimide | |
| | N-hydroxysulfosuccinimide + EDC | |
| -SH | Disulfide compound | -SH |
| Maleimide | None | -SH |
| iodoacetate | none | SH |

| | | |
|---|---|---|
| [a] EDC: 1-ethyl-3-(3-dimethylaminopropyl) carbodiimide hydrochloride; DCC: dicyclohexylcarbodiimide | | |

In some embodiments, the drug delivery devices comprise a hydrogel selected from the group consisting of collagen, alginate, polysaccharides, hyaluronic acid (HA), polyethylene glycol (PEG), poly(glycolide) (PGA), poly(L-lactide) (PLA), poly(lactide-co-glycolide) (PLGA), and poly lactic-coglycolic acid. In some embodiments, the polymer of the drug delivery device is in the form of a hydrogel, *e.g.,* an alginate hydrogel. In some embodiments, the alginate is modified by a cell adhesion peptide, *e.g.,* RGD. In some embodiments, the polymer, *e.g.,* an alginate, such as an alginate hydrogel, degrades over time, *e.g., in vivo.*

In some embodiments, the drug delivery devices, *e.g.,* hydrogels, comprise a biocompatible material. This biocompatible material is non-toxic or non-immunogenic. The drug delivery devices, *e.g.,* hydrogels, are bio-degradable/erodible in the body. In some embodiments, the drug delivery device, *e.g.,* hydrogel, degrades at a predetermined rate based on a physical parameter selected from the group consisting of temperature, pH, hydration status, and porosity, the cross-link density, type, and chemistry or the susceptibility of main chain linkages to degradation or it degrades at a predetermined rate based on a ratio of chemical polymers. For example, a high molecular weight polymer comprised solely of lactide degrades over a period of years, *e.g.,* 1-2 years, while a low molecular weight polymer comprised of a 50:50 mixture of lactide and glycolide degrades in a matter of weeks, *e.g.,* 1, 2, 3, 4, 6, 10 weeks. A calcium cross-linked gel composed of high molecular weight, high guluronic acid alginate degrade over several months (1, 2, 4, 6, 8, 10, 12 months) to years (1, 2, 5 years) *in vivo,* while a gel comprised of low molecular weight alginate, and/or alginate that has been partially oxidized, will degrade in a matter of weeks. In some embodiments, the drug delivery devices, *e.g.,* hydrogels, comprise a non-biodegradable material. Exemplary non-biodegradable materials include, but are not limited to, metal, plastic polymer, or silk polymer.

In some embodiments, the polymer of the drug delivery device has a higher molecular weight (MW) than the polymer of the refill. For example, the polymer of the drug delivery device comprises more than one strand of a polymer, *e.g.,* alginate. In some embodiments, the polymer, *e.g.,* alginate, strands are cross-linked. For example, the polymer is cross-linked by a cation, *e.g.,* a divalent or trivalent cation, such as Ca²⁺.

Alginates are versatile polysaccharide based polymers that may be formulated for specific applications by controlling the molecular weight, rate of degradation and method of scaffold formation. Alginate molecules are comprised of (1-4)-linked β-D-mannuronic acid (M units) and α L-guluronic acid (G units) monomers, which can vary in proportion and sequential distribution along the polymer chain. Coupling reactions can be used to covalently attach bioactive epitopes, such as the cell adhesion sequence RGD to the polymer backbone. Alginate polymers are formed into a variety of hydrogel types. Injectable hydrogels can be formed from low molecular weight (MW) alginate solutions upon addition of a cross-linking agents, such as calcium ions, while macroporous hydrogels are formed by lyophilization of high MW alginate discs. In some embodiments, the polymers, *e.g.,* alginates, of the drug delivery device, *e.g.,* hydrogel, are 0-100% crosslinked, *e.g.,* at least 1%, 5%, 10%, 20%, 30%, 40%, 50%, 60%, 70%, 80%, 90%, or more crosslinked. In other embodiments, the polymers, *e.g.,* alginates, of the drug delivery device, *e.g.,* hydrogel, are not crosslinked. In some examples, the polymers, *e.g.,* alginates, of the drug delivery device, *e.g.,* hydrogel, contain less than 50%, *e.g.,* less than 50%, 40%, 30%, 20%, 10%, 50%, 2%, 1%, or less, crosslinking. The RGD-modified alginate structure discloses "GGGGRGDSP" as SEQ ID NO: 26.

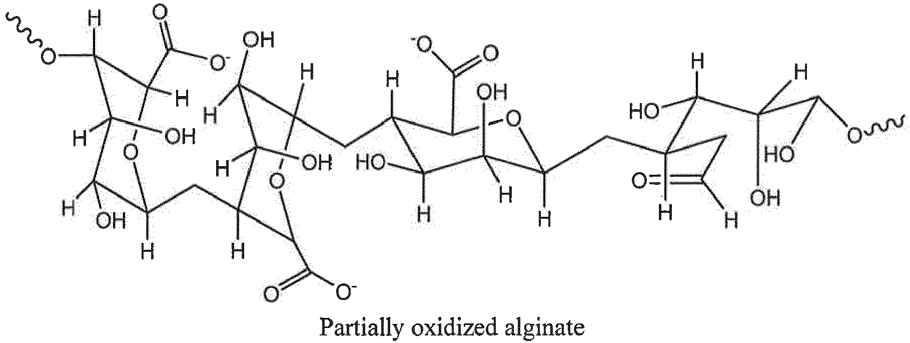

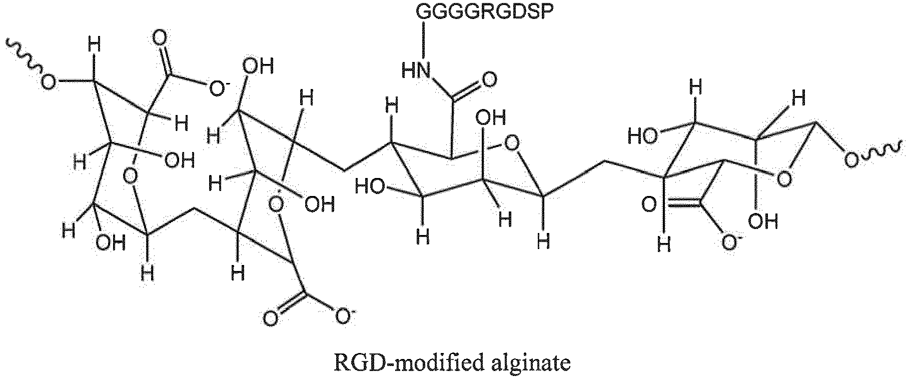

Differences in hydrogel formulation control the kinetics of hydrogel degradation. Release rates of pharmaceutical compositions, *e.g.,* small molecules, morphogens, or other bioactive substances, from alginate hydrogels is controlled by hydrogel formulation to present the pharmaceutical compositions in a spatially and temporally controlled manner. This controlled release eliminates systemic side effects and the need for multiple injections.

In some embodiments, the drug delivery device, *e.g.,* hydrogel, comprises polymers that are modified, *e.g.,* sites on the polymer molecule are modified with a methacrylic acid group (methacrylate (MA)) or an acrylic acid group (acrylate). Exemplary modified hydrogels are MA-alginate (methacrylated alginate) or MA-gelatin. In the case of MA-alginate or MA-gelatin, 50% corresponds to the degree of methacrylation of alginate or gelatin. This means that every other repeat unit contains a methacrylated group. The degree of methacrylation can be varied from 1% to 90%. Above 90%, the chemical modification may reduce solubility of the polymer water-solubility.

Polymers for use in the drug delivery devices of the present invention can also be modified with acrylated groups instead of methacrylated groups. The product would then be referred to as an acrylated-polymer. The degree of methacrylation (or acrylation) can be varied for most polymers. However, some polymers (*e.g.* PEG) maintain their water-solubility properties even at 100% chemical modification. After crosslinking, polymers normally reach near complete methacrylate group conversion indicating approximately 100% of cross-linking efficiency. For example, the polymers in the hydrogel are 50-100% crosslinked (covalent bonds). The extent of crosslinking correlates with the durability of the hydrogel. Thus, a high level of crosslinking (90-100%) of the modified polymers is desirable.

An exemplary drug delivery device of the present invention utilizes an alginate or other polysaccharide of a relatively low molecular weight, preferably of size which, after dissolution, is at the renal threshold for clearance by humans, *e.g.,* the alginate or polysaccharide is reduced to a molecular weight of 1000 to 80,000 Daltons. Preferably, the molecular mass is 1000 to 60,000 Daltons, particularly preferably 1000 to 50,000 Daltons. It is also useful to use an alginate material of high guluronate content since the guluronate units, as opposed to the mannuronate units, provide sites for ionic crosslinking through divalent cations to gel the polymer. Methods for making and using polymers containing polysaccharides such as alginates or modified alginates are known in the art. U.S. Patent Number 6,642,363.

In some embodiments, the drug delivery device comprises a macroporous polylactide-co-glycolide (PLG). PLG matrices release an encapsulated pharmaceutical composition from the drug delivery device, *e.g.,* hydrogel, gradually over the next days or weeks after administration to the site in or on the subject to be treated. For example, release is approximately 60% of the pharmaceutical composition load within the first 5 days, followed by slow and sustained release of the pharmaceutical composition over the next 10 days. This release profile mediates a rate of diffusion of the pharmaceutical composition to and/or through the surrounding tissue.

Other useful polysaccharides used in the drug delivery devices of the present invention may include agarose and microbial polysaccharides such as those listed below.

**Polysaccharide Hydrogel Materials**

| Polymers^{a} | Structure |
|---|---|
| Fungal | |
| Pullulan (N) | 1,4-;1,6-α-D-Glucan |
| Scleroglucan (N) | 1,3;1,6-α-D-Glucan |
| Chitin (N) | 1,4-β-D-Acetyl Glucosamine |
| Chitosan (C) | 1,4-β.-D-N-Glucosamine |
| Elsinan (N) Bacterial | 1,4-;1,3-α-D-Glucan |
| Xanthan gum (A) | 1,4-β.-D-Glucan with D-mannose; |
| | D-glucuronic |
| | Acid as side groups |
| Curdlan (N) | 1,3-β.-D-Glucan (with branching) |
| Dextran (N) | 1,6-α-D-Glucan with some 1,2;1,3-; |
| | 1,4-α-linkages |
| Gellan (A) | 1,4-β.-D-Glucan with rhamose, |
| | D-glucuronic acid |
| Levan (N) | 2,6-β-D-Fructan with some |
| | β-2,1-branching |
| Emulsan (A) | Lipoheteropolysaccharide |
| Cellulose (N) | 1,4- β-D-Glucan |

| | |
|---|---|
| ^{a} N-neutral, A = anionic and C=cationic. | |

In some embodiments, the drug delivery device, *e.g.,* hydrogel, of the present invention comprises one or more compartments.

The drug delivery devices, *e.g.,* hydrogels, of the present invention may comprise an external surface. Alternatively, or in addition, the drug delivery devices, *e.g.,* hydrogels, may comprise an internal surface. External or internal surfaces of the drug delivery device of the present invention may be solid or porous. Pore size of the drug delivery devices can be less than about 10 nm, between about 100 nm-20 µm, or greater than about 20 µm, *e.g.,* up to and including 1000 µm in diameter. For example, the pores may be nanoporous, microporous, or macroporous. For example, the diameter of nanopores are less than about 10 nm; micropore are in the range of about 100 µm-20 µm in diameter; and, macropores are greater than about 20 µm (preferably greater than about 100 µm and even more preferably greater than about 400 µm, *e.g.,* greater than 600 µm or greater than 800 µm). In one example, the drug delivery device, *e.g.,* hydrogel, is macroporous with open, interconnected pores of about 100-500 µm in diameter, e.g., 100-200, 200-400, or 400-500 µm.

In some embodiments, the drug delivery devices of the present invention are organized in a variety of geometric shapes (*e.g.,* discs, beads, pellets), niches, planar layers *(e.g.,* thin sheets). For example, discs of about 0.1-200 millimeters in diameter, *e.g.,* 5, 10, 20, 40, 50 millimeters may be implanted subcutaneously. The disc may have a thickness of 0.1 to 10 millimeters, *e.g.,* 1, 2, 5 millimeters. The discs are readily compressed or lyophilized for administration to a patient. An exemplary disc for subcutaneous administration has the following dimensions: 8 millimeters in diameter and 1 millimeter in ' thickness. In some embodiments, the drug delivery device may comprise multiple components. Multicomponent drug delivery devices are optionally constructed in concentric layers each of which is characterized by different physical qualities such as the percentage of polymer, the percentage of crosslinking of polymer, chemical composition of the hydrogel, pore size, porosity, and pore architecture, stiffness, toughness, ductility, viscoelasticity, and/or pharmaceutical composition.

A method of making a drug delivery device, *e.g.,* hydrogel, is carried out by providing a hydrogel material and covalently linking or noncovalently associating the hydrogel material with a pharmaceutical composition. Exemplary drug delivery devices and methods of making them are described in US 2012/0100182, PCT/US2010/057630, and PCT/US2012/35505.

The drug delivery devices of the present invention may be assembled *in vivo* in several ways. The hydrogel may be made from a gelling material, which is introduced into the body in its ungelled form where it gels *in situ.* Exemplary methods of delivering drug delivery device components to a site at which assembly occurs include injection through a needle or other extrusion tool, spraying, painting, or methods of deposit at a tissue site, *e.g.,* delivery using an application device inserted through a cannula. In one example, the ungelled or unformed hydrogel material is mixed with pharmaceutical compositions prior to introduction into the body or while it is introduced. The resultant *in vivo*/*in situ* assembled device, *e.g.,* hydrogel, contains a mixture of these pharmaceutical composition(s).

*In situ* assembly of the drug delivery device, *e.g.,* hydrogel, occurs as a result of spontaneous association of polymers or from synergistically or chemically catalyzed polymerization. Synergistic or chemical catalysis is initiated by a number of endogenous factors or conditions at or near the assembly site, *e.g.,* body temperature, ions or pH in the body, or by exogenous factors or conditions supplied by the operator to the assembly site, *e.g.,* photons, heat, electrical, sound, or other radiation directed at the ungelled material after it has been introduced. The energy is directed at the hydrogel material by a radiation beam or through a heat or light conductor, such as a wire or fiber optic cable or an ultrasonic transducer. Alternatively, a shear-thinning material, such as an ampliphile, is used which re-cross links after the shear force exerted upon it, for example by its passage through a needle, has been relieved.

In some embodiments, the drug delivery device, *e.g.,* hydrogel of the present invention is injectable. For example, the drug delivery devices, *e.g.,* hydrogels are created outside of the body as macroporous scaffolds. The hydrogels can be injected into the body because the pores collapse and the gel becomes very small and can fit through a needle. See, *e.g.,* WO 12/149358; and Bencherif et al. Proc. Natl. Acad. Sci. USA 109.48(2012):19590-5.

In some embodiments, the drug delivery device, *e.g.,* hydrogel of the present invention comprises multiple compartments. A multiple compartment drug delivery device is assembled *in vivo* by applying sequential layers of similarly or differentially doped gel or other scaffold material to the target site. Non-concentric compartments are formed by injecting material into different locations in a previously injected layer. A multi-headed injection device extrudes compartments in parallel and simultaneously. The layers are made of similar or different hydrogel compositions differentially doped with pharmaceutical compositions. Alternatively, compartments self-organize based on their hydro-philic/phobic characteristics or on secondary interactions within each compartment.

In certain embodiments, a drug delivery device containing compartments with distinct chemical and/or physical properties is provided. A compartmentalized drug delivery device is designed and fabricated using different compositions or concentrations of compositions for each compartment. Alternatively, the compartments are fabricated individually, and then adhered to each other *(e.g.,* a "sandwich" with an inner compartment surrounded on one or all sides with the second compartment). The compartments self-assemble and interface appropriately, either *in vitro* or *in vivo,* depending on the presence of appropriate precursors (*e.g*., temperature sensitive oligopeptides, ionic strength sensitive oligopeptides, block polymers, cross-linkers and polymer chains (or combinations thereof).

Alternatively, the compartmentalized drug delivery device is formed using a printing technology. Successive layers of a hydrogel precursor doped with pharmaceutical compositions is placed on a substrate then cross linked, for example by self-assembling chemistries. When the cross linking is controlled by chemical-, photo- or heat-catalyzed polymerization, the thickness and pattern of each layer is controlled by a masque, allowing complex three dimensional patterns to be built up when un-cross-linked precursor material is washed away after each catalyzation. (WT Brinkman et al., 2003. Biomacromolecules 4(4): 890-895.; W. Ryu et al., 2007 Biomaterials 28(6): 1174-1184; Wright, Paul K. (2001). 21st Century manufacturing. New Jersey: Prentice-Hall Inc.) Complex, multi-compartment layers are also built up using an inkjet device which "paints" different doped-scaffold precursors on different areas of the substrate. C. Choi, New Scientist, 25, 2003, v2379. These layers are built-up into complex, three dimensional compartments. The drug delivery device may also be built using any of the following methods: jetted photopolymer, selective laser sintering, laminated object manufacturing, fused deposition modeling, single jet inkjet, three dimensional printing, or laminated object manufacturing.

### B. Drug Refills

The drug refills of the present invention feature a dual functionality, such that the drug refills, not only permit a direct delivery of a pharmaceutical composition to the drug delivery devices, but also mask the potential toxicity of pharmaceutical composition, e.g., a chemo therapeutic for cancer treatment, until the pharmaceutical composition reaches the drug delivery device at the desired location.

The drug refills of the present invention comprise a pharmaceutical composition and a target, wherein the target is capable of binding to a target recognition moiety on the drug delivery device, wherein the drug refill is mobile until the target on the drug refill binds to the target recognition moiety on the drug delivery device. Upon binding of the target to the target recognition moiety, the drug refill delivers the pharmaceutical composition directly to the drug delivery device, thereby refilling the drug delivery device.

In some embodiments, the drug refill further comprises a nanocarrier. Exemplary nanocarriers may include, but not limited to, a polymer, a nanoparticle, a liposome nanoparticle, a dendrimer, a carbon nanotube, a micelle, a protein (*e.g.,* albumin), a silica, or a metal (*e.g.,* gold, silver, or iron) nanoparticle. In some embodiments, the polymer is selected from the group consisting of collagen, alginate, polysaccharides, hyaluronic acid (HA), polyethylene glycol (PEG), poly(glycolide) (PGA), poly(L-lactide) (PLA), or poly(lactide-co-glycolide) (PLGA), and poly lactic-coglycolic acid. In other embodiments, the polymer comprises a strand of a polymer. In yet another embodiment, the polymer is a free alginate strand, *i.e.,* the alginate strand does not cross-link to other alginate strands. In some embodiments, the free alginate strand, which is linked to a pharmaceutical composition and a target, fuses with the drug delivery device, *e.g.,* an alginate hydrogel, resulting in the direct delivery of the pharmaceutical composition from the drug refill to the drug delivery device. In some embodiments, the polymer in the drug refill comprises alginate, *e.g.,* modified by a cell adhesion peptide, *e.g.,* RGD.

In some embodiments, the polymer in the drug refill, *e.g.,* comprising alginate, has a MW of 1000 kDa or less, *e.g.,* 1000 kDa, 900 kDa, 800 kDa, 700 kDa, 600 kDa, 500 kDa, 400 kDa, 350 kDa, 325 kDa, 300 kDa, 290 kDa, 285 kDa, 280 kDa, 275, kDa, 270 kDa, 260 kDa, 250 kDa, 240 kDa, 230 kDa, 220 kDa, 210 kDa, 200 kDa, 180 kDa, 160 kDa, 150 kDa, 140 kDa, 120 kDa, 100 kDa, or less.

In other embodiments, the polymer in the drug refill, *e.g.,* comprising alginate, has a hydrodynamic radius (Rh) of 200 nm or less, *e.g.,* 200 nm, 180 nm, 160 nm, 140 nm, 120 nm, 100 nm, 90 nm, 80 nm, 70 nm, 60 nm, 50 nm, 44 nm, 40 nm, 35 nm, 30 nm, 25 nm, 20 nm, 17 nm, 15 nm, 12 nm, 10 nm, or less. The hydrodynamic radius is measured by using standard methods in the art.

The drug refills of the present invention are capable of circulating in the blood for sufficiently long periods of time and are capable of controlled drug delivery and blood-based targeting. In some embodiment, the drug refills comprise an alginate. Alginate has previously been reported to have long circulation times in the blood, being detectable in serum for at least one week as well as having PEG-like properties in its ability to increase the circulation time of nanoparticles following conjugation. Al-Shamkhani A & Duncan R (1995) Journal of bioactive and compatible polymers 10(1):4-13; and Kodiyan A, et al. (2012) ACS nano 6(6):4796-4805. The circulation lifetime primarily depends on the rate of excretion into the urine, uptake by the mononuclear phagocyte system in the liver and spleen, and drug stability. For example, alginate having a MW of 280 kDa (unoxidized) or 200 KDa (5% oxidized) and a hydrodynamic radius of 44 nm and 17 nm, respectively, will behave similarly to nanoparticles, and will enhance drug circulation time and reduce drug permeability in off-target tissues. Free alginate strands have a blood circulation lifetime of about two weeks. Imaging of individual organs revealed accumulation in the lungs, consistent with many nanoparticles, as well as a tendency to undergo significant clearance into the MPS-related organs, and to a lesser extent in the kidneys (Fig 6D), demonstrating that all of these organs contribute to alginate clearance.

In some embodiments, the pharmaceutical composition, *e.g.,* in the drug delivery device and/or refill, comprises a small molecule or a biologic. A biologic is a medicinal product manufactured in, extracted from, or semisyntehsized from biological sources which is different from chemically synthesized pharmaceuticals. In some embodiments, biologics used in the present invention may include, but not limited to antibodies, vaccines, blood, or blood components, allergenics, somatic cells, gene therapies, tissues, recombinant therapeutic protein, and living cells used in cell therapy. Biologics can be composed of sugars, proteins, or nucleic acids or complex combinations of these substances, or may be living cells or tissues, and they can be isolated from natural sources such as human, animal, or microorganism.

The pharmaceutical composition is attached to the target via a cleavable linker. Examples of cleavable linkers include a hydrolysable linker, a pH cleavage linker, an enzyme cleavable linker, or disulfide bonds that are cleaved through reduction by free thiols and other reducing agents; peptide bonds that are cleaved through the action of proteases and peptidase; nucleic acid bonds cleaved through the action of nucleases; esters that are cleaved through hydrolysis either by enzymes or through the action of water in vivo; hydrazones, acetals, ketals, oximes, imine, aminals and similar groups that are cleaved through hydrolysis in the body; photo-cleavable bonds that are cleaved by the exposure to a specific wavelength of light; mechano-sensitive groups that are cleaved through the application of ultrasound or a mechanical strain (*e.g.,* a mechanical strain created by a magnetic field on a magneto-responsive gel). For example, drugs targeted to a specific location *in vivo* may be released by an external stimuli such as a magnetic field or light. See, *e.g.,* Cezar et al. Advanced Healthcare Materials (2014); Zhao et al. Proc. Natl. Acad. Sci. USA 108(2011):67; Mura et al. Nat. Materials 12(2013):991. Upon cleavage of a bond between the pharmaceutical composition and the cleavable linker, a bond within the cleavable linker, or a bond between the pharmaceutical composition and the target, the pharmaceutical composition can be released from the drug delivery device to the desired location within a subject.

In some embodiments, the pharmaceutical composition is connected via a cleavable linkerto a nanocarrier in the drug refill. In some embodiments, the pharmaceutical composition is connected via a cleavable linkerto a polymer in the drug delivery device. Exemplary cleavable linkers are as described above. In some embodiments, the polymer (*e.g.,* alginate) is unoxidized or partially oxidized by 1%, 2%, 3%, 4%, 5%, 6%, 7%, 8%, 9%, 10%, 15%, 20% or more. For example the polymer is oxidized in order to introduce functional groups, such as aldehyde functional group for use in coupling of the polymer to a pharmaceutical composition.

In some embodiments, the pharmaceutical composition for use in the present invention may be toxic, *e.g.,* has undesired toxicity, and the drug refills of the present invention mask the toxicity of the pharmaceutical composition when administered into a subject. In some embodiments, the drug refills may mask the toxicity of the pharmaceutical composition by preventing the pharmaceutical composition from crossing the cell membrane. In other embodiments, the drug refills may mask the toxicity of the pharmaceutical composition by preventing the pharmaceutical composition from binding to the biological target of the pharmaceutical composition. In some embodiments, at least 10%, 20%, 30%, 40%, 50%, 55%, 60%, 65%, 70%, 75%, 80%, 85%, 90%, 95% or 100% of the toxicity of the pharmaceutical composition is masked by the drug refill. For example, when a chemotherapeutic targeting a tumor site is being delivered, the potential toxicity of the chemotherapeutic may cause damage to sites other than the desired tumor site, leading to unnecessary systematic toxicity. The drug delivery systems of the present invention allow for more efficient targeting of disease sites, and at the same time, minimize the potential toxicity of the drugs to be delivered through the development of the dual functionality drug refills. The drug refills of the present invention, not only permit a direct delivery of pharmaceutical composition to the drug delivery device, but also mask the potential toxicity of pharmaceutical composition to be delivered until the pharmaceutical composition reaches the drug delivery device at the desired location.

The pharmaceutical composition will only be unmasked upon delivery into the drug delivery device when the target is separated from the pharmaceutical composition, thus eliminating any side effects or toxicity associated with the pharmaceutical composition at any undesired sites. In some embodiments, the pharmaceutical composition may be delivered via fusion between the drug refill and the drug delivery device. For example, when the nanocarrier of the drug refill comprises a free polymer, *e.g.,* a free alginate strand, the free alginate strand, which is linked to a pharmaceutical composition and a target, may fuse with the drug delivery device, *e.g.,* an alginate hydrogel, which is linked to a target recognition moiety, upon interaction between the target and the target recognition moiety, thereby allowing the direct delivery of the pharmaceutical composition from the drug refill to the drug delivery device. In other embodiments, the pharmaceutical composition is delivered from the drug refill to the drug delivery device upon interaction between the target and the target recognition moiety inside the drug delivery device. Binding of the target on the drug refill to the target recognition moiety may occur inside or outside the drug delivery device.

The target in the drug refill of the present invention may be separated from the pharmaceutical composition by cleaving a bond between the pharmaceutical composition and the cleavable linker, a bond within the cleavable linker, or a bond between the pharmaceutical composition and the target. In some embodiments, the bond between the pharmaceutical composition and the cleavable linker, the bond within the cleavable linker, or the bond between the pharmaceutical composition and the target is cleaved by an enzymatic degradation of the bonds. In other embodiments, the bond between the pharmaceutical composition and the cleavable linker, the bond within the cleavable linker, or the bond between the pharmaceutical composition and the target is cleaved by hydrolysis of the bonds. In yet another embodiments, the bond between the pharmaceutical composition and linker, the bond within the cleavable linker, or the bond between the pharmaceutical composition and the target is cleaved by reduction of the bonds. In some embodiments, the bond between the pharmaceutical composition and the cleavable linker comprises a bond selected from the group consisting of a hydrazine bond, an acetal bond, a ketal bond, an oxime bond, an imine bond, and an aminal bond.

Another aspect of the present invention provides a system comprising a drug delivery device and a drug refill, wherein the drug delivery device comprises a carrier and a target recognition moiety and is suitable for implantation in a desirable location within a subject; wherein the drug refill comprises a pharmaceutical composition and a target; wherein the pharmaceutical composition is attached to the target via a cleavable linker capable of being cleaved within the subject, wherein the pharmaceutical composition has undesired toxicity and the drug refill masks the toxicity of the pharmaceutical composition, wherein the target and the target recognition moiety form a two-component binding pair; wherein the drug refill is mobile until the target on the drug refill binds to the target recognition moiety on the drug delivery device, wherein upon binding of the target to the target recognition moiety, the drug refill delivers the pharmaceutical composition directly to the drug delivery device, and wherein the pharmaceutical composition is released in a controlled manner from the drug delivery device to the desirable location within the subject.

Unlike the existing drug delivery systems which usually mediate delivery of pharmaceutical composition within minutes, the drug delivery systems of the present invention provide a more sustained release of pharmaceutical composition over a time scale of days, weeks, months or years. As a result, controlled release of the pharmaceutical composition from the drug delivery device can minimize potential side effects of the pharmaceutical composition while still maintaining the efficacy of the pharmaceutical composition.

In some embodiments, the pharmaceutical composition for use in the present invention is released by cleaving a bond between the pharmaceutical composition and the linker, a bond within the cleavable linker, or a bond between the pharmaceutical composition and the target.

In other embodiments, the bond between the pharmaceutical composition and linker, the bond within the cleavable linker, or the bond between the pharmaceutical composition and the target is cleaved by enzyme degradation, hydrolysis or reduction of the bond. In some embodiments, the bond between the pharmaceutical composition and the cleavable linker comprises a bond selected from the group consisting of a hydrazine bond, an acetal bond, a ketal bond, an oxime bond, an imine bond, and an aminal bond. In some embodiments, the pharmaceutical composition is not released from the drug refill to the desired location within a subject. In some embodiments, at least 50%, 55%, 60%, 65%, 70%, 75%, 80%, 85%, 90%, 95% or 100% of the pharmaceutical composition is released from the drug delivery device to the desired location within a subject.

The rate of releasing pharmaceutical composition from the drug delivery device to the desired location depends on a variety of factors including, but not limited to the pH, temperature, hydration status, ion concentration of the environment. In some embodiments, the pharmaceutical composition is released at a faster rate when the surrounding pH is lower, while the rate decreases as the pH increases. As a result, one of ordinary skill in the art would be able to regulate or control the rate of drug release by fine-tuning the pH of the solution. If a slow release of pharmaceutical composition from the drug delivery device is preferred, then the pH of the solution can be increased. Alternatively, a lower pH of the solution will result in a faster release of the pharmaceutical composition from the drug delivery device.

The drug delivery systems of the present invention provide highly selective targeting of drug refills to specific sites in the body, *e.g.,* a previously administered (*e.g.,* stationary) drug delivery device in the subject. Specific recognition between the drug delivery devices and drug refills is facilitated by the interaction between the target and the target recognition moiety. For example, at least 2%, *e.g.,* at least 2%, 4%, 6%, 8%, 10%, 15%, 20%, 30%, 40%, 50%, 60%, 70%, 80%, 90%, 95% or greater, of the drug refills (*e.g.,* comprising pharmaceutical composition(s)) administered to a subject travel to and are deposited at a specific drug delivery device, e.g., one that comprises a target recognition moiety that specifically binds to the target on the drug refill. Less than 98%, *e.g.,* less than 98%, 95%, 90%, 85%, 80%, 70%, 60%, 50%, 40%, 30%, 20%, 15%, 10% or 5% of the drug refills (*e.g.,* comprising pharmaceutical composition(s)) administered to a subject are deposited at a site other than a drug delivery device to which the target on the drug refill is targeted.

In some embodiments, the target and/or target recognition moiety comprises a nucleic acid, peptide, polysaccharide, lipid, small molecule, or combination thereof. In some embodiments, the target and the target recognition moiety comprise a nucleic acid. For example, the target on the drug refill comprises a nucleic acid sequence that is complementary to the nucleic acid sequence of the target recognition moiety on the drug delivery device. In some embodiments, the nucleic acid comprises DNA, RNA, modified DNA, modified RNA, locked nucleic acids (LNAs), peptide nucleic acids (PNAs) or, morpholinos. In some embodiments, the nucleic acid comprises siRNA, RNAi, mRNA and microRNA. In other examples, the nucleic acid comprises DNA or modified DNA.

DNA complementarity has been widely exploited to direct specific chemical interactions, including formation of complex nanostructures, surface functionalization, and nanoparticle and cell assemblies. See, *e.g.,* Douglas S, et al. (2009) Nature 459(7245):414-418; Wei B, Dai M, & Yin P, (2012) Nature 485(7400):623-626; Onoe H, et al. (2012) Langmuir 28(21):8120-8126; Gartner Z & Bertozzi C (2009) Proceedings of the National Academy of Sciences of the United States of America 106(12):4606-4610; Zhang Y, et al., (2013) Nature nanotechnology 8(11):865-872. The utility of DNA in these applications lies in the sequence specificity and strength of binding as well as the ease to regulate the interaction strength. One challenge to this type of application is DNA stability. In some embodiments, a nanocarrier, e.g., an alginate strand, is conjugated to the 3' end of DNA to overcome 3'-5' exonucleases (the major serum-based nuclease). See, *e.g.,* Shaw J et al., (1991) Nucleic acids research 19(4):747-750; Floege J, et al. (1999) The American journal of pathology 154(1):169-179; Gamper H, et al. (1993) Nucleic acids research 21(1):145-150. In some embodiments, the backbone of the nucleic acid is modified with phosphorothioate groups for increased endonuclease and chemical stability. See Campbell J, et al., (1990) Journal of biochemical and biophysical methods 20(3):259-267. The results presented herein demonstrate that DNA-mediated drug, *e.g.,* doxorubicin, refilling of the drug delivery device, *e.g.,* alginate hydrogels, dramatically inhibited tumor growth in a xenograft tumor model. The drug delivery system of the present invention can overcome toxicity related to the non-specific targeting of all permeable tissues.

In some embodiments, the drug refill comprises a pharmaceutical composition, a target and optionally a nanocarrier with the following connectivity (where "----"indicates a linkage, *e.g.,* a linker, a bond or plurality of bonds):
1) Target molecule (*e.g.,* TCO/DBCO/norbornene/DNA)----nanocarrier-pharmaceutical composition
2) Target molecule----pharmaceutical composition---nanocarrier
3) Nanocarrier----target molecule----pharmaceutical composition
4) Target molecule----pharmaceutical composition (with no nanocarrier).
In some examples, a target molecule linked directly to a pharmaceutical composition (without a nanocarrier) is useful as a refill to increase tissue penetration and is suitable for oral administration (*e.g.,* in pill form). In other examples, a refill comprising a nanocarrier is useful for cancer treatment applications.

In some embodiments, a linkage between a target molecule, nanocarrier, and/or pharmaceutical composition comprises a covalent bond/linker such as an amide, ester, hydrazide, ether, thioether, alkyl, peg linker, ketone, or amine. In other cases, the linkage comprises a noncovalent or ionic bond. In other examples, the linkage comprises a cleavable linker, such as a hydrolysable linker, a pH cleavable linker, an enzyme cleavable linker, or any one of the exemplary cleavable linkers described herein. For example, the polymer of the drug refill, *e.g.,* an alginate strand, may be conjugated to the target, *e.g.,* nucleic acid, at the 3'end, the 5' end or in the middle of the nucleic acid. In another example, the polymer of the drug refill, *e.g.,,* an alginate strand, is conjugated to the 3' end of the nucleic acid molecule, *e.g.,* DNA. Upon cleavage of the bond between the target and the pharmaceutical composition, the pharmaceutical composition will be released directly from the drug delivery device into the desired site.

In some embodiments, the target on the drug refill comprises one or more nucleotides that are complementary to one or more nucleotides of the target recognition moiety on the drug delivery device. For example, 50% or more (*e.g.,* 50%, 60%, 70%, 80%, 90%, 95%, or 100%) of the nucleotides of the target on the drug refill are complementary to 50% or more *(e.g.,* 50%, 60%, 70%, 80%, 90%, 95%, or 100%) of the nucleotides of the target recognition moiety on the drug delivery device.

In some examples, the melting temperature (Tm) of the target:target recognition moiety hybridization is at least 40°C, *e.g.,* at least 40 °C, 45 °C, 50 °C, 55 °C, 60 °C, 65 °C, 70°C, 75 °C, 80°C, 85 °C, 90 °C, 95 °C, or 100 °C.

Exemplary nucleic acids that can be used as a target on the drug refill and a target recognition moiety on the drug delivery device may include, but not limited to, DNA, modified DNA, RNA, modified RNA, locked nucleic acids (LNAs), peptide nucleic acids (PNAs), threose nucleic acids (TNA), hexitol nucleic acids (HNAs), bridge nucleic acids, cyclohexenyl nucleic acids, glycerol nucleic acids, morpholinos, phosphomorpholinos, as well as aptamers and catalytic nucleic acid versions thereof. For example, the nucleic acid contains a modified nitrogenous base. For example, modified DNA and RNA include 2'-o-methyl DNA, 2'-o-methyl RNA, 2'-fluoro-DNA, 2'-fluoro-RNA, 2'-methoxy-purine, 2'-fluoro-pyrimidine, 2'-methoxymethyl-DNA, 2'-methoxymethyl-RNA, 2'-acrylamido-DNA, 2'-acrylamido-RNA, 2'-ethanol-DNA, 2'-ethanol-RNA, 2'-methanol-DNA, 2'-methanol-RNA, and combinations thereof. In some cases, the nucleic acid contains a phosphate backbone. In other embodiments, the nucleic acid contains a modified backbone, *e.g.,* a phosphorothioate backbone, phosphoroborate backbone, methyl phosphonate backbone, phosphoroselenoate backbone, or phosphoroamidate backbone.

In some embodiments, the target, *e.g.,* nucleic acids, on the drug refill and the target recognition moiety, *e.g.,* nucleic acids, on the drug delivery device are single-stranded. In other embodiments, the target, *e.g.,* nucleic acids, on the drug refill and the target recognition moiety, *e.g.,* nucleic acids, on the drug delivery device are partially single-stranded and partially double-stranded, *e.g.,* due to secondary structures, such as hairpins.

In some embodiments, the target, *e.g.,* nucleic acids, on the drug refill and/or the target recognition moiety, *e.g.,* nucleic acids, on the drug delivery device comprise 8-50 nucleotides, *e.g.,* 8, 10, 12, 14, 16, 18, 20, 22, 24, 26, 28, 30, 32, 34, 36, 38, 40, 42, 44, 46, 48, or 50 nucleotides. In other embodiments, the target, e.g., nucleic acids, on the drug refill and/or the target recognition moiety, *e.g.,* nucleic acids, on the drug delivery device comprise 20 nucleotides.

In some embodiments, the target on the drug refill comprises a phosphorothioate nucleic acid molecule having the sequence of TTTTTTTTTTTTTTTTTTTT (SEQ ID NO: 1), also called (T)₂₀. In other embodiments, the target on the drug refill comprises a phosphorothioate nucleic acid molecule having the sequence of AAAAAAAAAAAAAAAAAAAA (SEQ ID NO: 2), also called (A)₂₀.

In some embodiments, the target recognition moiety on the drug delivery device comprises a phosphorothioate nucleic acid molecule having the sequence of TTTTTTTTTTTTTTTTTTTT (SEQ ID NO: 1), also called (T)₂₀. In other embodiments, the target recognition moiety on the drug delivery device comprises a phosphorothioate nucleic acid molecule having the sequence of AAAAAAAAAAAAAAAAAAAA (SEQ ID NO: 2), also called (A)₂₀.

In some embodiments, the targeting agent and/or the homing agent comprises a nucleic acid molecule, e.g., a phosphorothioate nucleic acid molecule, having the sequence of a nucleotide sequence in Table 1.

**Table 1: Exemplary Nucleic Acid Sequences, shown 3'-5'. Italicized stands for phosphorothioate linkages. FAM stands for 5' 6-fluorescein. Hex stands for 5' Hexachlorofluorescein. SH stands for C3 3' thiol.**

| Oligo Name | Oligo Sequence 3' to 5' | SEQ ID NO: |
|---|---|---|
| polyT-SH | SH-TTTTTTTTTTTTTTTTTTTT | 3 |
| polyA-SH | SH-AAAAAAAAAAAAAAAAAAAA | 4 |
| polyT-F | TTTTTTTTTTTTTTTTTTTT-FAM | 5 |
| polyA-F | AAAAAAAAAAAAAAAAAAAA-FAM | 6 |
| polyT-SH/Hex | SH-TTTTTTTTTTTTTTTTTTTT-HEX | 7 |
| thioT-SH | SH-*TTTTTTTTTTTTTTTTTTTT* | 8 |
| thioA-SH | *SH-AAAAAAAAAAAAAAAAAAAA* | 9 |

Other exemplary nucleic acid:complementary nucleic acid pairs are shown in the table below.

| Sequence (5' to 3') | SEQ ID NO: | Complement (5 ' to 3') | SEQ ID NO: |
|---|---|---|---|
| CATGGAGAGCGACGAGAGC GGC | 10 | | 11 |
| TGTAACTGAGGTAAGAGG | 12 | CCTCTTACCTCAGTTACA | 13 |
| CCCCCCCCCCCCCCCCCCCC | 14 | | 15 |
| ACACACACACACACACACAC ACAC | 16 | | 17 |
| GAATGAATGAATGAATGAAT GAAT | 18 | | 19 |
| GGATTGGATTGATTGGATTG ATTGGATT | 20 | | 21 |
| GTAAAACGACGGCCAGT | 22 | ACTGGCCGTCGTTTTAC | 23 |
| GCTAGTTATTGCTCAGCGG | 24 | | 25 |

In some embodiments, the target on the drug refill comprises biotin or desthiobiotin, and the target recognition moiety on the drug delivery device comprises avidin, neutravidin, streptavidin, or other form of avidin. Alternatively, the target on the drug refill comprises avidin, neutravidin, streptavidin, or other form of avidin and the target recognition moiety on the drug delivery device comprises biotin or desthiobiotin.

In some embodiments, the target on the drug refill comprises a bioorthogonal functional group and the target recognition moiety on the drug delivery device comprises a complementary functional group, where the bioorthogonal functional group is capable of chemically reacting with the complementary functional group to form a covalent bond. By bioorthogonal is meant a functional group or chemical reaction that can occur inside a living cell, tissue, or organism without interfering with native biological or biochemical processes. A bioorthogonal functional group or reaction is not toxic to cells. For example, a bioorthogonal reaction must function in biological conditions, e.g., biological pH, aqueous environments, and temperatures within living organisms or cells. For example, a bioorthogonal reaction must occur rapidly to ensure that covalent ligation between two functional groups occurs before metabolism and/or elimination of one or more of the functional groups from the organism. In other examples, the covalent bond formed between the two functional groups must be inert to biological reactions in living cells, tissues, and organisms.

In some embodiments, the binding of the target on the drug refill to the target recognition moiety occurs inside the drug delivery device. For example, when the drug delivery device, *e.g.,* hydrogel is placed in a tumor site, the target recognition moiety is present throughout the drug delivery device and the drug refill may penetrate inside the drug delivery device via diffusion. In other embodiments, the target on the drug refill binds to the target recognition moiety on the surface of the drug delivery device, *e.g.,* in the pores of the device and/or on the external surface of the device.

In some embodiments, the bioorthogonal reaction that links a drug refill to a drug delivery device is itself reversible, permitting the delivery of the drug through the reversal of the bond used to bind the drug to the target on the drug refill.

Exemplary bioorthogonal functional group/complementary functional group pairs used as the target and the target recognition moiety of the present invention may include, but not limited to, azide with phosphine; azide with cyclooctyne; nitrone with cyclooctyne; nitrile oxide with norbornene; oxanorbornadiene with azide; trans-cyclooctene with s-tetrazine; quadricyclane with bis(dithiobenzil)nickel(II).

In some embodiments, the bioorthogonal functional groups on the drug refills are capable of reacting by click chemistry with the complementary functional groups on the drug delivery devices, *e.g.,* using a reaction type described below. In some embodiments, the bioorthogonal functional group on the drug refill comprises an alkene, *e.g.,* a cyclooctene, *e.g.,* a transcyclooctene (TCO) or norbornene (NOR), and the complementary functional group on the drug delivery device comprises a tetrazine (Tz). In other embodiments, the bioorthogonal functional group on the drug refill comprises an alkyne, *e.g.,* a cyclooctyne such as dibenzocyclooctyne (DBCO), and the complementary functional group on the drug delivery device comprises an azide (Az). In some embodiments, the bioorthogonal functional group on the drug refill comprises a Tz, and the complementary functional group on the drug delivery device comprises an alkene such as transcyclooctene (TCO) or norbornene (NOR). Alternatively or in addition, the bioorthogonal functional group on the drug refill comprises an Az, and the complementary functional group on the drug delivery device comprises a cyclooctyne such as dibenzocyclooctyne (DBCO). TCO reacts specifically in a click chemistry reaction with a tetrazine (Tz) moiety. DBCO reacts specifically in a click chemistry reaction with an azide (Az) moiety. Norbornene reacts specifically in a click chemistry reaction with a tetrazine (Tz) moiety. Exemplary click chemistry reactions for use in the present invention are shown below. For example, copper(I)-catalyzed Azide-Alkyne Cycloaddition (CuAAC) comprises using a Copper (Cu) catalyst at room temperature. The Azide-Alkyne Cycloaddition is a 1,3-dipolar cycloaddition between an azide and a terminal or internal alkyne to give a 1,2,3-triazole.

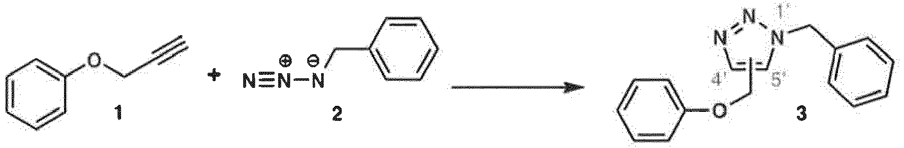

Another example of click chemistry includes Staudinger ligation, which is a reaction that is based on the classic Staudinger reaction of azides with triarylphosphines. It launched the field of bioorthogonal chemistry as the first reaction with completely abiotic functional. The azide acts as a soft electrophile that prefers soft nucleophiles such as phosphines. This is in contrast to most biological nucleophiles which are typically hard nucleophiles. The reaction proceeds selectively under water-tolerant conditions to produce a stable product. Phosphines are completely absent from living systems and do not reduce disulfide bonds despite mild reduction potential. Azides had been shown to be biocompatible in FDA-approved drugs such as azidothymidine and through other uses as cross linkers. Additionally, their small size allows them to be easily incorporated into biomolecules through cellular metabolic pathways

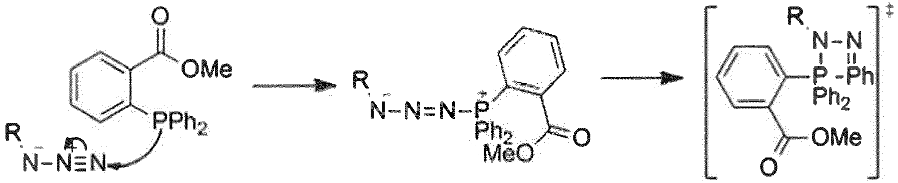

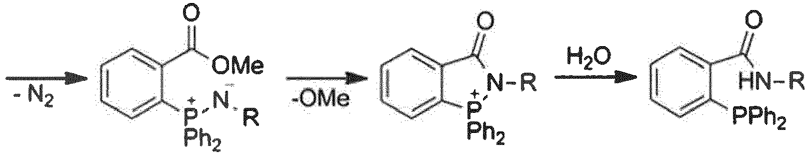

Copper-free click chemistry is a bioorthogonal reaction first developed by Carolyn Bertozzi as an activated variant of an azide alkyne cycloaddition. Unlike CuAAC, Cu-free click chemistry has been modified to be bioorthogonal by eliminating a cytotoxic copper catalyst, allowing reaction to proceed quickly and without live cell toxicity. Instead of copper, the reaction is a strain-promoted alkyne-azide cycloaddition (SPAAC). It was developed as a faster alternative to the Staudinger ligation, with the first generations reacting over sixty times faster. The incredible bioorthogonality of the reaction has allowed the Cu-free click reaction to be applied within cultured cells, live zebrafish, and mice. Cyclooctynes were selected as the smallest stable alkyne ring which increases reactivity through ring strain which has calculated to be 19.9 kcal/mol.

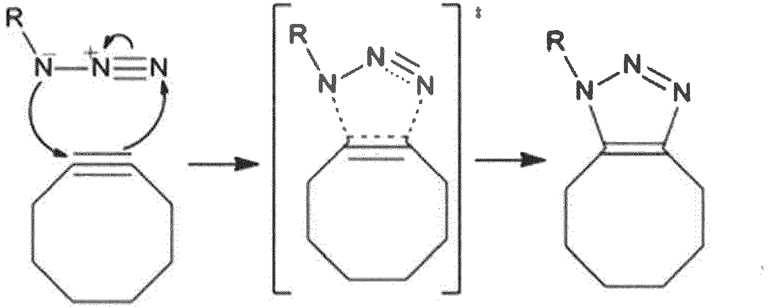

Copper-free click chemistry also includes nitrone dipole cycloaddition. Copper-free click chemistry has been adapted to use nitrones as the 1,3-dipole rather than azides and has been used in the modification of peptides.

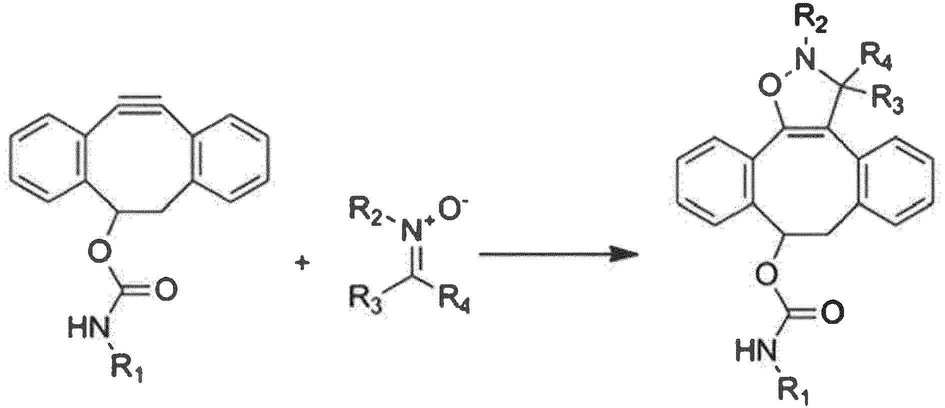

This cycloaddition between a nitrone and a cyclooctyne forms N-alkylated isoxazolines. The reaction rate is enhanced by water and is extremely fast with second order rate constants ranging from 12 to 32 M⁻¹•s⁻¹, depending on the substitution of the nitrone. Although the reaction is extremely fast, incorporating the nitrone into biomolecules through metabolic labeling has only been achieved through post-translational peptide modification.

Another example of click chemistry includes norbornene cycloaddition. 1,3 dipolar cycloadditions have been developed as a bioorthogonal reaction using a nitrile oxide as a 1,3-dipole and a norbornene as a dipolarophile. Its primary use has been in labeling DNA and RNA in automated oligonucleotide synthesizers.

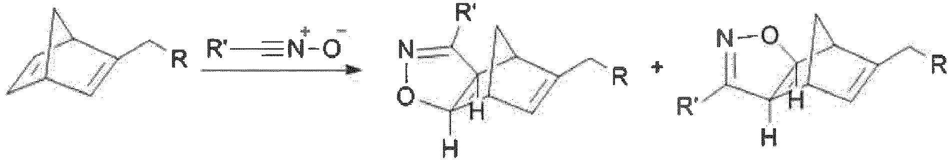

Norbornenes were selected as dipolarophiles due to their balance between strain-promoted reactivity and stability. The drawbacks of this reaction include the cross-reactivity of the nitrile oxide due to strong electrophilicity and slow reaction kinetics.

Another example of click chemistry includes oxanorbornadiene cycloaddition. The oxanorbornadiene cycloaddition is a 1,3-dipolar cycloaddition followed by a retro-Diels Alder reaction to generate a triazole-linked conjugate with the elimination of
a furan molecule. This reaction is useful in peptide labeling experiments, and it has also been used in the generation of SPECT imaging compounds.

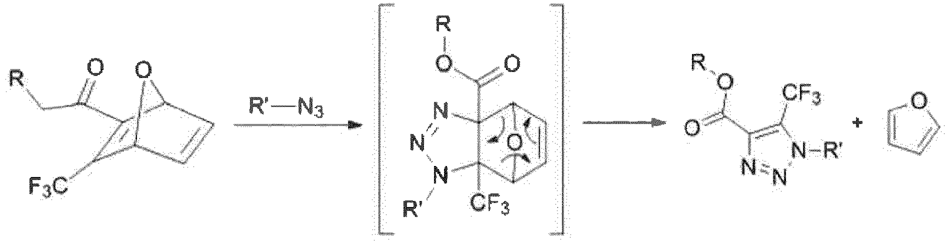

Ring strain and electron deficiency in the oxanorbornadiene increase reactivity towards the cycloaddition rate-limiting step. The retro-Diels Alder reaction occurs quickly afterwards to form the stable 1,2,3 triazole. Limitations of this reaction include poor tolerance for substituents which may change electronics of the oxanorbornadiene and low rates (second order rate constants on the order of 10⁻⁴).

Another example of click chemistry includes tetrazine ligation. The tetrazine ligation is the reaction of a trans-cyclooctene and an s-tetrazine in an inverse-demand Diels Alder reaction followed by a retro-Diels Alder reaction to eliminate nitrogen gas. The reaction is extremely rapid with a second order rate constant of 2000 M⁻¹-s⁻¹ (in 9:1 methanol/water) allowing modifications of biomolecules at extremely low concentrations.

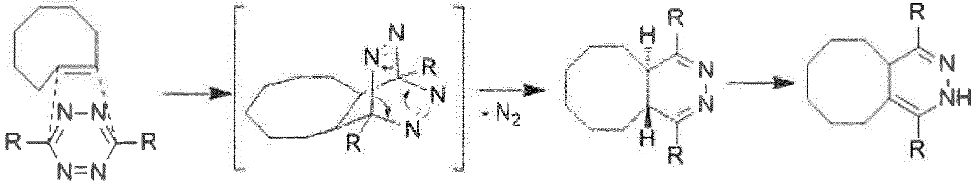

The highly strained trans-cyclooctene is used as a reactive dienophile. The diene is a 3,6-diaryl-s-tetrazine which has been substituted in order to resist immediate reaction with water. The reaction proceeds through an initial cycloaddition followed by a reverse Diels Alder to eliminate N₂ and prevent reversibility of the reaction.

Not only is the reaction tolerant of water, but it has been found that the rate increases in aqueous media. Reactions have also been performed using norbornenes as dienophiles at second order rates on the order of 1 M⁻¹•s⁻¹ in aqueous media. The reaction has been applied in labeling live cells and polymer coupling.

Another example of click chemistry includes is [4+1] cycloaddition. This isocyanide click reaction is a [4+1] cycloaddition followed by a retro-Diels Alder elimination of N₂.

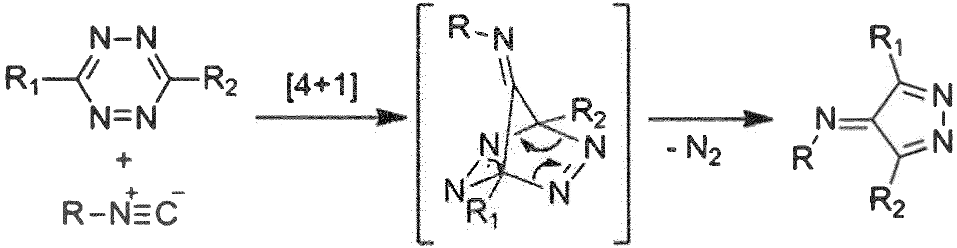

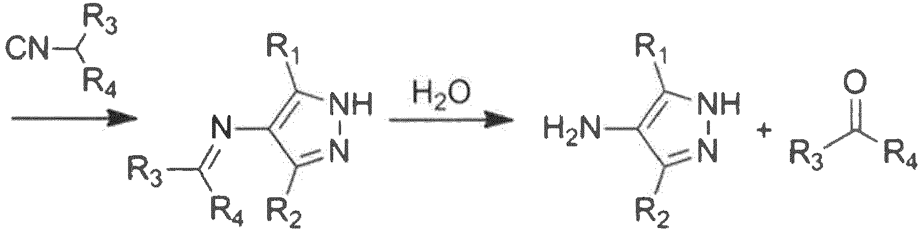

The reaction proceeds with an initial [4+1] cycloaddition followed by a reversion to eliminate a thermodynamic sink and prevent reversibility. This product is stable if a tertiary amine or isocyanopropanoate is used. If a secondary or primary isocyanide is used, the produce will form an imine which is quickly hydrolyzed.

Isocyanide is a favored chemical reporter due to its small size, stability, non-toxicity, and absence in mammalian systems. However, the reaction is slow, with second order rate constants on the order of 10⁻² M⁻¹•s⁻¹.

Another example of click chemistry includes quadricyclane ligation.
The quadricyclane ligation utilizes a highly strained quadricyclane to undergo [2+2+2] cycloaddition with π systems.

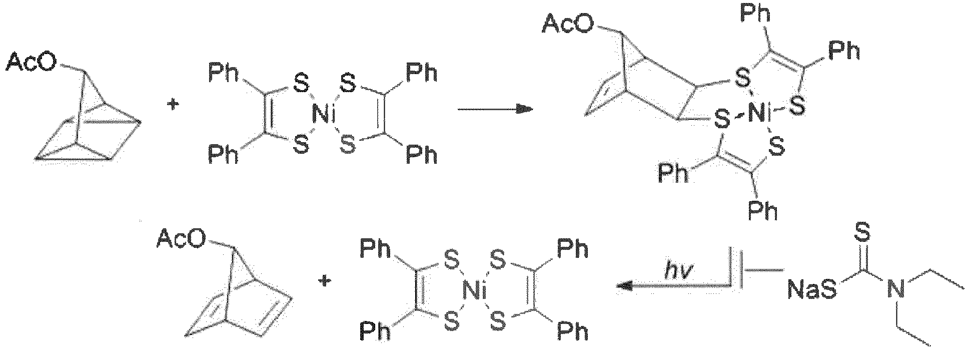

Quadricyclane is abiotic, unreactive with biomolecules (due to complete saturation), relatively small, and highly strained (∼80 kcal/mol). However, it is highly stable at room temperature and in aqueous conditions at physiological pH. It is selectively able to react with electron-poor π systems but not simple alkenes, alkynes, or cyclooctynes.

Bis(dithiobenzil)nickel(II) was chosen as a reaction partner out of a candidate screen based on reactivity. To prevent light-induced reversion to norbornadiene, diethyldithiocarbamate is added to chelate the nickel in the product.

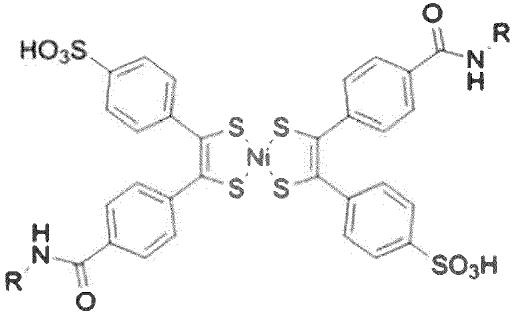

These reactions are enhanced by aqueous conditions with a second order rate constant of 0.25 M⁻¹•s⁻¹. Of particular interest is that it has been proven to be bioorthogonal to both oxime formation and copper-free click chemistry.

The exemplary click chemistry reactions have high specificity, efficient kinetics, and occur in vivo under physiological conditions. See, *e.g.,* Baskin et al. Proc. Natl. Acad. Sci. USA 104(2007):16793; Oneto et al. Acta biomaterilia (2014); Neves et al. Bioconjugate chemistry 24(2013):934; Koo et al. Angewandte Chemie 51(2012):11836; and Rossin et al. Angewandte Chemie 49(2010):3375. For a review of a wide variety of click chemistry reactions and their methodologies, see *e.g.,* Nwe K and Brechbiel MW, 2009 Cancer Biotherapy and Radiopharmaceuticals, 24(3): 289-302; Kolb HC et al., 2001 Angew. Chem. Int. Ed. 40: 2004-2021.

Exemplary bioorthogonal functional group/complementary functional group pairs are shown in the table below.

| Functional group | Paired with | Functional group | Reaction type (Reference) |
|---|---|---|---|
| azide | | Phosphine | Staudinger ligation (Saxon et al. Science 287(2000):2007-10) |
| azide | | Cyclooctyne, e.g., dibenzocyclooctyne, one of the cyclooctynes shown below, or other similar cyclooctynes: | Copper-free click chemistry (Jewett et al. J. Am. Chem. Soc. 132.11(2010):3688-90; Sletten et al. Organic Letters 10.14 (2008):3097-9; Lutz. Angew. Chem., Int. Ed 47.12(2008):2182) |
| | | 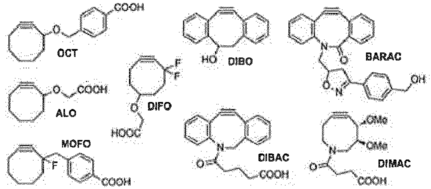 | |
| nitrone | | cyclooctyne | Nitrone Dipole Cycloaddition (Ning et al. Angew. Chem., Int. Ed 49.17 (2010):3065)^{;} |
| Nitrile oxide | | norbornene | Norbornene Cycloaddition (Gutsmiedl et al. Organic Letters 11.11 (2009):2405-8) |
| oxanorbornadie ne | | azide | Oxanorbornadiene Cycloaddition (Van Berkel et al. ChemBioChem 8.13(2007):1504-8) |
| Trans-cyclooctene, norbornene, or other alkene | | s-tetrazine | Tetrazine ligation (Hansell et al. J. Am. Chem. Soc. 133.35(2011):13828-31) |
| nitrile | | 1,2,4,5-tetrazine | [4+1] cycloaddition (Stackman et al. Organic and Biomol. Chem. 9.21(2011):7303) |
| quadricyclane | | Bis(dithiobenzil)nickel(II) | Quadricyclane Ligation (Sletten et al. J. Am. Chem. Soc. 133.44(2011):17570-3) |
| Ketone or aldehyde | | Hydrazines, hydrazones, oximes, amines, ureas, thioureas, etc. | Non-aldol carbonyl chemistry (Khomyakova EA, et al. Nucleosides Nucleotides Nucleic Acids. 30(7-8) (2011) 577-84 |
| Thiol | | maleimide | Michael addition (Zhou et al. Bioconjug Chem 2007 18(2):323-32.) |
| Dienes | | dienophiles | Diels Alder (Rossin et al. Nucl Med. (2013) 54(11):1989-95) |
| Tetrazine | | norbornene | Norbornene click chemistry (Knight et al. Org Biomol Chem. 2013 Jun 21;11(23):3817-25.) |

This list is non-exhaustive and non-limiting, as a person skilled in the art could readily identify a number of other possible biorthogonal-complementary functional group pairs.

In some embodiments, the ratio of target molecules on the drug refill (*e.g*., nucleic acid molecules, biotin, streptavidin, avidin, or a bioorthogonal functional group described herein) to the polymer molecules on the drug refill (*e.g*., alginate strands) is 1:1, 2:1, 3:1, 4:1, 5:1, 6:1, 7:1, 8:1, 9:1, 10:1, 15:1, 20:1, 30:1, 40:1, 50:1, 60:1, 70:1, 80:1, 90:1, 100:1, 120:1, 140:1, or higher.

In some embodiments, the ratio of the target recognition moiety on the drug delivery device (*e.g*., nucleic acid molecules, biotin, streptavidin, avidin, or a bioorthogonal functional group described herein) to the polymer molecule of the drug delivery device (*e.g*., alginate hydrogel) is at least 5:1, e.g., at least 5:1, 10:1, 20:1, 30:1, 40:1, 50:1, or greater. In some cases, at least 10 (e.g., at least 10, 20, 30, 40, 50, 60, 70, 80, 90, 100, 150, 200, 250, 300, or more) target recognition moieties are attached to each strand of the polymer (e.g., alginate).

In some embodiments, the binding affinity of the target on the drug refill for the target recognition moiety on the drug delivery device, *e.g*., measured by dissociation constant (K_{D}) is 500 µM or less. In other embodiments, the binding affinity of the target on the drug refill for the target recognition moiety on the drug delivery device is 1 mM or less, *e.g.,* 1 mM, 900 µM, 800 µM, 700 µM, 600 µM, 500 µM, 400 µM, 300 µM, 200 µM, 100 µM, 50 µM, 10 µM, 1 µM, 500 nM, 250 nM, 100 nM, 50 nM, 10 nM, 1 nM, 500 pM, 250 pM, 100 pM, 50 pM, 10 pM, 1 pM, 0.1 pM, 0.01 pM, or less. The K_{D} of the interaction between the target on the drug refill and the target recognition moiety on the drug delivery device is measured using standard methods in the art.

The drug delivery systems of the present invention, *i.e.,* the drug delivery device and the drug refill may be systemically administered, *e.g.,* enterally administered (*e.g.,* orally, buccally, sublingually, or rectally) or parenterally administered (*e.g*., intravenously, intra-arterially, intraosseously, intra-muscularly, intracerebrally, intracerebroventricularly, intrathecally, or subcutaneously). Other suitable modes of administration of the drug delivery systems of the invention include hypodermal, intraperitoneal, intraocular, intranasal, transdermal (e.g., via a skin patch), epidural, intracranial, percutaneous, intravaginal, intrauterineal, intravitreal, or transmucosal administration, or administration via injection, via aerosol-based delivery, or via implantation.

As shown in the Examples, in accordance to the drug delivery devices and drug refills described herein, orally administered pharmaceutical compositions were selectively targeted to the site of a drug delivery device. For example, the orally administered drugs accumulated in the intended site, *e.g.,* that of the drug delivery device, and did not accumulate elsewhere in the body. Thus, the drug delivery device/refill systems are suitable for the delivery of any orally available drugs for which there is a desire to have it concentrated at a desired location or locations. Exemplary orally available drugs include but are not limited to over the counter or prescription anti-inflammatory agents, steroids, immunosuppressive drugs, as well as other drugs/agents described herein.

In some embodiments, one drug delivery device of the present invention is administered to the subject. For example, the drug delivery device comprises, releases and/or delivers one or more kinds of pharmaceutical compositions to the desired locations. A drug refill comprising one or more kinds of pharmaceutical composition is administered repetitively such that the one drug delivery device is refilled with the one or more kinds of pharmaceutical composition. The drug delivery device is capable of being refilled through multiple administrations of a drug refill through the blood stream without having to replace the drug delivery device.

In some embodiments, the drug delivery system of the present invention comprises multiple drug delivery devices, *e.g*., at least 2, 3, 4, 5, 6, 7, 8, 9, 10, 11, 12, 13, 14, 15, 20, 25, 30, 35, 40, 45, 50 or more drug delivery devices. The drug delivery devices of the present invention may be located at the same desired location within a subject. Alternatively, the drug delivery devices of the present invention may be located at different desired locations within a subject.

In some embodiments, the drug delivery system of the present invention comprises multiple drug refills, *e.g.,* at least 2, 3, 4, 5, 6, 7, 8, 9, 10, 11, 12, 13, 14, 15, 20, 25, 30, 35, 40, 45, 50 or more drug refills. In some embodiments, each drug refill comprises a pharmaceutical composition and a different target and each drug delivery device comprises a different target recognition moiety. In some embodiments, each drug refill comprises a different pharmaceutical composition and a different target. Accordingly, each drug refill binds to a different drug delivery device, thereby allowing for independent refill of each drug delivery device at a same disease site, or at multiple disease sites. In other embodiments, each drug refill comprises multiple targets and each delivery device comprises multiple target recognition moieties. In other embodiments, multiple different drugs are delivered within one drug refill to the same drug delivery device. As demonstrated in the Example section, click chemistry-mediated targeting exhibited a high degree of specificity for the desired location within an animal model of ischemia. For example, a subject is administered one drug delivery device intratumorally for delivery of an anti-cancer pharmaceutical composition and a separate drug delivery device at a site of inflammation elsewhere in the body for delivery of an anti-inflammatory agent. Both drug delivery devices can be refilled in a single administration of a mixture of refills-for example, one drug refill comprises an anti-cancer drug and a target molecule specific for a target recognition moiety on the anti-cancer drug delivery device, and the other drug refill comprises an anti-inflammatory drug and a target molecule specific for a target recognition moiety on the anti-inflammatory drug delivery device. Delivery of drugs was successfully repeated over a period of at least one month through nine administrations.

Furthermore, the drug delivery systems of the present invention described herein permitted segregation of two different molecules through the use of two separate orthogonal chemistry systems. The spatial resolution of two different molecules targeting two different sites in the same animal demonstrates the utility of the drug delivery system described herein in combining incompatible therapies in patients. In some embodiments, a drug delivery device comprising a polymer comprising a target recognition moiety (*e.g.,* a Tz) is administered to one site in a body. A drug delivery device comprising a polymer comprising a different target recognition moiety (*e.g*., a Az) is administered to a separate site in the same ; body. Administration of a drug refill comprising a target that specifically recognizes the first target recognition moiety (*e.g*., TCO, which recognizes Tz) and a drug refill comprising a target that specifically recognizes the second target recognition moiety (*e.g*., DBCO, which recognizes Az) leads to homing of each drug refill to the respective drug delivery device. Administration of the different refills may occur simultaneously or consecutively. Administration of the refills can be repeated for multiple times. In some embodiments, the drug refills are repetitively administered for at least 5, 10, 20, 50, 100, 150, 200, 250, 300, 350, 400, 450, 500, 600, 700, 800, 900 or 1000 times.

The ability to repeatedly target drugs to a specific site within the body shows that implants within the body, such as drug delivery devices, *e.g*., hydrogels, can be selectively targeted by small molecules circulating in the blood. The efficient and repeated targeting or refilling of drug delivery devices (as described in the Examples) permit long-lasting, local drug delivery at sites in the body, such as sites of intravascular stenting, intra-tumor chemotherapy delivery devices or surgical implants. The drug delivery system of the present invention allows for lowered toxicity, improved dosing and drug availability at disease sites. Efficient homing of drug molecules to sites defined by an injected drug delivery device also permits controlled or on-demand release of drugs.

### III. Kits

The present invention provides a kit for drug delivery comprising a drug delivery device and a drug refill, wherein the drug delivery device comprises a carrier and a target recognition moiety and is suitable for implantation in a desired location within a subject; wherein the drug refill comprises a pharmaceutical composition and a target, wherein the pharmaceutical composition is attached to the target via a cleavable linker capable of being cleaved within the subject, wherein the pharmaceutical composition has undesired toxicity and the drug refill masks the toxicity of the pharmaceutical composition, wherein the target and the target recognition moiety form a two-component binding pair, wherein the drug refill is mobile until the target on the drug refill binds to the target recognition moiety on the drug delivery device, wherein upon binding of the target to the target recognition moiety, the drug refill delivers the pharmaceutical composition to the drug delivery device, the pharmaceutical composition is unmasked after delivery into the drug delivery device, and the pharmaceutical composition is released at a desirable location within the subject in a controlled manner over a time scale of days, weeks, months, or years, upon cleavage of the cleavable linker.

The kits of the present invention comprise a drug delivery device comprising a target recognition moiety and a carrier, *e.g.,* a polymer, a protein, a hydrogel (*e.g.,* a synthetic hydrogel or a biological hydrogel), an organogel, a ceramic, a composite, a metal, a wood, or a glass material. In some embodiments, the drug delivery devices comprise a polymer selected from the group consisting of collagen, alginate, polysaccharides, hyaluronic acid (HA), polyethylene glycol (PEG), poly(glycolide) (PGA), poly(L-lactide) (PLA), poly(lactide-co-glycolide) (PLGA), and poly lactic-coglycolic acid. In some embodiments, the polymer of the drug delivery device is in the form of a hydrogel, *e.g*., an alginate hydrogel.

The drug delivery devices in the kits of the present invention can be implanted or injected to a desired location within a subject. In some embodiments, the desired location is a tissue within a subject or at a site away from the tissue. In other embodiments, the desired location is an organ within a subject or at a site away from the organ. In some embodiments, the desired location is an implant, a prothestic, or any tissue or device that can be introduced into the body or on the surface of the body.

In some embodiments, the drug delivery devices in the kits of the present invention further comprise a pharmaceutical composition. In other embodiments, the drug delivery devices are implanted or injected to a desired location within the subject without a pharmaceutical composition.

The kits of the present invention also comprise a dual functionlity drug refill. The drug refills in the kits of the present invention, not only permit a direct delivery of pharmaceutical composition to the drug delivery devices, but also mask the potential toxicity of pharmaceutical composition to be delivered until the pharmaceutical composition reaches the drug delivery device at the desired location.

The drug refills in the kits of the present invention comprise a pharmaceutical composition and a target, wherein the pharmaceutical composition is attached to the target via a cleavable linker capable of being cleaved within the subject, wherein the pharmaceutical composition has undesired toxicity and the drug refill masks the toxicity of the pharmaceutical composition, wherein the target is capable of binding to a target recognition moiety on the drug delivery device, wherein the drug refill is mobile until the target on the drug refill binds to the target recognition moiety on the drug delivery device. Upon binding of the target to the target recognition moiety, the drug refill delivers the pharmaceutical composition directly to the drug delivery device, the pharmaceutical composition is unmasked after delivery into the drug delivery device, and the pharmaceutical composition is released at a desirable location within the subject in a controlled manner over a time scale of days, weeks, months, or years, upon cleavage of the cleavable linker.

The drug refills and/or the drug delivery devices in the kits of the present invention comprise a pharmaceutical composition. In some embodiments, the pharmaceutical composition comprises a small molecule o a biologic. A biologic is a medicinal product manufactured in, extracted from, or semisyntehsized from biological sources which is different from chemically synthesized pharmaceuticals. In some embodiments, biologics used in the present invention may include, but not limited to antibodies, vaccines, blood, or blood components, allergenics, somatic cells, gene therapies, tissues, recombinant therapeutic protein, and living cells used in cell therapy. Biologics can be composed of sugars, proteins, or nucleic acids or complex combinations of these substances, or may be living cells or tissues, and they can be isolated from natural sources such as human, animal, or microorganism.

The pharmaceutical composition is attached to the target via a cleavable linker. Examples of cleavable linkers include a hydrolysable linker, a pH cleavage linker, an enzyme cleavable linker, or any cleavable linker as described herein.

The pharmaceutical composition for use in the kits of the present invention has undesired toxicity, and the drug refills of the present invention mask the toxicity of the pharmaceutical composition when administered into a subject. In some embodiments, the drug refills in the kits of the present invention may mask the toxicity of the pharmaceutical composition by preventing the pharmaceutical composition from crossing the cell membrane. In other embodiments, the drug refills may mask the toxicity of the pharmaceutical composition by preventing the pharmaceutical composition from binding to the biological target of the pharmaceutical composition. In some embodiments, at least 10%, 20%, 30%, 40%, 50%, 55%, 60%, 65%, 70%, 75%, 80%, 85%, 90%, 95% or 100% of the toxicity of the pharmaceutical composition is masked by the drug refill. The drug delivery systems in the kits of the present invention allow for more efficient targeting of disease sites, and at the same time, minimizing the potential toxicity of the drugs to be delivered through the development of the dual functional drug refills. The drug refills in the kits of the present invention, not only permit a direct delivery of pharmaceutical composition to the drug delivery device, but also mask the potential toxicity of pharmaceutical composition to be delivered until the pharmaceutical composition reaches the drug delivery device at the desired location.

The pharmaceutical composition in the kits of the present invention will only be unmasked upon delivery into the drug delivery device. The pharmaceutical composition is unmasked by separating it from the target in the drug refill. The target in the drug refill is separated from the pharmaceutical composition by cleaving a bond between the pharmaceutical composition and the linker, a bond within the cleavable linker, or a bond between the pharmaceutical composition and the target. In some embodiments, the bond between the pharmaceutical composition and linker, the bond within the cleavable linker, or the bond between the pharmaceutical composition and the target is cleaved by an enzymatic degradation of the bonds. In other embodiments, the bond between the pharmaceutical composition and linker, the bond within the cleavable linker, or the bond between the pharmaceutical composition and the target is cleaved by hydrolysis of the bonds. In yet another embodiments the bond between the pharmaceutical composition and linker, the bond within the cleavable linker, or the bond between the pharmaceutical composition and the target is cleaved by reduction of the bonds.

In some embodiments, the bond between the pharmaceutical composition and the cleavable linker comprises a bond selected from the group consisting of a hydrazine bond, an acetal bond, a ketal bond, an oxime bond, an imine bond, and an aminal bond.

Release of the pharmaceutical composition from the drug delivery device in the kits of the present invention can be achieved in a controlled manner. Unlike the existing drug delivery systems which usually mediate delivery of pharmaceutical composition within minutes, the drug delivery systems of the present invention provide a more sustained release of pharmaceutical composition over a time scale of days, weeks, months or years.

In some embodiments, the pharmaceutical composition for use in the kits of the present invention is released by cleaving a bond between the pharmaceutical composition and the linker, a bond within the cleavable linker, or a bond between the pharmaceutical composition and the target. In other embodiments, the bond between the pharmaceutical composition and linker, the bond within the cleavable linker, or the bond between the pharmaceutical composition and the target is cleaved by enzyme degradation, hydrolysis or reduction of the bond. In some embodiments, the pharmaceutical composition is not released from the drug refill to the desired location within a subject. In some embodiments, the bond between the pharmaceutical composition and the cleavable linker comprises a bond selected from the group consisting of a hydrazine bond, an acetal bond, a ketal bond, an oxime bond, an imine bond, and an aminal bond. In some embodiments, at least 50%, 55%, 60%, 65%, 70%, 75%, 80%, 85%, 90%, 95% or 100% of the pharmaceutical composition is released from the drug delivery device to the desired location within a subject.

Specific recognition between the drug delivery devices and drug refills in the kits of the present invention is facilitated by the interaction between the target and the target recognition moiety. In some embodiments, the target and/or target recognition moiety comprises a nucleic acid, peptide, polysaccharide, lipid, small molecule, or combination thereof. In some embodiments, the target on the drug refill comprises biotin or desthiobiotin, and the target recognition moiety on the drug delivery device comprises avidin, neutravidin, streptavidin, or other form of avidin. Alternatively, the target on the drug refill comprises avidin, neutravidin, streptavidin, or other form of avidin and the target recognition moiety on the drug delivery device comprises biotin or desthiobiotin.

In some embodiments, the target on the drug refill comprises a bioorthogonal functional group and the target recognition moiety on the drug delivery device comprises a complementary functional group, where the bioorthogonal functional group is capable of chemically reacting with the complementary functional group to form a covalent bond. Exemplary bioorthogonal functional group/complementary functional group pairs used as the target and the target recognition moiety of the present invention may include, but not limited to, azide with phosphine; azide with cyclooctyne; nitrone with cyclooctyne; nitrile oxide with norbornene; oxanorbornadiene with azide; trans-cyclooctene with s-tetrazine; quadricyclane with bis(dithiobenzil)nickel(II).

In some embodiments, the bioorthogonal functional groups on the drug refills are capable of reacting by click chemistry with the complementary functional groups on the drug delivery devices. In some embodiments, the bioorthogonal functional group on the drug refill comprises an alkene, *e.g.,* a cyclooctene, *e.g.,* a transcyclooctene (TCO) or norbornene (NOR), and the complementary functional group on the drug delivery device comprises a tetrazine (Tz). In other embodiments, the bioorthogonal functional group on the drug refill comprises an alkyne, *e.g*., a cyclooctyne such as dibenzocyclooctyne (DBCO), and the complementary functional group on the drug delivery device comprises an azide (Az). In some embodiments, the bioorthogonal functional group on the drug refill comprises a Tz, and the complementary functional group on the drug delivery device comprises an alkene such as transcyclooctene (TCO) or norbornene (NOR). Alternatively or in addition, the bioorthogonal functional group on the drug refill comprises an Az, and the complementary functional group on the drug delivery device comprises a cyclooctyne such as dibenzocyclooctyne (DBCO).

The drug delivery systems in the kits of the present invention, *i.e.,* the drug delivery device and the drug refill may be systemically administered, *e.g.,* enterally administered (*e.g.,* orally, buccally, sublingually, or rectally) or parenterally administered (*e.g*., intravenously, intra-arterially, intraosseously, intra-muscularly, intracerebrally, intracerebroventricularly, intrathecally, or subcutaneously). Other suitable modes of administration of the drug delivery systems of the invention include hypodermal, intraperitoneal, intraocular, intranasal, transdermal (*e.g*., via a skin patch), epidural, intracranial, percutaneous, intravaginal, intrauterineal, intravitreal, or transmucosal administration, or administration via injection, via aerosol-based delivery, or via implantation.

In some embodiments, the kits of the present invention comprise one drug delivery device. In some embodiments, the kits of the present invention comprise multiple drug delivery devices, *e.g*., at least 2, 3, 4, 5, 6, 7, 8, 9, 10, 11, 12, 13, 14, 15, 20, 25, 30, 35, 40, 45, 50 or more drug delivery devices. The drug delivery devices of the present invention may be located at the same desired location within a subject. Alternatively, the drug delivery devices of the present invention may be located at different desired locations within a subject.

In some embodiments, the kits of the present invention comprise multiple drug refills, *e.g.,* at least 2, 3, 4, 5, 6, 7, 8, 9, 10, 11, 12, 13, 14, 15, 20, 25, 30, 35, 40, 45, 50 or more drug refills. In some embodiments, each drug refill comprises a pharmaceutical composition and a different target and each drug delivery device comprises a different target recognition moiety. In some embodiments, each drug refill comprises a different pharmaceutical composition and a different target. Accordingly, each drug refill binds to a different drug delivery device, thereby allowing for independent refill of each drug delivery device at a same disease site, or at multiple disease sites. In other embodiments, each drug refill comprises multiple targets and each delivery device comprises multiple target recognition moieties. In other embodiments, multiple different drugs are delivered within one drug refill to the same drug delivery device.

Furthermore, the kits of the present invention permit segregation of two different molecules through the use of two separate orthogonal chemistry systems. The spatial resolution of two different molecules targeting two different sites in the same animal demonstrates the utility of the drug delivery system described herein in combining incompatible therapies in patients. Administration of the refills can be repeated for multiple times. In some embodiments, the drug refills are repetitively administered for at least 5, 10, 20, 50, 100, 150, 200, 250, 300, 350, 400, 450, 500, 600, 700, 800, 900 or 1000 times.

### IV. Methods of Refilling a Drug Delivery Device

A method of refilling a drug delivery device *in vivo,* comprises the steps of: i) administering the drug delivery device as described herein to a subject, wherein the drug delivery device comprises a target recognition moiety, wherein the drug delivery device is suitable for implantation in a desired location within a subject; and ii) subsequently administering to the subject a drug refill comprising a pharmaceutical composition and a target, wherein the target and the target recognition moiety form a two-component binding pair; wherein the drug refill is mobile until the target on the drug refill binds to the target recognition moiety on the drug delivery device, and wherein upon binding of the target to the target recognition moiety, the drug refill delivers the pharmaceutical composition directly to the drug delivery device; thereby refilling the drug delivery device.

The drug delivery devices comprise a target recognition moiety and a carrier, *e.g.*, a polymer, a protein, a hydrogel (*e.g.,* a synthetic hydrogel or a biological hydrogel), an organogel, a ceramic, a composite, a metal, a wood, or a glass material. In some embodiments, the drug delivery devices comprise a polymer selected from the group consisting of collagen, alginate, polysaccharides, hyaluronic acid (HA), polyethylene glycol (PEG), poly(glycolide) (PGA), poly(L-lactide) (PLA), poly(lactide-co-glycolide) (PLGA), and poly lactic-coglycolic acid. In some embodiments, the polymer of the drug delivery device is in the form of a hydrogel, *e.g*., an alginate hydrogel.

The drug delivery devices of the present invention can be implanted or injected to a desired location within a subject. In some embodiments, the desired location is a tissue within a subject or at a site away from the tissue. In other embodiments, the desired location is an organ within a subject or at a site away from the organ. In some embodiments, the desired location is an implant, a prothestic, or any tissue or device that can be introduced into the body or on the surface of the body.

In some embodiments, the drug delivery devices of the present invention further comprise a pharmaceutical composition. In other embodiments, the drug delivery devices are implanted or injected to a desired location within the subject without a pharmaceutical composition.

The methods described herein also comprise a dual functionality drug refill. The drug refills, not only permit a direct delivery of pharmaceutical composition to the drug delivery devices, but also mask the potential toxicity of a pharmaceutical composition to be delivered until the pharmaceutical composition reaches the drug delivery device at the desired location.

The drug refills in the methods described herein comprise a pharmaceutical composition and a target, wherein the pharmaceutical composition is attached to the target directly or via a cleavable linker, wherein the pharmaceutical composition has undesired toxicity and the drug refill masks the toxicity of the pharmaceutical composition, wherein the target is capable of binding to a target recognition moiety on the drug delivery device, wherein the drug refill is mobile until the target on the drug refill binds to the target recognition moiety on the drug delivery device. Upon binding of the target to the target recognition moiety, the drug refill delivers the pharmaceutical composition directly to the drug delivery device, and the pharmaceutical composition is unmasked after delivery into the drug delivery device.

The drug refills and/or the drug delivery devices in the methods described herein comprise a pharmaceutical composition. The pharmaceutical composition may comprise a small molecule o a biologic. A biologic is a medicinal product manufactured in, extracted from, or semisyntehsized from biological sources which is different from chemically synthesized pharmaceuticals. In some embodiments, biologics used in the present invention may include, but not limited to antibodies, vaccines, blood, or blood components, allergenics, somatic cells, gene therapies, tissues, recombinant therapeutic protein, and living cells used in cell therapy. Biologics can be composed of sugars, proteins, or nucleic acids or complex combinations of these substances, or may be living cells or tissues, and they can be isolated from natural sources such as human, animal, or microorganism.

The pharmaceutical composition may be attached directly to the target, *e.g*., via a covalent bond, a non-covalent bond, or an ionic bond. The pharmaceutical composition may be attached to the target via a cleavable linker. Examples of cleavable linkers include a hydrolysable linker, a pH cleavage linker, an enzyme cleavable linker, or any cleavable linker as described herein.

The pharmaceutical composition for use in the methods described herein may be toxic, and the drug refills of the present invention mask the toxicity of the pharmaceutical composition when administered into a subject.
The drug refills in the methods described herein may mask the toxicity of the pharmaceutical composition by preventing the pharmaceutical composition from crossing the cell membrane. The drug refills may mask the toxicity of the pharmaceutical composition by preventing the pharmaceutical composition from binding to the biological target of the pharmaceutical composition. At least 10%, 20%, 30%, 40%, 50%, 55%, 60%, 65%, 70%, 75%, 80%, 85%, 90%, 95% or 100% of the toxicity of the pharmaceutical composition may be masked by the drug refill. Accordingly, the drug refills in the methods described herein, not only permit a direct delivery of pharmaceutical composition to the drug delivery device, but also mask the potential toxicity of pharmaceutical composition to be delivered until the pharmaceutical composition reaches the drug delivery device at the desired location.

The pharmaceutical composition in the methods described herein will be unmasked upon delivery into the drug delivery device. The pharmaceutical composition is unmasked by separating it from the target in the drug refill. The target in the drug refill may be separated from the pharmaceutical composition by cleaving a bond between the pharmaceutical composition and the linker, a bond within the cleavable linker, or a bond between the pharmaceutical composition and the target. The bond between the pharmaceutical composition and linker, the bond within the cleavable linker, or the bond between the pharmaceutical composition and the target may be cleaved by an enzymatic degradation of the bonds. The bond between the pharmaceutical composition and linker, the bond within the cleavable linker, or the bond between the pharmaceutical composition and the target may be cleaved by hydrolysis of the bonds. The bond between the pharmaceutical composition and linker, the bond within the cleavable linker, or the bond between the pharmaceutical composition and the target may be cleaved by reduction of the bonds.

The bond between the pharmaceutical composition and the cleavable linker may comprise a bond selected from the group consisting of a hydrazine bond, an acetal bond, a ketal bond, an oxime bond, an imine bond, and an aminal bond.

Release of the pharmaceutical composition from the drug delivery device in the methods described herein can be achieved in a controlled manner. Unlike the existing drug delivery systems which usually mediate delivery of pharmaceutical composition within minutes, the drug delivery systems of the present invention provide a more sustained release of pharmaceutical composition over a time scale of days, weeks, months or years.

The pharmaceutical composition for use in the methods described herein is released by cleaving a bond between the pharmaceutical composition and the linker, a bond within the cleavable linker, or a bond between the pharmaceutical composition and the target. The bond between the pharmaceutical composition and linker, the bond within the cleavable linker, or the bond between the pharmaceutical composition and the target may be cleaved by enzyme degradation, hydrolysis or reduction of the bond. The bond between the pharmaceutical composition and the cleavable linker may comprise a bond selected from the group consisting of a hydrazine bond, an acetal bond, a ketal bond, an oxime bond, an imine bond, and an aminal bond. In some embodiments, the pharmaceutical composition is not released from the drug refill to the desired location within a subject. At least 50%, 55%, 60%, 65%, 70%, 75%, 80%, 85%, 90%, 95% or 100% of the pharmaceutical composition may be released from the drug delivery device to the desired location within a subject.

Specific recognition between the drug delivery devices and drug refills in the methods described herein is facilitated by the interaction between the target and the target recognition moiety. The target and/or target recognition moiety may comprise a nucleic acid, peptide, polysaccharide, lipid, small molecule, or combination thereof. The target on the drug refill may comprise biotin or desthiobiotin, and the target recognition moiety on the drug delivery device may comprise avidin, neutravidin, streptavidin, or other form of avidin. Alternatively, the target on the drug refill may comprise avidin, neutravidin, streptavidin, or other form of avidin and the target recognition moiety on the drug delivery device may comprise biotin or desthiobiotin.

The target on the drug refill may comprise a bioorthogonal functional group and the target recognition moiety on the drug delivery device may comprise a complementary functional group, where the bioorthogonal functional group is capable of chemically reacting with the complementary functional group to form a covalent bond. Exemplary bioorthogonal functional group/complementary functional group pairs used as the target and the target recognition moiety of the present invention may include, but not limited to, azide with phosphine; azide with cyclooctyne; nitrone with cyclooctyne; nitrile oxide with norbornene; oxanorbornadiene with azide; trans-cyclooctene with s-tetrazine; quadricyclane with bis(dithiobenzil)nickel(II).

The bioorthogonal functional groups on the drug refills may be capable of reacting by click chemistry with the complementary functional groups on the drug delivery devices. The bioorthogonal functional group on the drug refill may comprise an alkene, *e.g.,* a cyclooctene, *e.g.,* a transcyclooctene (TCO) or norbornene (NOR), and the complementary functional group on the drug delivery device may comprise a tetrazine (Tz). The bioorthogonal functional group on the drug refill may comprise an alkyne, *e.g.,* a cyclooctyne such as dibenzocyclooctyne (DBCO), and the complementary functional group on the drug delivery device may comprise an azide (Az). In some embodiments, the bioorthogonal functional group on the drug refill may comprise a Tz, and the complementary functional group on the drug delivery device may comprise an alkene such as transcyclooctene (TCO) or norbornene (NOR). Alternatively or in addition, the bioorthogonal functional group on the drug refill comprises an Az, and the complementary functional group on the drug delivery device comprises a cyclooctyne such as dibenzocyclooctyne (DBCO).

The drug delivery device and the drug refill in the methods described herein may be systemically administered, *e.g.,* enterally administered (*e.g.,* orally, buccally, sublingually, or rectally) or parenterally administered (*e.g*., intravenously, intra-arterially, intraosseously, intra-muscularly, intracerebrally, intracerebroventricularly, intrathecally, or subcutaneously). Other suitable modes of administration of the drug delivery systems of the invention include hypodermal, intraperitoneal, intraocular, intranasal, transdermal (*e.g*., via a skin patch), epidural, intracranial, percutaneous, intravaginal, intrauterineal, intravitreal, or transmucosal administration, or administration via injection, via aerosol-based delivery, or via implantation.

### V. Methods of Treatment

The refillable drug delivery systems of the present invention are useful for the treatment of various diseases, including cancer. As shown in the Examples present herein, when doxorubicin was delivered into tumor-bearing mice by DNA-mediated binding of the drug refill to the drug delivery device, repeated drug refilling of the drug delivery device resulted in a significant inhibition of tumor growth. Bioorthogonal click chemistry was also demonstrated to be efficient for targeting circulating small molecules to drug delivery devices, *e.g.*, hydrogels, resident intramuscularly in diseased tissues. Small molecules were repeatedly targeted to the diseased area over the course of at least one month. Two bioorthogonal reactions were used to segregate two small molecules injected as a mixture to two separate locations in a mouse disease model. In addition to direct treatment of tumors, the drug delivery system of the present invention are suitable for treatment of other diseases, such as wound healing, inflammation, inflammatory diseases, suitable for ocular drug delivery, or delivery of drugs to vascular drug delivery devices.

Accordingly, the systems of the present invention are for use in maintaining or reducing the size of a tumor in a subject in need thereof, wherein i) the drug delivery device as described herein is administered to a desired location within the subject, wherein the pharmaceutical composition comprises an anti-cancer drug; ii) the drug refill as described herein is subsequently administered to the subject; iii) the target on the drug refill is allowed to bind to the target recognition moiety on the drug delivery device to thereby deliver the pharmaceutical composition directly to the drug delivery device; iv) the pharmaceutical composition is allowed to be released from the drug delivery device to the desired location within the subject; v) steps ii)-iv) are optionally repeated to thereby maintain or reduce the size of the tumor in the subject.

Also described herein is a method of reducing cancer progression in a subject in need thereof, comprising the steps of: i) administering the drug delivery device as described herein to a desired location within the subject, wherein the pharmaceutical composition comprises an anti-cancer drug; ii) subsequently administering the drug refill as described herein to the subject orally, intraperitoneally, intravenously, or intra-arterially; iii) allowing the target on the drug refill to bind to the target recognition moiety on the drug delivery device, thereby delivering the pharmaceutical composition directly to the drug delivery device; iv) allowing the pharmaceutical composition to be released from the drug delivery device to the desired location within the subject; v) optionally, repeating steps ii-iv); thereby reducing cancer progression in the subject.

Also described herein is a method of preventing tumor recurrence in a subject in need thereof, comprising the steps of: i) administering the drug delivery device as described herein to a desired location within the subject, wherein the pharmaceutical composition comprises an anti-cancer drug; ii) subsequently administering the drug refill as described herein to the subject orally, intraperitoneally, intravenously, or intra-arterially; iii) allowing the target on the drug refill to bind to the target recognition moiety on the drug delivery device, thereby delivering the pharmaceutical composition directly to the drug delivery device; iv) allowing the pharmaceutical composition to be released from the drug delivery device to the desired location within the subject; v) optionally, repeating steps ii-iv); thereby preventing tumor recurrence in the subject.

In some embodiments, the subject suffers from a cancer, comprising a solid cancer or a hematological cancer. In some embodiments, the desired location is a tumor site or a site away from the tumor site within the subject. In other embodiments, the drug refill is administered orally, buccally, sublingually, rectally, intravenously, intra-arterially, intraosseously, intra-muscularly, intracerebrally, intracerebroventricularly, intrathecally, subcutaneously, intraperitoneally, intraocularly, intranasally, transdermally, epidurally, intracranially, percutaneously, intravaginaly, intrauterineally, intravitreally, transmucosally, or via injection, via aerosol-based delivery, or via implantation. In some embodiments, the anti-cancer drug comprises doxorubicin.

Described herein is a method of promoting wound healing in a subject, comprising the steps of: i) administering the drug delivery device as described herein to a desired location within the subject, wherein the pharmaceutical composition promotes angiogenesis and/or maturation or remodeling of an existing blood vessel; ii) subsequently administering the drug refill as described herein to the subject; iii) allowing the target on the drug refill to bind to the target recognition moiety on the drug delivery device, thereby delivering the pharmaceutical composition directly to the drug delivery device; iv) allowing the pharmaceutical composition to be released from the drug delivery device to the desired location within the subject; v) optionally, repeating steps ii-iv); thereby promoting wound healing in the subject.

Described herein is a method of reducing or controlling inflammation in a subject in need thereof, comprising the steps of: i) administering the drug delivery device as described herein to a desired location within the subject, wherein the pharmaceutical composition comprises an anti-inflammatory agent; ii) subsequently administering the drug refill as described herein to the subject; iii) allowing the target on the drug refill to bind to the target recognition moiety on the drug delivery device, thereby delivering the pharmaceutical composition directly to the drug delivery device; iv) allowing the pharmaceutical composition to be released from the drug delivery device to the desired location within the subject; v) optionally, repeating steps ii-iv); thereby reducing or controlling inflammation in the subject.

The systems of the present invention are also for use in treating an eye disease in a subject in need thereof, wherein i) the drug delivery device as described herein is administered to a desired location within the subject, wherein the pharmaceutical composition treats said eye disease; ii) the drug refill as described herein is subsequently administered to the subject; iii) the target on the drug refill is allowed to bind to the target recognition moiety on the drug delivery device, thereby delivering the pharmaceutical composition directly to the drug delivery device; iv) the pharmaceutical composition is allowed to be released from the drug delivery device to the desired location within the subject; v) steps ii)-iv) are optionally repeated to thereby treat the eye disease in the subject.

Described herein is a method of treating arrhythmia in a subject in need thereof, comprising the steps of: i) administering the drug delivery device as described herein to a desired location within the subject, wherein the pharmaceutical composition treats said arrhythmia; ii) subsequently administering the drug refill as described herein to the subject; iii) allowing the target on the drug refill to bind to the target recognition moiety on the drug delivery device, thereby delivering the pharmaceutical composition directly to the drug delivery device; iv) allowing the pharmaceutical composition to be released from the drug delivery device to the desired location within the subject; v) optionally, repeating steps ii-iv); thereby treating arrhythmia in the subject.

The desired location may be the heart of the subject. The drug refill may be administered orally, buccally, sublingually, rectally, intravenously, intra-arterially, intraosseously, intra-muscularly, intracerebrally, intracerebroventricularly, intrathecally, subcutaneously, intraperitoneally, intraocularly, intranasally, transdermally, epidurally, intracranially, percutaneously, intravaginaly, intrauterineally, intravitreally, transmucosally, or via injection, via aerosol-based delivery, or via implantation.

Described herein is a method of evaluating patient medication adherence comprising administering a drug delivery device to a subject in need thereof, where the drug delivery device comprises a target recognition moiety and is suitable for implantation in a desired location within a subject; subsequently administering a drug refill comprising a pharmaceutical composition and a target to the subject, wherein the target comprises a detectable label linked thereto, wherein the target and the target recognition moiety form a two-component binding pair, and wherein the drug refill travels to and binds to the drug delivery device; detecting said label on said drug delivery device; and comparing the level of said label on said drug delivery device to the level of said label on said drug delivery device prior to administration of said drug refill; thereby evaluating patient medication adherence.

In some embodiments, the drug delivery devices comprise a target recognition moiety. In some embodiments, the drug delivery device further comprises a carrier, *e.g*., a polymer, a protein, a hydrogel (*e.g.,* a synthetic hydrogel or a biological hydrogel), an organogel, a ceramic, a composite, a metal, a wood, or a glass material. In some embodiments, the drug delivery devices comprise a polymer selected from the group consisting of collagen, alginate, polysaccharides, hyaluronic acid (HA), polyethylene glycol (PEG), poly(glycolide) (PGA), poly(L-lactide) (PLA), poly(lactide-co-glycolide) (PLGA), and poly lactic-coglycolic acid. In some embodiments, the polymer of the drug delivery device is in the form of a hydrogel, *e.g.*, an alginate hydrogel.

The drug delivery devices of the present invention can be implanted or injected to a desired location within a subject. In some embodiments, the desired location is a tissue within a subject or at a site away from the tissue. In other embodiments, the desired location is an organ within a subject or at a site away from the organ. In some embodiments, the desired location is an implant, a prothestic, or any tissue or device that can be introduced into the body or on the surface of the body.

In some embodiments, the drug delivery devices of the present invention further comprise a pharmaceutical composition. In other embodiments, the drug delivery devices are implanted or injected to a desired location within the subject without a pharmaceutical composition.

The systems for use according to the present invention or the methods described herein also comprise a dual function drug refill. The drug refills, not only permit a direct delivery of pharmaceutical composition to the drug delivery devices, but also mask the potential toxicity of pharmaceutical composition to be delivered until the pharmaceutical composition reaches the drug delivery device at the desired location.

The drug refills in the systems for use according to the present invention or the methods described herein comprise a pharmaceutical composition and a target, wherein the pharmaceutical composition is attached to the target via a cleavable linker, wherein the pharmaceutical composition has undesired toxicity and the drug refill masks the toxicity of the pharmaceutical composition, wherein the target is capable of binding to a target recognition moiety on the drug delivery device, wherein the drug refill is mobile until the target on the drug refill binds to the target recognition moiety on the drug delivery device. Upon binding of the target to the target recognition moiety, the drug refill delivers the pharmaceutical composition directly to the drug delivery device, the pharmaceutical composition is unmasked after delivery into the drug delivery device, and the pharmaceutical composition is released at a desirable location within the subject in a contolled manner over a time scale of days, weeks, months, or years, upon cleavage of the cleavable linker.

The drug refills and/or the drug delivery devices in the systems for use or in the methods described herein comprise a pharmaceutical composition. In some embodiments, the pharmaceutical composition comprises a small molecule o a biologic. A biologic is a medicinal product manufactured in, extracted from, or semisyntehsized from biological sources which is different from chemically synthesized pharmaceuticals. In some embodiments, biologics used in the present invention may include, but not limited to antibodies, vaccines, blood, or blood components, allergenics, somatic cells, gene therapies, tissues, recombinant therapeutic protein, and living cells used in cell therapy. Biologics can be composed of sugars, proteins, or nucleic acids or complex combinations of these substances, or may be living cells or tissues, and they can be isolated from natural sources such as human, animal, or microorganism.

In some embodiments, the pharmaceutical composition, e.g., in the drug delivery device and/or the drug refill, comprises an anti-cancer drug, a drug that promotes wound healing, a drug that treats or prevents infection, or a drug that promotes vascularization. For example, the pharmaceutical composition comprises an anti-cancer drug. For example, the anti-cancer drug comprises a chemotherapeutic, *e.g*., a cancer vaccine.

In some embodiments, an anti-cancer drug includes a small molecule, a peptide or polypeptide, a protein or fragment thereof (*e.g*., an antibody or fragment thereof), or a nucleic acid.

Exemplary anti-cancer drugs for use in the present invention may include, but are not limited to, Abiraterone Acetate, Abitrexate (Methotrexate), Abraxane (Paclitaxel Albumin-stabilized Nanoparticle Formulation), ABVD, ABVE, ABVE-PC, AC, AC-T, Adcetris (Brentuximab Vedotin), ADE, Ado-Trastuzumab Emtansine, Adriamycin (Doxorubicin Hydrochloride), Adrucil (Fluorouracil), Afatinib Dimaleate, Afinitor (Everolimus), Aldara (Imiquimod), Aldesleukin, Alemtuzumab, Alimta (Pemetrexed Disodium), Aloxi (Palonosetron Hydrochloride), Ambochlorin (Chlorambucil), Aminolevulinic Acid, Anastrozole, Aprepitant, Aredia (Pamidronate Disodium), Arimidex (Anastrozole), Aromasin (Exemestane), Arranon (Nelarabine), Arsenic Trioxide, Arzerra (Ofatumumab), Asparaginase Erwinia chrysanthemi, Avastin (Bevacizumab), Axitinib, Azacitidine, BEACOPP, Bendamustine Hydrochloride, BEP, Bevacizumab, Bexarotene, Bexxar (Tositumomab and I 131 Iodine Tositumomab), Bicalutamide, Bleomycin, Bortezomib, Bosulif (Bosutinib), Bosutinib, Brentuximab Vedotin, Busulfan, Busulfex (Busulfan), Cabazitaxel, Cabozantinib-S-Malate, CAF, Campath (Alemtuzumab), Camptosar (Irinotecan Hydrochloride), Capecitabine, CAPOX, Carboplatin, Carboplatin-Taxol, Carfilzomib, Casodex (Bicalutamide), CeeNU (Lomustine), Cerubidine (Daunorubicin Hydrochloride), Cervarix (Recombinant HPV Bivalent Vaccine), Cetuximab, Chlorambucil, Chlorambucil-Prednisone, CHOP, Cisplatin, Clafen (Cyclophosphamide), Clofarabine, Clofarex (Clofarabine), Clolar (Clofarabine), CMF, Cometriq (Cabozantinib-S-Malate), COPP, COPP-ABV, Cosmegen (Dactinomycin), Crizotinib, CVP, Cyclophosphamide, Cyfos (Ifosfamide), Cytarabine, Cytarabine (Liposomal), Cytosar-U (Cytarabine), Cytoxan (Cyclophosphamide), Dabrafenib, Dacarbazine, Dacogen (Decitabine), Dactinomycin, Dasatinib, Daunorubicin Hydrochloride, Decitabine, Degarelix, Denileukin Diftitox, Denosumab, DepoCyt (Liposomal Cytarabine), DepoFoam (Liposomal Cytarabine), Dexrazoxane Hydrochloride, Docetaxel, Doxil (Doxorubicin Hydrochloride Liposome), Doxorubicin Hydrochloride, Doxorubicin Hydrochloride Liposome, Dox-SL (Doxorubicin Hydrochloride Liposome), DTIC-Dome (Dacarbazine), Efudex (Fluorouracil), Elitek (Rasburicase), Ellence (Epirubicin Hydrochloride), Eloxatin (Oxaliplatin), Eltrombopag Olamine, Emend (Aprepitant), Enzalutamide, Epirubicin Hydrochloride, EPOCH, Erbitux (Cetuximab), Eribulin Mesylate, Erivedge (Vismodegib), Erlotinib Hydrochloride, Erwinaze (Asparaginase Erwinia chrysanthemi), Etopophos (Etoposide Phosphate), Etoposide, Etoposide Phosphate, Evacet (Doxorubicin Hydrochloride Liposome), Everolimus, Evista (Raloxifene Hydrochloride), Exemestane, Fareston (Toremifene), Faslodex (Fulvestrant), FEC, Femara (Letrozole), Filgrastim, Fludara (Fludarabine Phosphate), Fludarabine Phosphate, Fluoroplex (Fluorouracil), Fluorouracil, Folex (Methotrexate), Folex PFS (Methotrexate), Folfiri, Folfiri-Bevacizumab, Folfiri-Cetuximab, Folfirinox, Folfox, Folotyn (Pralatrexate), FU-LV, Fulvestrant, Gardasil (Recombinant HPV Quadrivalent Vaccine), Gazyva (Obinutuzumab), Gefitinib, Gemcitabine Hydrochloride, Gemcitabine-Cisplatin, Gemcitabine-Oxaliplatin, Gemtuzumab Ozogamicin, Gemzar (Gemcitabine Hydrochloride), Gilotrif (Afatinib Dimaleate), Gleevec (Imatinib Mesylate), Glucarpidase, Goserelin Acetate, Halaven (Eribulin Mesylate), Herceptin (Trastuzumab), HPV Bivalent Vaccine (Recombinant), HPV Quadrivalent Vaccine (Recombinant), Hycamtin (Topotecan Hydrochloride), Hyper-CVAD, Ibritumomab Tiuxetan, Ibrutinib, ICE, Iclusig (Ponatinib Hydrochloride), Ifex (Ifosfamide), Ifosfamide, Ifosfamidum (Ifosfamide), Imatinib Mesylate, Imbruvica (Ibrutinib), Imiquimod, Inlyta (Axitinib), Intron A (Recombinant Interferon Alfa-2b), Iodine 131 Tositumomab and Tositumomab, Ipilimumab, Iressa (Gefitinib), Irinotecan Hydrochloride, Istodax (Romidepsin), Ixabepilone, Ixempra (Ixabepilone), Jakafi (Ruxolitinib Phosphate), Jevtana (Cabazitaxel), Kadcyla (Ado-Trastuzumab Emtansine), Keoxifene (Raloxifene Hydrochloride), Kepivance (Palifermin), Kyprolis (Carfilzomib), Lapatinib Ditosylate, Lenalidomide, Letrozole, Leucovorin Calcium, Leukeran (Chlorambucil), Leuprolide Acetate, Levulan (Aminolevulinic Acid), Linfolizin (Chlorambucil), LipoDox (Doxorubicin Hydrochloride Liposome), Liposomal Cytarabine, Lomustine, Lupron (Leuprolide Acetate), Lupron Depot (Leuprolide Acetate), Lupron Depot-Ped (Leuprolide Acetate), Lupron Depot-3 Month (Leuprolide Acetate), Lupron Depot-4 Month (Leuprolide Acetate), Marqibo (Vincristine Sulfate Liposome), Matulane (Procarbazine Hydrochloride), Mechlorethamine Hydrochloride, Megace (Megestrol Acetate), Megestrol Acetate, Mekinist (Trametinib), Mercaptopurine, Mesna, Mesnex (Mesna), Methazolastone (Temozolomide), Methotrexate, Methotrexate LPF (Methotrexate), Mexate (Methotrexate), Mexate-AQ (Methotrexate), Mitomycin C, Mitozytrex (Mitomycin C), MOPP, Mozobil (Plerixafor), Mustargen (Mechlorethamine Hydrochloride), Mutamycin (Mitomycin C), Myleran (Busulfan), Mylosar (Azacitidine), Mylotarg (Gemtuzumab Ozogamicin), Nanoparticle Paclitaxel (Paclitaxel Albumin-stabilized Nanoparticle Formulation), Navelbine (Vinorelbine Tartrate), Nelarabine, Neosar (Cyclophosphamide), Neupogen (Filgrastim), Nexavar (Sorafenib Tosylate), Nilotinib, Nolvadex (Tamoxifen Citrate), Nplate (Romiplostim), Obinutuzumab, Ofatumumab, Omacetaxine Mepesuccinate, Oncaspar (Pegaspargase), Ontak (Denileukin Diftitox), OEPA, OPPA, Oxaliplatin, Paclitaxel, Paclitaxel Albumin-stabilized Nanoparticle Formulation, Palifermin, Palonosetron Hydrochloride, Pamidronate Disodium, Panitumumab, Paraplat (Carboplatin), Paraplatin (Carboplatin), Pazopanib Hydrochloride, Pegaspargase, Peginterferon Alfa-2b, PEG-Intron (Peginterferon Alfa-2b), Pemetrexed Disodium, Perjeta (Pertuzumab), Pertuzumab, Platinol (Cisplatin), Platinol-AQ (Cisplatin), Plerixafor, Pomalidomide, Pomalyst (Pomalidomide), Ponatinib Hydrochloride, Pralatrexate, Prednisone, Procarbazine Hydrochloride, Proleukin (Aldesleukin), Prolia (Denosumab), Promacta (Eltrombopag Olamine), Provenge (Sipuleucel-T), Purinethol (Mercaptopurine), Radium 223 Dichloride, Raloxifene Hydrochloride, Rasburicase, R-CHOP, R-CVP, Recombinant HPV Bivalent Vaccine, Recombinant HPV Quadrivalent Vaccine, Recombinant Interferon Alfa-2b, Regorafenib, Revlimid (Lenalidomide), Rheumatrex (Methotrexate), Rituxan (Rituximab), Rituximab, Romidepsin, Romiplostim, Rubidomycin (Daunorubicin Hydrochloride), Ruxolitinib Phosphate, Sclerosol Intrapleural Aerosol (Talc), Sipuleucel-T, Sorafenib Tosylate, Sprycel (Dasatinib), Stanford V, Sterile Talc Powder (Talc), Steritalc (Talc), Stivarga (Regorafenib), Sunitinib Malate, Sutent (Sunitinib Malate), Sylatron (Peginterferon Alfa-2b), Synovir (Thalidomide), Synribo (Omacetaxine Mepesuccinate), Tafinlar (Dabrafenib), Talc, Tamoxifen Citrate, Tarabine PFS (Cytarabine), Tarceva (Erlotinib Hydrochloride), Targretin (Bexarotene), Tasigna (Nilotinib), Taxol (Paclitaxel), Taxotere (Docetaxel), Temodar (Temozolomide), Temozolomide, Temsirolimus, Thalidomide, Thalomid (Thalidomide), Toposar (Etoposide), Topotecan Hydrochloride, Toremifene, Torisel (Temsirolimus), Tositumomab and I¹³¹ Iodine Tositumomab, Totect (Dexrazoxane Hydrochloride), Trametinib, Trastuzumab, Treanda (Bendamustine Hydrochloride), Trisenox (Arsenic Trioxide), Tykerb (Lapatinib Ditosylate), Vandetanib, VAMP, Vectibix (Panitumumab), VeIP, Velban (Vinblastine Sulfate), Velcade (Bortezomib), Velsar (Vinblastine Sulfate), Vemurafenib, VePesid (Etoposide), Viadur (Leuprolide Acetate), Vidaza (Azacitidine), Vinblastine Sulfate, Vincasar PFS (Vincristine Sulfate), Vincristine Sulfate, Vincristine Sulfate Liposome, Vinorelbine Tartrate, Vismodegib, Voraxaze (Glucarpidase), Vorinostat, Votrient (Pazopanib Hydrochloride), Wellcovorin (Leucovorin Calcium), Xalkori (Crizotinib), Xeloda (Capecitabine), XELOX, Xgeva (Denosumab), Xofigo (Radium 223 Dichloride), Xtandi (Enzalutamide), Yervoy (Ipilimumab), Zaltrap (Ziv-Aflibercept), Zelboraf (Vemurafenib), Zevalin (Ibritumomab Tiuxetan), Zinecard (Dexrazoxane Hydrochloride), Ziv-Aflibercept, Zoladex (Goserelin Acetate), Zoledronic Acid, Zolinza (Vorinostat), Zometa (Zoledronic Acid), and Zytiga (Abiraterone Acetate).

In some embodiments, the anti-cancer drug comprises doxorubicin.

In some embodiments, the pharmaceutical composition, *e.g*., in the drug delivery device and/or the drug refill, comprises a drug that promotes wound healing or vascularization. In some embodiments, the pharmaceutical composition comprises a drug that reduces ischemia, *e.g.,* due to peripheral artery disease (PAD) or damaged myocardial tissues due to myocardial infarction. For example, the drug comprises a protein or fragment thereof, *e.g.,* a growth factor or angiogenic factor, such as vascular endothelial growth factor (VEGF), *e.g.,* VEGFA, VEGFB, VEGFC, or VEGFD, and/or IGF, *e.g.,* IGF-1, fibroblast growth factor (FGF), angiopoietin (ANG) (e.g., Ang1 or Ang2), matrix metalloproteinase (MMP), delta-like ligand 4 (DLL4), or combinations thereof. These drugs that promote wound healing or vascularization are non-limiting, as the skilled artisan would be able to readily identify other drugs that promote wound healing or vascularization.

In some embodiments, the pharmaceutical composition, *e.g*., in the drug delivery device and/or the drug refill, comprises an anti-proliferative drug, *e.g*., mycophenolate mofetil (MMF), azathioprine, sirolimus, tacrolimus, paclitaxel, biolimus A9, novolimus, myolimus, zotarolimus, everolimus, or tranilast. These anti-proliferative drugs are non-limiting, as the skilled artisan would be able to readily identify other anti-proliferative drugs.

In some embodiments, the pharmaceutical composition, *e.g.*, in the drug delivery device and/or the drug refill, comprises an anti-inflammatory drug, *e.g.*, corticosteroid anti-inflammatory drugs (*e.g*., beclomethasone, beclometasone, budesonide, flunisolide, fluticasone propionate, triamcinolone, methylprednisolone, prednisolone, or prednisone); or non-steroidal anti-inflammatory drugs (NSAIDs) (*e.g*., acetylsalicylic acid, diflunisal, salsalate, choline magnesium trisalicylate, ibuprofen, dexibuprofen, naproxen, fenoprofen, ketoprofen, dexketoprofen, fluribiprofen, oxaprozin, loxoprofen, indomethacin, tolmetin, sulindac, etodolac, ketorolac, diclofenac, aceclofenac, nabumetone, piroxicam, meloxicam, tenoxicam, droxicam, lomoxicam, isoxicam, mefenamic acid, meclofenamic acid, flufenamic acid, tolfenamic acid, celecoxib, rofecoxib, valdecoxib, parecoxib, lumiracoxib, etoricoxib, firocoxib, nimesulide, licofelone, H-harpaide, or lysine clonixinate). These anti-inflammatory drugs are non-limiting, as the skilled artisan would be able to readily identify other anti-inflammatory drugs.

In some embodiments, the pharmaceutical composition, *e.g*., in the drug delivery device and/or the drug refill, comprises a drug that prevents or reduces transplant rejection, *e.g.,* an immunosuppressant. Exemplary immunosuppressants include calcineurin inhibitors (*e.g*., cyclosporine, Tacrolimus (FK506)); mammalian target of rapamycin (mTOR) inhibitors (*e.g*., rapamycin, also known as Sirolimus); antiproliferative agents (*e.g*.,azathioprine, mycophenolate mofetil, mycophenolate sodium); antibodies (*e.g*., basiliximab, daclizumab, muromonab); corticosteroids (*e.g*., prednisone). These drugs that prevent or reduce transplant rejection are non-limiting, as the skilled artisan would be able to readily identify other drugs that prevent or reduce transplant rejection.

In some embodiments, the pharmaceutical composition, *e.g*., in the drug delivery device and/or the drug refill, comprises an anti-thrombotic drug, *e.g.,* an anti-platelet drug, an anticoagulant drug, or a thrombolytic drug.

Exemplary anti-platelet drugs include an irreversible cyclooxygenase inhibitor (*e.g*., aspirin or triflusal); an adenosine diphosphate (ADP) receptor inhibitor (*e.g*., ticlopidine, clopidogrel, prasugrel, or tricagrelor); a phosphodiesterase inhibitor (*e.g*., cilostazol); a glycoprotein IIB/IIIA inhibitor (*e.g*., abciximab, eptifibatide, or tirofiban); an adenosine reuptake inhibitor (*e.g.,* dipyridamole); or a thromboxane inhibitor (*e.g.,* thromboxane synthase inhibitor, a thromboxane receptor inhibitor, such as terutroban). These anti-platelet drugs are non-limiting, as the skilled artisan would be able to readily identify other anti-platelet drugs.

Exemplary anticoagulant drugs include coumarins (*e.g*., warfarin, acenocoumarol, phenprocoumon, atromentin, brodifacoum, or phenindione); heparin and derivatives thereof (*e.g*., heparin, low molecular weight heparin, fondaparinux, or idraparinux); factor Xa inhibitors (*e.g*., rivaroxaban, apixaban, edoxaban, betrixaban, darexaban, letaxaban, or eribaxaban); thrombin inhibitors (*e.g*., hirudin, lepirudin, bivalirudin, argatroban, or dabigatran); antithrombin protein; batroxobin; hementin; and thrombomodulin. These anticoagulant drugs are non-limiting, as the skilled artisan would be able to readily identify other anticoagulant drugs.

Exemplary thrombolytic drugs include tissue plasminogen activator (t-PA) (*e.g.,* alteplase, reteplase, or tenecteplase); anistreplase; streptokinase; or urokinase.

In other embodiments, the pharmaceutical composition, *e.g*., in the drug delivery device and/or the drug refill, comprises a drug that prevents restenosis, *e.g*., an anti-proliferative drug, an anti-inflammatory drug, or an anti-thrombotic drug. Exemplary anti-proliferative drugs, anti-inflammatory drugs, and anti-thrombotic drugs are described herein.

In some embodiments, the pharmaceutical composition, *e.g*., in the drug delivery device and/or the drug refill, comprises a drug that treats or prevents infection, *e.g*., an antibiotic. Suitable antibiotics include, but are not limited to, beta-lactam antibiotics (*e.g*., penicillins, cephalosporins, carbapenems), polymyxins, rifamycins, lipiarmycins, quinolones, sulfonamides, macrolides lincosamides, tetracyclines, aminoglycosides, cyclic lipopeptides (*e.g.,* daptomycin), glycylcyclines (*e.g.,* tigecycline), oxazonidinones (*e.g.,* linezolid), and lipiarmycines (*e.g*., fidazomicin). For example, antibiotics include erythromycin, clindamycin, gentamycin, tetracycline, meclocycline, (sodium) sulfacetamide, benzoyl peroxide, and azelaic acid. Suitable penicillins include amoxicillin, ampicillin, bacampicillin, carbenicillin, cloxacillin, dicloxacillin, flucloxacillin, mezlocillin, nafcillin, oxacillin, penicillin g, penicillin v, piperacillin, pivampicillin, pivmecillinam, and ticarcillin. Exemplary cephalosporins include cefacetrile, cefadroxil, cephalexin, cefaloglycin, cefalonium, cefaloridine, cefalotin, cefapirin, cefatrizine, cefazaflur, cefazedone, cefazolin, cefradine, cefroxadine, ceftezole, cefaclor, cefamandole, cefmetazole, cefonicid, cefotetan, cefoxitin, cefprozil, cefuroxime, cefuzonam, cfcapene, cefdaloxime, cefdinir, cefditoren, cefetamet, cefixime, cefmenoxime, cefodizime, cefotaxime, cefpimizole, cefpodoxime, cefteram, ceftibuten, ceftiofur, ceftiolene, ceftizoxime, ceftriaxone, ceftazidime, cefclidine, cefepime, ceflurprenam, cefoselis, cefozopran, cefpirome, cequinome, ceftobiprole, ceftaroline, cefaclomezine, cefaloram, cefaparole, cefcanel, cefedrlor, cefempidone, cefetrizole, cefivitril, cefmatilen, cefmepidium, cefovecin, cefoxazole, cefrotil, cefsumide, cefuracetime, and ceftioxide. Monobactams include aztreonam. Suitable carbapenems include imipenem/cilastatin, doripenem, meropenem, and ertapenem. Exemplary macrolides include azithromycin, erythromycin, larithromycin, dirithromycin, roxithromycin, and telithromycin. Lincosamides include clindamycin and lincomycin. Exemplary streptogramins include pristinamycin and quinupristin/dalfopristin. Suitable aminoglycoside antibiotics include amikacin, gentamycin, kanamycin, neomycin, netilmicin, paromomycin, streptomycin, and tobramycin. Exemplary quinolones include flumequine, nalidixic acid, oxolinic acid, piromidic acid, pipemidic acid, rosoxacin, ciprofloxacin, enoxacin, lomefloxacin, nadifloxacin, norfloxacin, ofoxacin, pefloxacin, rufloxacin, balofloxacin, gatifloxacin, repafloxacin, levofloxacin, moxifloxacin, pazufloxacin, sparfloxacin, temafloxacin, tosufloxacin, besifloxacin, clinafoxacin, gemifloxacin, sitafloxacin, trovafloxacin, and prulifloxacin. Suitable sulfonamides include sulfamethizole, sulfamethoxazole, and trimethoprim-sulfamethoxazone. Exemplary tetracyclines include demeclocycline, doxycycline, minocycline, oxytetracycline, tetracycline, and tigecycline. Other antibiotics include chloramphenicol, metronidazole, tinidazole, nitrofurantoin, vancomycin, teicoplanin, telavancin, linezolid, cycloserine, rifampin, rifabutin, rifapentin, bacitracin, polymyxin B, viomycin, and capreomycin. The skilled artisan could readily identify other antibiotics useful in the devices and methods described herein.

In some embodiments, the pharmaceutical composition, *e.g*., in the drug delivery device and/or the drug refill, comprises a drug that reduces macular degeneration. One common current treatment for macular degeneration involves the injection of anti-angiogenesis compounds intraocularly (Lucentis, Eylea). The repeated intraocular injections are sometimes poorly tolerated by patients, leading to low patient compliance. As described herein, the ability to noninvasively refill drug depots for macular degeneration significantly improves patient compliance and patient tolerance of disease, *e.g*., macular degeneration, treatment. Controlled, repeated release made possible by device refilling allows for fewer drug dosings and improved patient comfort.

In some embodiments, the pharmaceutical composition, *e.g*., in the drug delivery device and/or the drug refill, comprises a drug that prevents immunological rejection. Prior to the invention described herein, to prevent immunological rejection of cells, tissues or whole organs, patients required lifelong therapy of systemic anti-rejection drugs that cause significant side effects and deplete the immune system, leaving patients at greater risk for infection and other complications. The ability to locally release anti-rejection drugs from a drug delivery device and to repeatedly refill the drug delivery device allows for more local anti-rejection therapy with fewer systemic side effects, improved tolerability and better efficacy.

In some embodiments, the pharmaceutical composition, *e.g*., in the drug delivery device and/or the drug refill, comprises a drug that prevents thrombosis. Some vascular devices such as vascular grafts and coated stents suffer from thrombosis, in which the body mounts a thrombin-mediated response to the devices. Anti-thrombotic drugs, released from these devices, allows for temporary inhibition of the thrombosis process, but the devices have limited drugs and cannot prevent thrombosis once the drug supply is exhausted. Since these devices are implanted for long periods of time (potentially for the entire lifetime of the patient), temporary thrombosis inhibition is not sufficient. The ability to repeatedly refill these devices with anti-thrombotic drugs and release the drug significantly improves clinical outcomes and allows for long-term thrombosis inhibition.

In some embodiments, the pharmaceutical composition, *e.g*., in the drug delivery device and/or the drug refill, comprises a drug that treats inflammation. Chronic inflammation is characterized by persistent inflammation due to non-degradable pathogens, viral infections, or autoimmune reactions and can last years and lead to tissue destruction, fibrosis, and necrosis. In some cases, inflammation is a local disease, but clinical interventions are almost always systemic. Anti-inflammatory drugs given systemically have significant side-effects including gastrointestinal problems, cardiotoxicity, high blood pressure and kidney damage, allergic reactions, and possibly increased risk of infection. The ability to repeatedly release anti-inflammatory drugs such as NSAIDs and COX-2 inhibitors could reduce these side effects. Refillable drug delivery devices implanted or injected at a site of inflammation creates the ability to deliver long term and local anti-inflammatory care while avoiding systemic side effects.

The pharmaceutical composition is attached to the target via a cleavable linker. Examples of cleavable linkers include a hydrolysable linker, a pH cleavage linker, an enzyme cleavable linker, or any cleavable linker as described herein.

In some embodiments, the pharmaceutical composition in the systems for use in the therapeutic treatments described or for use in the methods described herein may be toxic, and the drug refills of the present invention mask the toxicity of the pharmaceutical composition when administered into a subject. In some embodiments, the drug refills may mask the toxicity of the pharmaceutical composition by preventing the pharmaceutical composition from crossing the cell membrane. In other embodiments, the drug refills may mask the toxicity of the pharmaceutical composition by preventing the pharmaceutical composition from binding to the biological target of the pharmaceutical composition. In some embodiments, at least 10%, 20%, 30%, 40%, 50%, 55%, 60%, 65%, 70%, 75%, 80%, 85%, 90%, 95% or 100% of the toxicity of the pharmaceutical composition is masked by the drug refill. Accordingly, the drug refills not only permit a direct delivery of pharmaceutical composition to the drug delivery device, but also mask the potential toxicity of pharmaceutical composition to be delivered until the pharmaceutical composition reaches the drug delivery device at the desired location.

The pharmaceutical composition in the methods described herein will be unmasked upon delivery into the drug delivery device. The pharmaceutical composition is unmasked by separating it from the target in the drug refill. The target in the drug refill may be separated from the pharmaceutical composition by cleaving a bond between the pharmaceutical composition and the linker, a bond within the cleavable linker, or a bond between the pharmaceutical composition and the target. The bond between the pharmaceutical composition and linker, the bond within the cleavable linker, or the bond between the pharmaceutical composition and the target may be cleaved by an enzymatic degradation of the bonds. The bond between the pharmaceutical composition and linker, the bond within the cleavable linker, or the bond between the pharmaceutical composition and the target may be cleaved by hydrolysis of the bonds. The bond between the pharmaceutical composition and linker, the bond within the cleavable linker, or the bond between the pharmaceutical composition and the target may be cleaved by reduction of the bonds.

The bond between the pharmaceutical composition and the cleavable linker may comprise a bond selected from the group consisting of a hydrazine bond, an acetal bond, a ketal bond, an oxime bond, an imine bond, and an aminal bond.

Release of the pharmaceutical composition from the drug delivery device in the methods described herein can be achieved in a controlled manner. Unlike the existing drug delivery systems which usually mediate delivery of pharmaceutical composition within minutes, the drug delivery systems of the present invention provide a more sustained release of pharmaceutical composition over a time scale of days, weeks, months or years.

The pharmaceutical composition for use in the methods described herein may be released by cleaving a bond between the pharmaceutical composition and the linker, a bond within the cleavable linker, or a bond between the pharmaceutical composition and the target. The bond between the pharmaceutical composition and linker, the bond within the cleavable linker, or the bond between the pharmaceutical composition and the target is cleaved by enzyme degradation, hydrolysis or reduction of the bond. In some embodiments, the bond between the pharmaceutical composition and the cleavable linker comprises a bond selected from the group consisting of a hydrazine bond, an acetal bond, a ketal bond, an oxime bond, an imine bond, and an aminal bond. In some embodiments, the pharmaceutical composition is not released from the drug refill to the desired location within a subject. In some embodiments, at least 50%, 55%, 60%, 65%, 70%, 75%, 80%, 85%, 90%, 95% or 100% of the pharmaceutical composition is released from the drug delivery device to the desired location within a subject.

Specific recognition between the drug delivery devices and drug refills in the methods described herein is facilitated by the interaction between the target and the target recognition moiety. In some embodiments, the target and/or target recognition moiety comprises a nucleic acid, peptide, polysaccharide, lipid, small molecule, or combination thereof. In some embodiments, the target on the drug refill comprises biotin or desthiobiotin, and the target recognition moiety on the drug delivery device comprises avidin, neutravidin, streptavidin, or other form of avidin. Alternatively, the target on the drug refill comprises avidin, neutravidin, streptavidin, or other form of avidin and the target recognition moiety on the drug delivery device comprises biotin or desthiobiotin.

The target on the drug refill may comprise a bioorthogonal functional group and the target recognition moiety on the drug delivery device comprises a complementary functional group, where the bioorthogonal functional group is capable of chemically reacting with the complementary functional group to form a covalent bond. Exemplary bioorthogonal functional group/complementary functional group pairs used as the target and the target recognition moiety of the present invention may include, but not limited to, azide with phosphine; azide with cyclooctyne; nitrone with cyclooctyne; nitrile oxide with norbornene; oxanorbornadiene with azide; trans-cyclooctene with s-tetrazine; quadricyclane with bis(dithiobenzil)nickel(II).

The bioorthogonal functional groups on the drug refills may be capable of reacting by click chemistry with the complementary functional groups on the drug delivery devices. The bioorthogonal functional group on the drug refill may comprise an alkene, *e.g*., a cyclooctene, *e.g.,* a transcyclooctene (TCO) or norbornene (NOR), and the complementary functional group on the drug delivery device comprises a tetrazine (Tz). The bioorthogonal functional group on the drug refill may comprise an alkyne, *e.g.,* a cyclooctyne such as dibenzocyclooctyne (DBCO), and the complementary functional group on the drug delivery device comprises an azide (Az). The bioorthogonal functional group on the drug refill may comprise a Tz, and the complementary functional group on the drug delivery device comprises an alkene such as transcyclooctene (TCO) or norbornene (NOR). Alternatively or in addition, the bioorthogonal functional group on the drug refill comprises an Az, and the complementary functional group on the drug delivery device comprises a cyclooctyne such as dibenzocyclooctyne (DBCO).

The drug delivery device and the drug refill in the methods described herein may be systemically administered, *e.g.,* enterally administered (*e.g.,* orally, buccally, sublingually, or rectally) or parenterally administered (*e.g*., intravenously, intra-arterially, intraosseously, intra-muscularly, intracerebrally, intracerebroventricularly, intrathecally, or subcutaneously). Other suitable modes of administration of the drug delivery systems of the invention include hypodermal, intraperitoneal, intraocular, intranasal, transdermal (*e.g*., via a skin patch), epidural, intracranial, percutaneous, intravaginal, intrauterineal, intravitreal, or transmucosal administration, or administration via injection, via aerosol-based delivery, or via implantation.

The methods described herein may comprise one drug delivery device. A drug refill comprising one or more kinds of pharmaceutical composition is administered repetitively such that the one drug delivery device is refilled with the one or more kinds of pharmaceutical composition. The drug delivery device is capable of being refilled through multiple administrations of a drug refill through the blood stream without having to replace the drug delivery device.

The methods described herein may comprise multiple drug delivery devices, *e.g.,* at least 2, 3, 4, 5, 6, 7, 8, 9, 10, 11, 12, 13, 14, 15, 20, 25, 30, 35, 40, 45, 50 or more drug delivery devices. The drug delivery devices of the present invention may be located at the same desired location within a subject. Alternatively, the drug delivery devices of the present invention may be located at different desired locations within a subject.

The methods described herein may comprise multiple drug refills, *e.g.,* at least 2, 3, 4, 5, 6, 7, 8, 9, 10, 11, 12, 13, 14, 15, 20, 25, 30, 35, 40, 45, 50 or more drug refills. Each drug refill may comprise a pharmaceutical composition and a different target and each drug delivery device comprises a different target recognition moiety. Each drug refill may comprise a different pharmaceutical composition and a different target. Accordingly, each drug refill binds to a different drug delivery device, thereby allowing for independent refill of each drug delivery device at a same disease site, or at multiple disease sites. Each drug refill may comprise multiple targets and each delivery device may comprise multiple target recognition moieties. Multiple different drugs may be delivered within one drug refill to the same drug delivery device.

Furthermore, the methods described herein permit segregation of two different molecules through the use of two separate orthogonal chemistry systems. The spatial resolution of two different molecules targeting two different sites in the same animal demonstrates the utility of the drug delivery system described herein in combining incompatible therapies in patients. Administration of the refills can be repeated for multiple times. The drug refills may be repetitively administered for at least 5, 10, 20, 50, 100, 150, 200, 250, 300, 350, 400, 450, 500, 600, 700, 800, 900 or 1000 times.

The drug delivery devices, the drug refill and/or pharmaceutical composition of the present invention are formulated to be compatible with its intended route of administration. Examples of routes of administration include parenteral, *e.g*., intravenous, intraperitoneal, intradermal, subcutaneous, oral (*e.g.,* inhalation), transdermal (*i.e.,* topical), transmucosal, and rectal administration. Solutions or suspensions used for parenteral, intradermal, or subcutaneous application can include the following components: a sterile diluent such as water for injection, saline solution, fixed oils, polyethylene glycols, glycerine, propylene glycol or other synthetic solvents; antibacterial agents such as benzyl alcohol or methyl parabens; antioxidants such as ascorbic acid or sodium bisulfite; chelating agents such as ethylenediaminetetraacetic acid (EDTA); buffers such as acetates, citrates or phosphates, and agents for the adjustment of tonicity such as sodium chloride or dextrose. The pH can be adjusted with acids or bases, such as hydrochloric acid or sodium hydroxide. The parenteral preparation can be enclosed in ampoules, disposable syringes or multiple dose vials made of glass or plastic.

The drug delivery devices, the drug refills, and/or pharmaceutical compositions of the present invention are suitable for injectable use and include sterile aqueous solutions (where water soluble) or dispersions and sterile powders for the extemporaneous preparation of sterile injectable solutions or dispersion. For intravenous administration, suitable carriers include physiological saline, bacteriostatic water, CREMOPHOR EL™ (BASF, Parsippany, N.J.) or phosphate buffered saline (PBS). In all cases, the composition must be sterile and should be fluid to the extent that easy syringeability exists. It must be stable under the conditions of manufacture and storage and must be preserved against the contaminating action of microorganisms such as bacteria and fungi. The carrier can be a solvent or dispersion medium containing, for example, water, ethanol, polyol (for example, glycerol, propylene glycol, and liquid polyethylene glycol, and the like), and suitable mixtures thereof. The proper fluidity can be maintained, for example, by the use of a coating such as lecithin, by the maintenance of the required particle size in the case of dispersion and by the use of surfactants. Prevention of the action of microorganisms can be achieved by various antibacterial and antifungal agents, for example, parabens, chlorobutanol, phenol, ascorbic acid, thimerosal, and the like. In many cases, it will be preferable to include isotonic agents, for example, sugars, polyalcohols such as manitol, sorbitol, sodium chloride in the composition. Prolonged absorption of the injectable compositions can be brought about by including in the composition an agent which delays absorption, for example, aluminum monostearate and gelatin.

Sterile injectable solutions suitable for use in the present invention can be prepared by incorporating a pharmaceutical composition in the required amount in an appropriate solvent with one or a combination of ingredients enumerated above, as required, followed by filtered sterilization. Generally, dispersions are prepared by incorporating the active compound into a sterile vehicle that contains a basic dispersion medium and the required other ingredients from those enumerated above. In the case of sterile powders for the preparation of sterile injectable solutions, methods of preparation are vacuum drying and freeze-drying that yields a powder of the active ingredient plus any additional desired ingredient from a previously sterile-filtered solution thereof.

In some embodiments, the pharmaceutical compositions are prepared with carriers that will protect the compound against rapid elimination from the body, such as sustained/controlled release formulations, including implants and microencapsulated delivery systems. Methods for preparation of such formulations will be apparent to those skilled in the art.

It is especially advantageous to formulate oral or parenteral compositions in dosage unit form for ease of administration and uniformity of dosage. Dosage unit form as used herein refers to physically discrete units suited as unitary dosages for the subject to be treated; each unit containing a predetermined quantity of active compound calculated to produce the desired therapeutic effect in association with the required pharmaceutical carrier. The specification for the dosage unit forms of the invention is dictated by and directly dependent on the unique characteristics of the active compound and the particular therapeutic effect to be achieved, and the limitations inherent in the art of compounding such an active compound for the treatment of individuals.

In some examples, a pharmaceutical composition described herein is incorporated into or onto the drug delivery device, *e.g.,* onto the polymer (*e.g.,* hydrogel, such as alginate) of the device. In such cases, 0-100 mg (*e.g.,* 5-100 mg, 10-100 mg, 20-100 mg, 30-100 mg, 40-100 mg, 50-100 mg, 60-100 mg, 70-100 mg, 80-100 mg, 90-100 mg, 1-95 mg, 1-90 mg, 5-95 mg, 5-90 mg, 5-80 mg, 5-70 mg, 5-60 mg, 5-50 mg, 5-40 mg, 5-30 mg, or 5-20 mg) of the pharmaceutical composition is present in the drug delivery device. For example, the pharmaceutical composition is present in the drug delivery device at a weight/weight concentration of at least 5% (*e.g.,* at least 5%, 10%, 15%, 20%, 25%, 30%, 35%, 40%, 45%, 50%, 55%, 60%, 65%, 70%, 75%, 80%, 85%, 90%, 95%, 98%, 99%, or more).

In some embodiments, the drug delivery device, *e.g*., hydrogel, comprises 0.1 mg to 25 mg pharmaceutical composition per mL of hydrogel, *e.g.,* 0.5 mg to 15 mg, 1 mg to 10 mg, 1 mg to 5 mg, 5 mg to 10 mg, or 1 mg to 3 mg per mL, *e.g*., about 1.6 mg per mL.

In other embodiments, a pharmaceutical composition described herein is conjugated to a drug refill of the invention. For example, the pharmaceutical composition is conjugated to the drug refill polymer, *e.g*., alginate strand, at a weight/weight ratio of 1:100 to 100:1, *e.g.,* 1:100, 5:100, 1:10, 1:5, 3:10, 4:10, 1:2, 6:10, 7:10, 8:10, 9:10, 1:1, 2:1, 3:1, 4:1, 5:1, 6:1, 10:1, 20:1, 30:1, 40:1, 50:1, 60:1, 70:1, 80:1, 90:1, or 100:1. In other examples, the pharmaceutical composition is conjugated to the target molecule of the refill, *e.g.,* at a molar ratio of 10:1 to 1:10, *e.g.,* 10:1, 9:1, 8:1, 7:1, 6:1, 5:1, 4:1, 3:1, 2:1, 1:1, 1:2, 1:3, 1:4, 1:5, 1:6, 1:7, 1:8, 1:9, or 1:10 pharmaceutical composition:target molecule.

The drug delivery device is administered to a subject in need thereof. For example, the subject is a mammal, *e.g*., human, monkey, primate, dog, cat, horse, cow, pig, sheep, or goat. For example, the subject is a human.

The invention will be further described in the following examples, which do not limit the scope of the invention described in the claims.

### EXAMPLES

### DNA synthesis

DNA oligonucleotides were either synthesized using standard automated solid-phase phosphoramidite coupling methods on a PerSeptive Biosystems Expedite 8909 DNA synthesizer or purchased from Integrated DNA Technologies. All reagents and phosphoramidites for DNA synthesis were purchased from Glen Research. Oligonucleotides were purified by reverse-phase HPLC using a C18 stationary phase and an acetonitrile/100 mM triethyl ammonium acetate (TEAA) gradient and quantitated using UV spectroscopy carried out on a Nanodrop ND1000 Spectrophotometer. 3'-thiol DNA was synthesized using 3'-Thiol-Modifier C3 S-S CPG columns. Phosphorothioates were synthesized using 0.05 M Sulfurizing Reagent II in pyridine/acetonitrile. DNA sequences used are summarized in Table 1.

### Alginate Oxidation

Medical grade, high guluronic acid content, high M_{w} alginate (MVG) was purchased from FMC Biopolymers (Princeton, NJ). A 1% solution of sodium alginate (1.0 g, 4 micromoles) in water was mixed with sodium periodate (54 mg, 252 micromoles) at room temperature and stirred. The reaction was stopped after 24 h by the addition of ethylene glycol (30 mg, 28.5 µL). Solution was precipitated with an excess amount of isopropyl alcohol. The precipitates, collected by centrifugation, were redissolved in distilled water and precipitated again with isopropanol. The oxidized alginate was freeze-dried under reduced pressure to yield a white product (0.9 g, 90% yield). The molecular size and hydrodynamic radius were analyzed with gel permeation chromatography (Viscotek) comprised of a laser refractometer, a differential viscometer, and a right angle laser light scattering detector.

### BMPH, Dye-750 and DNA conjugation to alginate

100 mg of alginate (0.4 micromoles, 1 eq.) was dissolved overnight in 100 mL 2-(N-morpholino)ethanesulfonic acid (MES) buffer (100 mM MES, 300 mM NaCl, pH=5.5). 1-ethyl-3-(3-dimethylaminopropyl)carbodiimide (EDC) (7.7 mg, 40 micromoles, Sigma E7750) was added and stirred for 5 minutes. N-beta-Maleimidopropionic acid hydrazide-TFA (BMPH) (5.94 mg, 20 micromoles, Thermo Scientific 22297) and/or HiLyte Fluor™ 750 hydrazide (3.76 mg, 3.6 micromoles, Anaspec 81268) were added and the reaction was stirred for 20 hours. Samples were precipitated in 80% isopropanol, resuspended in water and precipitated again. Samples were dried under high vacuum.

¹H NMR spectra were recorded on a Varian Inova-500 (500 MHz) at ambient temperature in 99.9% D2O. All NMR solvents were purchased from Cambridge Isotope Laboratories.

Alginate-BMPH (10 mg, ∼0.04 micromoles) was dissolved in Tris buffer (100 mM, pH 8.0) overnight. 3'-thiol DNA (0.4 µmoles) was dissolved in 200 µL Tris (100 mM, pH 8.0) and incubated with 20 µL (tris(2-carboxyethyl)phosphine) (TCEP) (0.5M) for 30 minutes. DNA was centrifuged on a 3K MW cut-off (MWCO) centrifugal filter device (Nanosep 3K Omera filter, PALL OD003C33) to remove TCEP and thiol small molecules and mixed with alginate. The mixture was incubated for 20 hours and then transferred to a 100K MWCO centrifugal filter device (Nanosep 100K Omega Filter, PALL OD100C33), centrifuged to remove DNA. Supplemented with 500 µL distilled water and centrifuged again. Samples were diluted to 0.5% alginate, sterile filtered and freeze-dried to dryness. Final weight: ∼9 mg.

### Doxorubixin-alginate coupling

Adipic acid dihydrazide (587 mg, 3.3 mmoles, Sigma A0638) was dissolved in aqueous calcium chloride (1 M) at a concentration of 0.5M. Doxorubicin hydrochloride (10 mg, 17 µmols, Sigma D1515, 10 mg/mL in DMSO) was added to the solution and stirred for 48 hours at 37 °C. Solutions were directly purified on a Agilent 1200 Series Prep-Scale HPLC. Product fractions were freeze-dried. Yield: 40% Observed Product Mass: 700.3 Daltons.
BMPH-conjugated alginate (5 mg) was dissolved in 5 mL Tris-HCl (100 mM, pH 8.0) overnight. To this solution was added 160 mg adipic-doxorubicin (228 µmoles) and stirred overnight. The solution was buffer exchanged into water using a 3K MWCO centrifugal filter device (Nanosep 3K Omera filter, PALL OD003C33). Solution was sterile filtered and freeze-dried. Overall yield: 80%.

### Doxorubicin release from alginate

Doxorubicin or doxorubicin-hydrazde was combined with alginate (oxidized and unoxidized) and gelled with calcium chloride and incubated in PBS at 37 °C. At different time points, solution was changed and doxorubicin concentrations were measured on a molecular devices spectramax plate reader (ex: 530, em: 590).

### In vitro cytotoxicity of alginate-coupled doxorubicin

The cytotoxic effects of hydrazone-linked doxorubicin-alginate and doxorubicin alone were evaluated using the Alamar Blue assay. MDA-MB-231 cells were seeded at a density of 60,000 cells/well in 24-well flat-bottomed plates and incubated for 24 h. Cells were washed twice with PBS and incubated in the culture medium with various concentrations of doxorubicin or alginate-linked doxorubicin for 24 h at 37 °C. Cell viability was evaluated by Alamar blue assay according to vendor instructions and read on a molecular devices spectramax plate reader (ex: 530, em: 590).

### Alginate circulation measurements:

C57/B6 mice were injected retro-orbitally with 100 µL 0.5% fluorescent alginate (unoxidized alginate, Dye-750). Mice were shaved on the front and back and imaged with an IVIS Spectrum in vivo imager (ex: 745, em: 820) over 30 days. Shaving was repeated before every image collection. Fluorescence in the blood was measured through quantification of fluorescence in the snout area.

### In vitro interaction studies (Figure 2)

15-20 µL droplets of 0.5% unoxidized alginate conjugated to polyA-SH were immersed in a calcium chloride solution (100 mM) for 60 minutes. Calcium-alginate gels were washed twice with PBS containing 1 mM calcium chloride. 500 µL of 0.8 µM fluorescent (Fl) oligonucleotides (polyT-Fl or polyA-Fl) were added and incubated for 1, 5, 30 minutes or 16 hours on an orbital shaker. Samples were washed twice with 500 µL PBS (1 mM CaCl₂) and imaged on a fluorescence microscope under the green channel. For quantification, gels were de-crosslinked in 100 µL 100 mM EDTA for 30 minutes and fluorescence read on a molecular devices spectramax plate reader (excitation 485, emission 538, cutoff 530).

### In vitro interaction studies (Figure 3)

15-20 µL droplets of DNA-conjugated alginate (0.5% unoxidized alginate conjugated to polyA-SH, polyT-SH or unconjugated) were immersed in a calcium chloride solution (100 mM) for 60 minutes. Calcium-alginate gels were washed twice with PBS containing 1 mM calcium chloride. 500 µL of 0.05% alginate conjugated to polyT-SH/HEX (Figs. 3A-C) or to polyA-SH and Dye-750 was added and incubated for 5, 10, 30, 60 minutes (Figs. 3A-C) or 30 mins (Figs. 3D-F) on an orbital shaker. Samples were washed twice with 500 µL PBS (1 mM CaCl₂) and imaged on a fluorescence microscope (green channel) or on an IVIS Spectrum CT in vivo imager (ex: 745, em: 820). For quantification, gels for Figs. 3A-C were de-crosslinked in 100 µL 100 mM Ethylenediaminetetraacetic acid (EDTA) for 30 minutes and fluorescence read on a molecular devices spectramax plate reader.

### In vivo Hydrogel Homing

All animal experiments were performed according to established animal protocols. Tumors were created by injecting C57/B6 mice subcutaneously with 100,000 B16-F10 melanoma cells (American Type Culture Collection, VA) in 100 µL PBS in the back of the neck. Animals were monitored for the onset of tumour growth (approximately 2 weeks). 20 mg/mL alginate conjugated to DNA (polyT-SH or polyA-SH) was crosslinked with 4% wt/v CaSO₄ (1.22 M) and 50 µL was injected into the center of tumors with a 23-gauge needle. Any covering hair on the tumors was shaved.

One day later, mice were injected retro-orbitally with 100 µL 0.5% DNA-conjugated fluorescent alginate (unoxidized alginate, polyA-SH, Dye-750). Mice were imaged every 24 hours for six days on an IVIS Spectrum *in vivo* imager (ex: 745, em: 820). Mice were euthanized for humane reasons when tumours grew to > 20 mm in one direction or when the tumor became ulcerated through skin with a non-healing open wound present. Mice that died or had to be euthanized prior to completion of experiment were not included in the analysis.

### Xenograft Tumor Studies

All animal experiments were performed according to established animal protocols. Tumors were created by injecting 10⁶ MDA-MB-231 cells (American Type Culture Collection, VA) combined with Matrigel (BD Biosciences, CA) to a total volume of 200 µL (100 µL PBS and 100 µL Matrigel) into the hind limbs of 8 week old J:Nu (*Foxn1^{nu}*/*Foxn1^{nu}*) mice (Jackson Labs, ME). 35 days following tumor inoculation tumors were injected with alginate gels. 20 mg/mL alginate conjugated to DNA (thioT-SH or unconjugated) was mixed with doxorubicin (1.6 mg drug per mL of gel), then crosslinked with 4% wt/v CaSO₄ (1.22 M) and then 50 µL of gel were injected into tumors with a 23-gauge needle. 2, 3, 4, and 5 weeks after initial tumoral gel injection, mice were injected retro-orbitally with 100 µL 0.5% DNA-conjugated alginate carrying doxorubicin (5% oxidized alginate, thioA-SH, Dye-750, 160 µg Dox-hydrazide = 120 µg Dox). Throughout the study, tumor area was measured twice per week with digital calipers in two dimensions and the product of the two dimensional values was used to approximate tumor area. Mice were sacrificed on day 49. Mice that died or had to be euthanized prior to completion of experiment were not included in the analysis.

### 3-(p-Benzylamino)-1,2,4,5 Tetrazine synthesis

3-(p-Benzylamino)-1,2,4,5-tetrazine was synthesized according to standard methods. For example, 50 mmol of 4-(Aminomethyl)benzonitrile hydrochloride and 150 mmol formamidine acetate were mixed while adding 1 mol of anhydrous hydrazine. The reaction was stirred at 80 °C for 45 minutes and then cooled to room temperature, followed by addition of 0.5 mol of sodium nitrite in water. 10% HCl was then added dropwise to acidify the reaction to form the desired product. The oxidized acidic crude mixture was then extracted with DCM, basified with NaHCO₃, and immediately extracted again with DCM. The final product was recovered by rotary evaporation, and purified by HPLC. Chemicals were purchased from Sigma-Aldrich.

### Azide and Tetrazine conjugation to alginate

200 mg of alginate (0.8 micromoles, 1 eq.) was dissolved overnight in 200 mL MES buffer (100 mM MES, 300 mM NaCl, pH=5.5). 11-Azido-3,6,9-trioxaundecan-1-amine (349 mg, 1.6 mmole, 2000 eq, Sigma-Aldrich 17758) or tetrazine-amine (300 mg, 1.6 mmole, 2000 eq) was added to the solution and stirred for an additional 1 hour at room temperature. A mixture of 1-Ethyl-3-[3-dimethylaminopropyl]carbodiimide hydrochloride (306.7 mg, 1.6 mmole, 2000 eq, Sigma-Aldrich E7750) and sulfo-N-hydroxysuccinimide (173 mg, 800 µmoles, 1000 eq, Thermo Fisher 24510) was added in three equal doses eight hours apart and stirred for an additional eight hours. Samples were dialyzed against 4 L of water with successively lower NaCl content, changing solution 2-3 times per day. NaCl per 4 L of water: 30 g, 25 g, 20 g, 15 g, 10 g, 5 g, 0 g, 0 g, 0 g, 0 g, 0 g. Samples were lyophilized under high vacuum. Final weight: 150-160 mg. Yield: 75-80%. ¹H NMR spectra were recorded on a Varian Inova-500 (500 MHz) at ambient temperature in 99.9% D₂O. Deuterium oxide was purchased from Cambridge Isotope Laboratories. For estimation of substitution, azide peak at 1-1.2 ppm and tetrazine peak at 7.5, 8.5 and 10.4 ppm were compared to the three urinate protons between 3 and 4.2 ppm. See Figures 17-18.

### In vitro interaction studies azide-DBCO

15-20 µL droplets of 2% unoxidized alginate conjugated to azide or unconjugated controls were immersed in a calcium chloride solution (100 mM) for 60 minutes. Calcium-alginate gels were washed twice with PBS containing 1 mM calcium chloride. 500 µL of 100 µM Cy7-DBCO (Click Chemistry Tools) were added and incubated for four hours on an orbital shaker. Samples were washed twice with 500 µL PBS (1 mM CaCl₂). For quantification, gels were digested with 3.5 mg/mL alginate lyase (Aldrich A1603) in 100 µL PBS overnight and fluorescence read on a molecular devices spectramax plate reader (excitation 745, emission 780). Reaction with click partner was also confirmed through incubation of calcium-crosslinked gels with 100 equivalents of DBCO-Cy7 (alginate-azide) followed by attenuated total reflectance (ATR)/infrared (IR) spectroscopy (ATIR) analysis. ATIR was measured on a Vertex70 machine with atmospheric adjustment. See Figures 19-20.

### In vitro interaction studies tetrazine-TCO

15-20 µL droplets of 2% unoxidized alginate conjugated to tetrazine or unconjugated controls were immersed in a calcium chloride solution (100 mM) for 60 minutes. Calcium-alginate gels were washed twice with PBS containing 1 mM calcium chloride. 500 µL of 100 µM Cy7-TCO (Click Chemistry Tools) were added and incubated for four hours on an orbital shaker. Samples were washed twice with 500 µL PBS (1mM CaCl₂). For quantification, gels were digested with 3.5 mg/mL alginate lyase (Aldrich A1603) in 100 µL PBS overnight and fluorescence read on a molecular devices spectramax plate reader (excitation 745, emission 780). Reaction with click partner was also confirmed through incubation of calcium-crosslinked gels with 100 equivalents of TCO-Cy5 (alginate-tetrazine) followed by ATIR analysis. ATIR was measured on a Vertex70 machine with atmospheric adjustment. See Figures 21-22.

### Mouse Model of Hind-limb Ischemia and Alginate Gel Implantation

8-week old, female CD-1 background outbred strains (Charles River Laboratories, MA). Animals were anesthetized by intra-peritoneal injections of ketamine (80 mg/kg) and xylazine (5 mg/kg). Hindlimb ischemia was induced by unilateral external iliac artery and vein ligation. At the time of surgery, mice were randomized to one of two groups (n = 3 per group). 20 mg/mL alginate conjugated to azide or tetrazine or unconjugated control was crosslinked with 4% wt/v CaSO₄ (1.22 M) in PBS and 50µL alginate-azide/tetrazine hydrogel was injected through a 25 gauge needle near the distal end of the ligation site (n=3 each group). In control animals following surgery, unconjugated alginate was injected intramuscularly. Incisions were closed by 5-0 Ethilon sutures (Johnson & Johnson, NJ). Ischemia in the hindlimb was confirmed by laser Doppler perfusion imaging (LDPI) system (Perimed AB, Sweden).

### In Vivo Hydrogel Targeting

24 hours after surgery and gel implantation, mice were injected retro-orbital with 100 µL 20 µg/mL Cy7-TCO or Cy7-DBCO (Click Chemistry Tools). Mice were imaged at 1, 5, 30 minutes, 6 and 24 hours after IV-injection on an IVIS Spectrum in vivo imager (ex: 745, em:820). For repeated gel targeting, mice bearing alginate-Az were re-injected with 20ug/mL Cy7-TCO and imaged on the IVIS Spectrum daily. Mice did not show adverse effects from these administrations and no mouse had to be euthanized prior to completion of experiment. No mice were excluded from the analysis for any reason.

### Spatial Segregation of Molecular Targeting

Hind-limb ischemia was induced as described above, and alginate-tetrazine gels implanted intra-muscularly (n=3). Alginate-azide gels (n=3) were injected into the mammary fat pad of mice on the opposite side from the ischemic limb. A mixture of 100 µL Cy5-TCO (Click Chemistry Tools, 20 µg/mL) and Cy7-DBCO (Click Chemistry Tools, 20ug/mL) was injected retro-orbital. Mice were imaged 48 hours after IV injection in the Cy5 (Ex: 640, Em:680) and Cy7 (Ex: 740, Em: 820) regions and an optical image. Regions of Interest (ROIs) were placed blind based on the optical image based on visible gel swelling in mammary fat pad and sutures in the hind-limb. Fluorescence was quantified based on "Radiance" in the areas.

### Muscle Isolation and Fluorescence Quantitation

24-hours after IV injection of Cy7-DBCO, mice (n=3) were sacrificed. Both alginate-azide-injected and control hindlimb muscles were isolated. Samples were digested for 2 hours in 2 mL of 250 units/ml collagenase II, 1 unit/mL dispase II and 1 mg/mL alginate lyase. Samples were centrifuged (5 mins, 500 g) supernatant removed and pellet was resuspended and digestion repeated. The two digests were combined together and quantified on a molecular devices spectramax plate reader (excitation 745, emission 780) using a Cy7-DBCO dilution series for quantitation.

### Statistical Testing

All data was compared based on pair-wise comparison using the two-tailed, homoscedastic Students' t-test.

### Oral Delivery and Hydrogel Targeting to Alginate-Azide

24 hours after surgery and gel implantation, mice were administered through oral gavage 100 µL 1 mg/mL Cy7-DBCO (Click Chemistry Tools). Mice were imaged at 1, 30 minutes, 6 and 24 hours and every day for 1 week after the oral administration on an IVIS Spectrum in vivo imager (ex: 745, em:820). Fluorescence was quantified at the alginate injection site and on the mirror location in the contralateral (ctrl) limb. Mean fluorescence six days after IV-injection is shown in Figure 14. Error bars show SEM. p-value represents a one-tailed t-test either paired (against Alg-Az Ctrl conditions) or unpaired (the other conditions).

### Example 1: Alginate circulation time in the blood

Efficient blood-based refilling of drug payloads relies on sufficient circulation lifetimes that allow payloads to encounter and bind to the primary device. The circulation time of alginate (285 KDa, 44 nm hydrodynamic radius (Rh)) conjugated to a near-IR probe (Fig. 6A) was analyzed following intravenous (IV) administration to mice. Quantification of fluorescence (Fig. 6B) demonstrated that this alginate remained in circulation for at least 14 days, with a circulatory half-life of about seven days (Fig. 6C). Imaging of individual organs revealed accumulation in the lungs, liver, spleen, and to a lesser extent in the kidneys (Fig. 6D), demonstrating that all of these organs contribute to removal of the circulating alginate from the bloodstream. With a long circulation time, alginate can serve as an efficient intravascular drug carrier with the capability of extravasating and interacting with the primary drug delivery device.

### Example 2: DNA-mediated binding of drug-surrogates to alginate gels in vitro

Experiments were performed to determine whether device refilling with drug payloads could be mediated by complementary DNA binding between target calcium-alginate gel and free alginate strands conjugated to a drug payload. DNA (see Table 1 for a list of DNA used) was conjugated by its 3' end to alginate strands at a ratio of two molecules of DNA coupled per molecule of alginate. The ability of alginate-conjugated DNA to retain nucleic acid binding-activity was then tested. Alginate conjugated to (T)₂₀ (SEQ ID NO: 1) oligonucleotides was ionically crosslinked with calcium to form a gel and then was incubated with fluorescently-labeled complementary (A)₂₀ (SEQ ID NO: 2) or non-complementary (T)₂₀ (SEQ ID NO: 1) oligonucleotides (Fig. 2A) in phosphate buffer with 1mM calcium chloride. Complementary oligonucleotides bound to the gel surface in a sequence-specific manner while non-complementary oligonucleotides showed little binding (Figs. 2B-C).

The ability of DNA-conjugated alginate gels to bind soluble, DNA-conjugated alginate strands was next tested *in vitro,* as a model for drug refilling *in vivo.* DNA-conjugated or unconjugated alginate was gelled and incubated with free alginate strands coupled to fluorescently-labeled complementary DNA for variable times in a buffer with physiological calcium concentration (Fig. 3A). Alginate strands bearing complementary DNA specifically bound to DNA-bearing gel surfaces, with about 5-fold selectivity as compared to control gel surfaces unconjugated to DNA; this selectivity was constant over time (Figs. 3B-C). To more completely represent a drug-bearing alginate-DNA conjugate, alginate conjugated to DNA was modified along its backbone with a near-IR dye to mimic drug loading, and tested for association with complementary or non-complementary DNA-bearing alginate gels. With one hour of incubation, the fluorescently-labeled alginate strands selectively bound to complementary-DNA-bearing gels, as compared to control gels bearing non-complementary DNA (Figs. 3D-F).

Due to the possibility for non-DNA mediated interactions in the presence of calcium that could crosslink free alginate strands to the gels, *in vitro* alginate binding studies were preformed under a physiologically-relevant soluble calcium concentration of 1 mM. Under these conditions, a small amount of alginate does bind to the gels in a DNA-independent manner *in vitro* (Figs. 3C and 3F). However, DNA-mediated binding of the alginates outpaced non-specific interactions by five-fold. This data show that DNA-conjugated alginate strands bind to complementary DNA-conjugated alginate gels.

### Example 3: In Vivo DNA-mediated alginate homing

Experiments were performed to determine whether fluorescently-labeled free alginate strands could home *in vivo* to a target gel through DNA-mediated targeting. DNA was conjugated to alginate through the 3' end to increase serum exonuclease stability. See, *e.g.,* Shaw J, Kent K, Bird J, Fishback J, & Froehler B (1991) Nucleic acids research 19(4):747-750; Floege J, et al. (1999) The American journal of pathology 154(1): 169-179; and Gamper H, et al. (1993) Nucleic acids research 21(1):145-150. A melanoma cancer model was chosen for these studies due to the well established enhanced permeability and retention effect in these tumors, which provides a means for passive accumulation of bloodborne nanoparticles in tumor tissue. See, e.g., Maeda H, Wu J, Sawa T, Matsumura Y, & Hori K (2000) Journal of controlled release: official journal of the Controlled Release Society 65(1-2):271-284. Mice bearing tumors between 10-20 mm³ in size received intra-tumor injections of DNA-conjugated calcium-crosslinked alginate gels. At 24 hours, fluorescently-labeled free alginate strands conjugated to complementary-DNA were administered intravenously. Imaging of mice first revealed that IV-administered strands collected in the tumors of all mice at 24 hours post-administration, likely through the EPR effect (Figs. 4A-B). In mice receiving free alginate strands conjugated to complementary DNA, the strands continued to aggregate in the tumors over the next few days in a statistically significant manner relative to controls. Over the course of one week, mice receiving non-complementary DNA-conjugated gels, or those not injected with gel showed a progressive loss of fluorescence in the tumor, reaching background level of fluorescence by day 5. In contrast, mice receiving complementary DNA-conjugated gels showed continued accumulation of fluorescence on days 2 and 3 after injection and significant retention of fluorescent alginate in the tumor. Total alginate retention is quantified as the area under the curve (Fig. 4C); DNA-mediated alginate homing produced significantly higher free strand retention in the tumor as compared to controls. This data indicate that alginate strands containing a DNA target molecule accumulated at the site of a previously administered alginate drug delivery device containing the complementary DNA as a target recognition moiety.

### Example 4: Drug refilling slows tumor growth

The therapeutic efficacy of the refillable drug delivery device described herein was analyzed by examining the ability of the targeting technology to inhibit tumor growth over several weeks. Drug-delivering alginate hydrogels destined for intra-tumor injection demonstrated sustained release of doxorubicin over a period of weeks (Fig. 8). To carry IV-administered drug payloads, alginate strands were partially oxidized to introduce aldehyde functional groups, allowing coupling of hydrazine-doxorubicin through a hydrolyzable hydrazone linker. See, *e.g.,* Bouhadir K, et al. (2001) Biotechnology progress 17(5):945-950; and Bouhadir K, Alsberg E, & Mooney D (2001) Biomaterials 22(19):2625-2633. Oxidized alginate demonstrated sustained release of doxorubicin (Fig. 9B) without inhibiting the cytotoxicity of released doxorubicin (Fig. 9C).

The breast cancer MDA-MB-231 model was chosen because it is a slow-growing tumor, widely used to test chemotherapeutic strategies. See, *e.g.,* Choi K, et al. (2011) ACS nano 5(11):8591-8599; and Tien J, Truslow J, & Nelson C (2012) PloS one 7(9). Immunocompromised mice bearing MDA-MB-231 xenograft tumors were injected intra-tumor with phosphorothioate DNA-conjugated alginate gels releasing doxorubicin (80 µg per animal) or bolus doxorubicin in PBS. After two weeks, mice received weekly IV administrations of free alginate strands conjugated to complementary phosphorothioate DNA and doxorubicin (120 µg per animal), or bolus doxorubicin (120 µg per animal) as a control (Fig. 5A). Targeted drug refilling inhibited tumor growth significantly, as compared to treatment with bolus doxorubicin control (Fig. 5B). The ability of targeted therapy to reduce tumor size was assessed by monitoring the change in tumor size in the three days following each IV administration. Tumors shrank after the first two drug targeted treatments, but continued to grow in mice receiving bolus doxorubicin controls (Fig. 5C).

Strikingly, the first two refillings of the gel appeared to yield the greatest impact, with a less pronounced effect for the second two refillings. This may have been due to saturation of DNA strands on the targeted gel or degradation of the phosphorothioate oligonucleotides on the gel over the course of the experiment. Alternatively, the drug delivery system may have shrunk the tumor to a size in which enhanced permeability was no longer prominent, leading to inefficient targeting.

These results demonstrated that drug reloading using these methods had a significant clinical benefit. In addition, experiments were performed to further confirm that the effect was specific to the interaction of complementary DNA strands *in vivo* and that differences in doxorubicin pharmacokinetics or release could not account for the effects seen. Specifically, the following additional controls were tested (see Table 2): 1) intratumoral injections of alginate with encapsulated doxorubicin but no conjugated DNA, coupled with IV administration of DNA-conjugated alginate and hydrazone-linked doxorubicin, 2) intratumoral injections of alginate gels with encapsulated doxorubicin but no bound DNA, coupled with IV administration of free doxorubicin.

**Table 2: Tumor therapy experimental groups**

| Group | Intratumor Injection | Retro-orbital Injections |
|---|---|---|
| Targeted therapy | 50µL of alginate gel (2% w/v in PBS; ionically crosslinked) conjugated to thioT-DNA and mixed with doxorubicin (0.08 mg/animal) | 100 µL of alginate (0.5% w/v in PBS, 5% oxidized) conjugated to thioA-DNA and dox-hydrazide (0.12 mg dox/animal) |
| No DNA control | 50 µL of alginate gel(2% w/v in PBS; ionically crosslinked) mixed with doxorubicin (0.08 mg/animal) | 100 µL of alginate (0.5% w/v in PBS, 5% oxidized) conjugated to thioA-DNA and dox-hydrazide (0.12 mg dox/animal) |
| Bolus IV control | 50 µL of alginate gel (2% w/v in PBS; ionically crosslinked) mixed with doxorubicin (0.08 mg/animal) | 100 µL doxorubicin (0.12 mg/animal) in PBS |
| All bolus ctrl | 50 µL of PBS with doxorubicin (0.08 mg/animal) | 100 µL doxorubicin (0.12 mg/animal) in PBS |

The total dose of drug injected directly into the tumor and delivered via IV administration was kept constant across all groups, as was the frequency of IV administration. After 7 weeks of tumor growth, tumors treated with the drug reloading technology were markedly smaller than tumors in any of the control conditions (Fig. 5D). Quantification of the tumor sizes confirmed the smaller size of the tumors treated with the drug reloading technology compared to the controls (p<0.05 targeted compared to first two controls, Fig. 5E). This data demonstrate that systemic administration of a cancer drug refill to recharge a drug delivery device previously administered intra-tumorally reduced tumor size in a breast cancer mouse model.

### Example 5: Specific binding of bioorthogonal functional groups to hydrogels

The binding specificity of fluorescently-labeled bioorthogonal functional groups (e.g., trans-cyclooctene (TCO) and dibenzycyclooctyne (DBCO) molecules) to hydrogels was evaluated *in vitro.* Polymer, *e.g.,* alginate, was modified with either tetrazine (Tz) or azide (Az) through carbodiimide chemistry. Nuclear Magnetic Resonance (NMR) analysis demonstrated that, on average, 100 molecules of Tz or Az were attached to each polymer *(e.g.,* alginate) strand, constituting 400 nanomoles of target *(e.g.,* bioorthogonal functional group, *e.g.,* Tz or Az) per mg of polymer. The functional groups, *e.g.,* Tz or Az, were labeled with a fluorescent moiety, *e.g*., Cy7, for detection.

Cy7-labeled trans-cyclooctene specifically bound to tetrazine-modified alginate, but not to unmodified alginate (Figures 10A,C). Similarly, fluorescent DBCO bound alginate-azide gels but had little interaction with unmodified alginate gels (Figures 10B,D). This data indicate that bioorthogonal functional groups bind specifically to hydrogels modified with the corresponding binding partner of the functional group.

### Example 6: Bioorthogonal functional groups target gels in vivo

Experiments were performed to determine whether bioorthogonal functional groups labeled with near-IR (NIR) fluorophores could target gels *in vivo. In vivo* gels were resident at a disease site in an animal model of lower limb ischemia. 50 µL of tetrazine-modified, azide-modified, or unmodified alginate gels were implanted intra-muscularly in the limbs of mice subjected to femoral artery ligation, in a similar manner as described in Chen et al. J. Pharmaceutical research 24(2006):258; and Silva et al. J. Biomaterials 31(2010):1235. Twenty-four hours post-surgery, NIR-labeled DBCO or TCO-molecules were administered intravenously (IV), and the animals were monitored over 24 hours through live animal fluorescence imaging. The NIR-labeled small molecules circulated in the animal's system and were eliminated mainly through the bladder in the first 24 hours (Figures 16A-B) After 24-hours, fluorescence was observed in the limbs implanted with modified gels (e.g., Tz- or Za-modified gels), but not in control gels, which lacked the target recognition motifs (e.g., Tz or Az) (Figure 11).

To quantify the dose of circulating molecules delivered to intra-muscular gels, azide-modified gels were isolated from mouse muscles and digested. The amount of targeted molecules on the gels was quantified by fluorescence. An average of 106 picomoles of targeting compound, corresponding to 6.5% of the initially injected dose, localized to the intra-muscular disease area, as shown in the table below.

**Quantitation of small molecule targeting in muscles**

| Site | Moles SM | St. Dev |
|---|---|---|
| Ischemic Limb with Alginate-Az gel | 6.54% | 3% |
| Control Limb (no gel) | 0.12% | 0.1% |

This number of molecules that were targeted to the gels constitutes 0.03% of the total available azide sites on the gel, demonstrating that multiple gel fillings/refillings are possible for each gel due to the excess target recognition sites. This data show that systemically administered bioorthogonal functional groups accumulate at the site of a previously administered/implanted drug delivery device. Because a single injected dose targets about .03% of the available azide sites on the gel, the device may be refilled up to 3,300 times. For example, the gel in Fig. 5A is refilled four times, while the gel in Fig. 12A is refilled nine times over the period of one month. In some cases, with respect to reversible/cleavable chemistry, toehold exchange (WO 2012058488 A1) is utilized to reverse binding of DNA-conjugated nanoparticles to the gel.

### Example 7: Repeated administrations of bioorthogonal functional groups to target and refill gels in vivo

Multiple intravascular administrations were performed to repeatedly target and fill an intramuscular gel in injured mice. Mice with hind limb ischemia carrying azide-modified gels (e.g., alginate-Az gels) were repeatedly administered NIR-labeled DBCO, approximately once every three days for one month (Figure 12A). Over the one-month time course, limb fluorescence increased in a step-wise fashion, corresponding to fluorophore administrations (Figure 12B), with each dose increasing fluorescence to a similar extent (Figure 12C). Thus, gel homing using the methods described herein was achieved in an animal model of ischemia. A small but not significant decrease in fluorescence in the limb was observed between 24 and 48 hours after IV injection, likely due to residual unbound fluorophores removed from the gel. This data demonstrate that systemically administered drug refills accumulate at the site of the drug delivery device each time the refill is administered. The same device can be recharged multiple times by systemically administed refills.

### Example 8: Targeting of two different bioorthogonal functional groups to two separate gel sites in vivo

Experiments were conducted to determine whether two different bioorthogonal functional groups, *e.g.,* DBCO and TCO, could selectively home to their respective binding partners (*i.e.,* target recognition moieties, *e.g.,* molecules) to achieve spatial separation of drug-devices in the same animal. Two sites on a mouse were used. Tetrazine-modified gels were implanted intra-muscularly in the hind limb of mice subjected to hind-limb ischemia as a first target site. Azide-modified gels were implanted into the mammary fat pad of the same mice as a second target site. A mixture of Cy7-labeled DBCO and Cy5-labeled TCO was administered intravenously 24 hours after gel administration. The two bioorthogonal functional groups were efficiently and specifically targeted to their respective disease sites (Figure 13). This data indicate that systemic administration of two different types of drug refills leads to accumulation of each type of drug refill at its respective drug delivery device site.

### Example 9: Oral delivery and targeting to alginate-azide hydrogel

Mouse models of hind limb ischemia were generated as described above. Hydrogels containing alginate modified with azide were implanted intramuscularly into the ischemic site in the mice. 24 hours after surgery and gel implantation, mice were orally administered Cy7-DBCO, *e.g*., through oral gavage. Mice were imaged 1, 30 minutes, 6, and 24 hours, and every day for 1 week after the oral administration. Fluorescence was quantified at the alginate implantation site and on the mirror location in the contralateral limb (control limb). Orally administered Cy7-DBCO was targeted to the azide-modified alginate hydrogel. See Figure 14. These data indicate that oral delivery of a drug refill leads to accumulation of the orally administered drug at the site of the alginate drug delivery device.

### Example 10: Targeting DBCO To Gels Modified With Azide

A set of images showing targeting of the IV-injected fluorescently labeled DBCO to an intraosseous gel modified with azide or unmodified control gel in the femur of a mouse is shown in Fig. 23. 50 µL of azide-modified or unmodified alginate gels were injected intraosseus into the bones of mice. Twenty-four hours post-surgery, NIR-labeled DBCO molecules were administered intravenously (IV), and the animals were monitored over 24 hours through live animal fluorescence imaging. After 24-hours, fluorescence was observed in the limbs implanted with modified gels (*e.g*., Az-modified gels), but not in control gels, which lacked the target recognition motifs (*e.g.,* Az).

As shown in Fig. 24, IV-injected fluorescently labeled DBCO was targeted to a gel modified with azide injected into the right knee joint of a mouse. 50 µL of azide-modified alginate gels were injected into knee joint of mice. Twenty-four hours post-surgery, NIR-labeled DBCO molecules were administered intravenously (IV), and the animals were monitored over 24 hours through live animal fluorescence imaging. After 24-hours, fluorescence was observed in the limbs implanted with modified gels (e.g., Az-modified gels).

As shown in Fig. 25, IV-injected fluorescently-labeled DBCO was targeted to a gel modified with azide compared to control gel injected into the tumor of a mouse. The images in Figure 25 were taken 24 hours after IV injection. Lewis Lung Carcinoma (LLC) tumors were induced in C57B6 mice. Once the tumors were 5-8 mm in diameter, 50 µL of azide-modified alginate gels were injected into the tumors. Twenty-four hours post tumoral injection, NIR-labeled DBCO molecules (see, Fig. 26) were administered intravenously (IV), and the animals were monitored over 24 hours through live animal fluorescence imaging. After 8-hours, fluorescence was observed in the tumors implanted with modified gels (*e.g.,* Az-modified gels), but not in control gels, which lacked the target recognition motifs (*e.g.,* Az).

As shown in Fig. 28, IV-injected fluorescently-labeled DBCO was targeted to a gel modified with azide compared to control gel injected into the tumor of a mouse. The images in Figure 28 were taken 24 hours after IV injection. MDA-MB-231 breast cancer tumors were injected together with control alginate or alginate-azide gels into mice. 30 days after injection of gel, mice were treated with Cy7-DBCO intravenously, and the animals were imaged through live animal fluorescence imaging after 48 hours. Mice carrying tumor and control alginate gels showed significantly less fluorescence localization to tumor than mice carrying alginate-azide gel in tumor.

An illustration showing capture of a small molecule by an azide-modified gel and the subsequent release of the small molecule through hydrolysis after the capture is shown in Fig. 26. The molecule in this example is a Cy7 fluorophore conjugated to DBCO through a hydrolyzable hydrazone linker.

As shown in Fig. 27A, IV-injected small molecule was targeted to a gel modified with azide injected into the tumor of a mouse. The small molecule, a Cy7 fluorophore conjugated to DBCO through a hydrolyzable hydrazone linker, is subsequently released, leading to loss of the Cy7 fluorescence signal. A line graph showing the quantification of the small molecule at the tumor site over time (hours) demonstrating release of the small molecule is illustrated in Fig. 27B. Lewis Lung Carcinoma (LLC) tumors were induced in mice. Once the tumors were 5-8 mm in diameter, 50 µL of azide-modified alginate gels were injected into the tumors. Twenty-four hours post tumoral injection, NIR-labeled DBCO molecules (see, Fig. 26) were administered intravenously (IV), and the animals were monitored over four days through live animal fluorescence imaging. Fig. 27B shows quantitation of tumor fluorescence 24, 48, 72 and 96 hours after IV fluorophore administration showing that the fluorescence decreases over time as Cy7 fluorophore is cleaved from the gel and is cleared from the tumor.

### Example 11: Synthesis and Characterization of Doxorubicin Prodrug Coupled With Bioorthogonal Functional Groups

Experiments were performed to synthesize a prodrug molecule which coupled doxorubicin with a bioorthogonal functional group, DBCO (Fig. 29A). DBCO-maleimide (5 mg, Santa Cruz, sc-397265A) was dissolved in dimethylformamide (500 µL) and to this was added 6.21 µL ethanedithiol (Sigma 02390). The reaction was vortexed for 10 seconds and allowed to sit for 10 minutes. Sample was purified on HPLC with a gradient of 5% acetonitrile/95% TEAA to 75% acetonitrile/25% TEAA over 10 minutes on a zorbax C18 5 µm HPLC column. HPLC fractions corresponding to the product were rot-evaped to dryness to lead to the DBCO-thiol with 70% yield.

Doxorubicin-EMCH (5.25 mg MedKoo Biosciences 201550) was dissolved in 525 µL DMSO and added to dry DBCO-thiol. The reaction was swirled to dissolve DBCO-thiol and allowed to sit for 5 minutes. Sample was purified on HPLC with a gradient of 5% acetonitrile/95% TEAA to 75% acetonitrile/25% TEAA over 10 minutes on a zorbax C18 5 µm HPLC column. HPLC fractions corresponding to the product were lyophilized to dryness to yield prodrug in 60% yield.

Quantification of prodrug was done by dissolving the prodrug in 40% 2-hydroxypropyl beta cyclodextrin. Concentration was determined through measuring the absorbance of prodrug solutions at 498 nm and comparing to the extinction coefficient of doxorubicin. The material was diluted to standard concentrations, sterile filtered, aliquoted and frozen for animal experiments.

Active doxorubicin could be released from the prodrug by hydrolysis (Fig. 29B). The kinetics of hydrolysis of the doxorubicin prodrug was analyzed by examining the rate of the hydrolysis reaction at different pH conditions. Doxorubicin prodrug was prepared at a concentration of 172 nM in phosphate buffered saline of different pH ranging from pH 5.5 to pH 7.5 (Fig. 29C). Cleavage of prodrug was monitored by liquid chromatography / mass spectrometry through integration of the product peak over time. As shown in Fig. 29C, hydrolysis of the prodrug occurred at a faster rate when the pH of the prodrug solution was lower, whereas the hydrolysis reaction slowed down when the pH increased.

Cell toxicity assays for free doxorubin and doxorubicin prodrug were performed using cancer cells Lewis Lung Carcinoma LLC1. LLC1 cells were plated in 96-well plates 24 hours prior to start of toxicity experiments. Doxorubicin and prodrug were dissolved in 1X phosphate buffered saline (pH 7.4) at different concentrations from 0.02 µM to 50 µM. These concentrations were added in triplicate to cancer cells and incubated for 1 hour. After incubated, the cells were washed 2X with DMEM and cells were cultured for 24 hours. Cells were tested for metabolic activity through addition of 10 µL alamar blue solution (Thermo Scientific 88951) for 1 hour and read on a fluorescence plate reader 530/580 excitation/emission spectrum. As demonstrated in Fig. 29D, the doxorubicin prodrugs were significantly less toxic than free doxorubicin.

Having demonstrated that doxorubin could be released from the doxorubicin prodrug upon hydrolysis, the ability of doxorubicin to be released from the drug delivering azide-modified alginate hydrogels was subsequently analyzed (Fig. 29E). 400 µL of calcium-crosslinked azide-modified alginate hydrogels were combined with the prodrug (0.14 mg) and mixed for 4 hours. 50 µL of resultant hydrogel-prodrug conjugates were injected into 1.7 mL tubes and incubated in 1 mL Tris-HCll solution. The solution was changed daily and released doxorubicin was measured through fluorescence spectroscopy of the removed solution. Azide-alginate demonstrated sustained release of doxorubicin over a period of weeks (Fig. 29E).

Those skilled in the art will recognize, or be able to ascertain using no more than routine experimentation, many equivalents to the specific embodiments of the invention described herein. Such equivalents are intended to be encompassed by the following claims.

## Claims

1. A system comprising a drug delivery device and a drug refill,
wherein the drug delivery device comprises a carrier and a target recognition moiety and is suitable for implantation in a desirable location within a subject;
wherein the drug refill comprises a pharmaceutical composition and a target, wherein the pharmaceutical composition is attached to the target via a cleavable linker capable of being cleaved within the subject;
wherein the target and the target recognition moiety form a two-component binding pair;
wherein the drug refill is mobile until the target on the drug refill binds to the target recognition moiety on the drug delivery device;
wherein upon binding of the target to the target recognition moiety, the drug refill delivers the pharmaceutical composition directly to the drug delivery device, thereby refilling, the drug delivery device; and
wherein the pharmaceutical composition is released at the desirable location within the subject in a controlled manner over a time scale of days, weeks, months, or years, upon cleavage of the cleavable linker.

2. The system of claim 1, wherein the pharmaceutical composition comprises a small molecule or a biologic.

3. The system of claim 1, wherein the pharmaceutical composition is unmasked after delivery into the drug delivery device; optionally wherein
the pharmaceutical composition is unmasked by separating it from the target; optionally wherein
the target is separated from the pharmaceutical composition by cleaving a linkage between the pharmaceutical composition and the cleavable linker, a linkage within the cleavable linker, or a linkage between the pharmaceutical composition and the target; optionally wherein
the linkage between the pharmaceutical composition and the cleavable linker, the linkage within the cleavable linker, or the linkage between the pharmaceutical composition and the target is cleaved by enzyme degradation, hydrolysis or reduction of the linkage; or optionally wherein
the linkage between the pharmaceutical composition and the cleavable linker comprises a linkage selected from the group consisting of a hydrazine linkage, a hydrazide linkage, a hydrazone linkage, an acetal linkage, a ketal linkage, an oxime linkage, an imine linkage, an amine linkage, an ether linkage, a thioether linkage, an alkyl linkage, a ketone linkage, and a disulfide linkage.

4. The system of claim 1, wherein the pharmaceutical composition has undesired toxicity and wherein the drug refill masks the toxicity of the pharmaceutical composition; optionally (a) wherein the drug refill masks the toxicity of the pharmaceutical composition by preventing the pharmaceutical composition from crossing the cell membrane; or (b) wherein the drug refill masks the toxicity of the pharmaceutical composition by preventing the pharmaceutical composition from binding to the biological target of the pharmaceutical composition.

5. The system of claim 2, wherein the pharmaceutical composition comprises a drug selected from the group consisting of an anti-cancer drug, a drug that promotes wound healing, a drug that promotes vascularization, a drug that treats or prevents infection, a drug that prevents restenosis, a drug that reduces macular degeneration, a drug that prevents immunological rejection, a drug that prevents thrombosis, and a drug that treats inflammation; optionally wherein the anti-cancer drug comprises doxorubicin.

6. The system of claim 1, wherein the carrier comprises a polymer, a protein, a synthetic hydrogel, a biological hydrogel, an organogel, a ceramic, a composite, a metal, a wood, or a glass material; optionally wherein the hydrogel is selected from the group consisting of collagen, alginate, polysaccharide, hyaluronic acid (HA), polyethylene glycol (PEG), poly(glycolide) (PGA), poly(L-lactide) (PLA), poly(lactide-co-glycolide) (PLGA), and poly lactic-coglycolic acid; optionally wherein the hydrogel comprises an alginate hydrogel.

7. The system of claim 1, wherein the desired location is (i) a tissue within a subject; or (ii) an organ within a subject.

8. The system of claim 1, optionally wherein the pharmaceutical composition is released by cleaving a linkage between the pharmaceutical composition and the cleavable linker, a linkage within the cleavable linker, or a linkage between the pharmaceutical composition and the target;
optionally wherein the linkage between the pharmaceutical composition and the cleavable linker, the linkage within the cleavable linker, or the linkage between the pharmaceutical composition and the target is cleaved by enzyme degradation, hydrolysis or reduction of the linkage; or
optionally wherein the linkage between the pharmaceutical composition and the cleavable linker comprises a linkage selected from the group consisting of a hydrazine linkage, a hydrazide linkage, a hydrazone linkage, an acetal linkage, a ketal linkage, an oxime linkage, an imine linkage, an amine linkage, an ether linkage, a thioether linkage, an alkyl linkage, a ketone linkage, and a disulfide linkage.

9. The system of claim 1, wherein the cleavable linker is a pH-cleavable linker.

10. The system of claim 1, wherein the target comprises a bioorthogonal functional group and the target recognition moiety comprises a complementary functional group, wherein the bioorthogonal functional group is capable of chemically reacting with the complementary functional group to form a covalent linkage; optionally wherein the bioorthogonal functional group comprises an alkyne and the complementary functional group comprises an azide, or the bioorthogonal functional group comprises an azide and the complementary functional group comprises an alkyne; optionally wherein the alkyne comprises a cyclooctyne; optionally wherein the cyclooctyne comprises dibenzocyclooctyne (DBCO).

11. A system comprising a drug delivery device and a drug refill,
wherein the drug delivery device comprises a carrier and a target recognition moiety and is suitable for implantation in a desirable location within a subject;
wherein the drug refill comprises a pharmaceutical composition and a target,
wherein the pharmaceutical composition is attached to the target via a cleavable linker capable of being cleaved within the subject;
wherein the pharmaceutical composition has undesired toxicity and the drug refill masks the toxicity of the pharmaceutical composition,
wherein the target and the target recognition moiety form a two-component binding pair;
wherein the drug refill is mobile until the target on the drug refill binds to the target recognition moiety on the drug delivery device;
wherein upon binding of the target to the target recognition moiety, the drug refill delivers the pharmaceutical composition directly to the drug delivery device;
wherein the pharmaceutical composition is released in a controlled manner from the drug delivery device to the desirable location within the subject;
optionally wherein (i) the pharmaceutical composition is released at the desirable location within the subject over a time scale of days, weeks, months or years upon cleavage of the cleavable linker; or (ii) the pharmaceutical composition is released by cleaving a linkage between the pharmaceutical composition and the cleavable linker, a linkage within the cleavable linker, or a linkage between the pharmaceutical composition and the target;
optionally wherein the linkage between the pharmaceutical composition and the cleavable linker, the linkage within the cleavable linker, or the linkage between the pharmaceutical composition and the target is cleaved by enzyme degradation, hydrolysis or reduction of the linkage;
optionally wherein the linkage between the pharmaceutical composition and the cleavable linker comprises a linkage selected from the group consisting of a hydrazine linkage, a hydrazide linkage, a hydrazone linkage, an acetal linkage, a ketal linkage, an oxime linkage, an imine linkage, an amine linkage, an ether linkage, a thioether linkage, an alkyl linkage, a ketone linkage, and a disulfide linkage.

12. The system of any one of claims 1 to 11, wherein the cleavable linker comprises a hydrazone linkage.

13. A stationary drug delivery device comprising a pharmaceutical composition, a target recognition moiety and a carrier, wherein the target recognition moiety is capable of binding to a target on a drug refill, wherein the drug refill comprises a pharmaceutical composition and a target, wherein the pharmaceutical composition is attached to the target via a cleavable linker capable of being cleaved within a subject, wherein the pharmaceutical composition is released at a desirable location within the subject in a controlled manner over a time scale of days, weeks, months, or years, upon cleavage of the cleavable linker, and wherein the drug delivery device is suitable for implantation in a desired location within a subject.

14. A drug refill comprising a pharmaceutical composition and a target,
wherein the pharmaceutical composition is attached to the target via a cleavable linker capable of being cleaved within a subject,
wherein the pharmaceutical composition has undesired toxicity and the drug refill masks the toxicity of the pharmaceutical composition,
wherein the target is capable of binding to a target recognition moiety on a drug delivery device,
wherein the drug refill is mobile until the target binds to the target recognition moiety on a drug delivery device,
wherein upon binding of the target to the target recognition moiety, the drug refill delivers the pharmaceutical composition to the drug delivery device, and the pharmaceutical composition is unmasked after delivery into the drug delivery device, and the pharmaceutical composition is released at a desirable location within the subject in a controlled manner over a time scale of days, weeks, months, or years, upon cleavage of the cleavable linker.

15. A kit for drug delivery comprising a drug delivery device and a drug refill,
wherein the drug delivery device comprises a carrier and a target recognition moiety and is suitable for implantation in a desired location within a subject;
wherein the drug refill comprises a pharmaceutical composition and a target, wherein the pharmaceutical composition is attached to the target via a cleavable linker capable of being cleaved within the subject;
wherein the pharmaceutical composition has undesired toxicity and the drug refill masks the toxicity of the pharmaceutical composition;
wherein the target and the target recognition moiety form a two-component binding pair;
wherein the drug refill is mobile until the target on the drug refill binds to the target recognition moiety on the drug delivery device; and
wherein upon binding of the target to the target recognition moiety, the drug refill delivers the pharmaceutical composition to the drug delivery device, the pharmaceutical composition is unmasked after delivery into the drug delivery device, and the pharmaceutical composition is released at a desirable location within the subject in a controlled manner over a time scale of days, weeks, months, or years, upon cleavage of the cleavable linker.

16. The system of any one of claims 1 to 11, for use in (a) maintaining or reducing the size of a tumor in a subject in need thereof or (b) treating an eye disease in a subject in need thereof, wherein
i) the drug delivery device is administered to a desired location within the subject, wherein the pharmaceutical composition (a) comprises an anti-cancer drug or (b) treats the eye disease;
ii) the drug refill is subsequently administered to the subject;
iii) the target on the drug refill is allowed to bind to the target recognition moiety on the drug delivery device to thereby deliver the pharmaceutical composition directly to the drug delivery device;
iv) the pharmaceutical composition is allowed to be released from the drug delivery device to the desired location within the subject;
v) steps ii-iv) are optionally repeated to thereby (a) maintain or reduce the size of the tumor in the subject or (b) treat the eye disease in the subject; optionally wherein (i) the desired location is (a) a tumor site or a site away from the tumor site within the subject or (b) is the eye of the subject; optionally wherein said refill is administered orally, buccally, sublingually, rectally, intravenously, intra-arterially, intraosseously, intra-muscularly, intracerebrally, intracerebroventricularly, intrathecally, subcutaneously, intraperitoneally, intraocularly, intranasally, transdermally, epidurally, intracranially, percutaneously, intravaginaly, intrauterineally, intravitreally, transmucosally, or via injection, via aerosol-based delivery, or via implantation; or (ii) the anti-cancer drug comprises doxorubicin; or (iii) the tumor comprises a solid tumor or a hematological tumor.

## Patentansprüche

1. System, welches eine Wirkstoff-Verabreichungseinrichtung und eine Wirkstoff-Auffüllung umfasst,
worin die Wirkstoff-Verabreichungseinrichtung einen Träger und eine Zielerkennungsgruppe umfasst und zur Implantation an einem gewünschten Ort in einer Person geeignet ist;
worin die Wirkstoff-Auffüllung eine pharmazeutische Zusammensetzung und ein Ziel umfasst, worin die pharmazeutische Zusammensetzung an das Ziel über eine spaltbare Verbindung angefügt ist, die geeignet ist in der Person gespalten zu werden;
worin das Ziel und die Zielerkennungsgruppe ein Zweikomponenten-Bindungspaar ausbilden;
worin die Wirkstoff-Auffüllung mobil ist, bis das Ziel auf der Wirkstoff-Auffüllung an die Zielerkennungsgruppe auf der Wirkstoff-Verabreichungseinrichtung bindet;
wobei nach Binden des Ziels an die Zielerkennungsgruppe, die Wirkstoff-Auffüllung die pharmazeutische Zusammensetzung direkt an die Wirkstoff-Verabreichungseinrichtung liefert, wodurch die Wirkstoff-Verabreichungseinrichtung aufgefüllt wird; und
wobei die pharmazeutische Zusammensetzung nach Spaltung der spaltbaren Verbindung, an dem gewünschten Ort in der Person auf eine gesteuerte Weise freigesetzt wird, über einen Zeitraum von Tagen, Wochen, Monaten, oder Jahren.

2. System nach Anspruch 1, worin die pharmazeutische Zusammensetzung eine niedermolekulare Verbindung oder ein biologisches Präparat umfasst.

3. System nach Anspruch 1, worin die pharmazeutische Zusammensetzung nach Liefern in die Wirkstoff-Verabreichungseinrichtung demaskiert wird;
wahlweise wobei die pharmazeutische Zusammensetzung durch Trennen von dem Ziel demaskiert wird;
wahlweise wobei das Ziel von der pharmazeutischen Zusammensetzung durch Spalten einer Verbindung zwischen der pharmazeutischen Zusammensetzung und der spaltbaren Verbindung, einer Verbindung in der spaltbaren Verbindung, oder einer Verbindung zwischen der pharmazeutischen Zusammensetzung und den Ziel getrennt wird;
wahlweise wobei die Verbindung zwischen der pharmazeutischen Zusammensetzung und der spaltbaren Verbindung, die Verbindung in der spaltbaren Verbindung, oder die Verbindung zwischen der pharmazeutischen Zusammensetzung und dem Ziel durch enzymatischen Abbau, Hydrolyse oder Reduktion der Verbindung gespalten wird; oder
wahlweise wobei die Verbindung zwischen der pharmazeutischen Zusammensetzung und der spaltbaren Verbindung eine Verbindung umfasst, ausgewählt aus der Gruppe bestehend aus einer Hydrazin-Verbindung, einer Hydrazid-Verbindung, einer Hydrazon-Verbindung, einer Acetal-Verbindung, einer Ketal-Verbindung, einer Oxim-Verbindung, einer Imin-Verbindung, einer Amin-Verbindung, einer Ether-Verbindung, einer Thioether-Verbindung, einer Alkyl-Verbindung, einer Keton-Verbindung, und einer Disulfid-Verbindung.

4. System nach Anspruch 1, worin die pharmazeutische Zusammensetzung unerwünschte Toxizität aufweist und worin die Wirkstoff-Auffüllung die Toxizität der pharmazeutischen Zusammensetzung maskiert;
wahlweise (a) worin die Wirkstoff-Auffüllung die Toxizität der pharmazeutischen Zusammensetzung dadurch maskiert, dass sie verhindert, dass die pharmazeutische Zusammensetzung die Zellmembran überquert; oder (b) worin der Wirkstoff-Auffüllung die Toxizität der pharmazeutischen Zusammensetzung dadurch maskiert, dass sie verhindert, dass die pharmazeutische Zusammensetzung an das biologische Ziel der pharmazeutischen Zusammensetzung bindet.

5. System nach Anspruch 2, worin die pharmazeutische Zusammensetzung einen Wirkstoff umfasst, ausgewählt aus der Gruppe bestehend aus einem Anti-Krebs-Wirkstoff, einem Wirkstoff, der Wundheilung fördert, einem Wirkstoff, der Vaskularisierung fördert, einem Wirkstoff, der eine Infektion behandelt oder dieser vorbeugt, einem Wirkstoff, der Restenose verhindert, einem Wirkstoff, der Makuladegeneration reduziert, einem Wirkstoff, der eine immunologische Abstoßung verhindert, einem Wirkstoff, der Thrombose verhindert, und einem Wirkstoff, der eine Entzündung behandelt; wahlweise worin der Anti-Krebs-Wirkstoff Doxorubicin umfasst.

6. System nach Anspruch 1, worin der Träger ein Polymer, ein Protein, ein synthetisches Hydrogel, ein biologisches Hydrogel, ein Organogel, eine Keramik, ein Komposit, ein Metall, ein Holz, oder ein Glasmaterial umfasst; wahlweise worin das Hydrogel ausgewählt ist, aus der Gruppe bestehend aus Kollagen, Alginat, Polysaccharid, Hyaluronsäure (HA), Polyethylenglykol (PEG), Polyglykolid (PGA), Poly-(L-lactid) (PIA), Polylactidcoglykolid (PLGA), und Polylactidcoglykolidsäure; wahlweise worin das Hydrogel ein Alginathydrogel umfasst.

7. System nach Anspruch 1, worin der gewünschte Ort (i) ein Gewebe in einer Person; oder (ii) ein Organ in einer Person ist.

8. System nach Anspruch 1, wahlweise wobei die pharmazeutische Zusammensetzung durch Spalten einer Verbindung zwischen der pharmazeutischen Zusammensetzung und der spaltbaren Verbindung, einer Verbindung in der spaltbaren Verbindung, oder einer Verbindung zwischen der pharmazeutischen Zusammensetzung und dem Ziel freigesetzt wird;
wahlweise wobei die Verbindung zwischen der pharmazeutischen Zusammensetzung und der spaltbaren Verbindung, der Verbindung in der spaltbaren Verbindung, oder der Verbindung zwischen der pharmazeutischen Zusammensetzung und dem Ziel durch enzymatischen Abbau, Hydrolyse oder Reduktion der Verbindung gespalten wird; oder
wahlweise wobei die Verbindung zwischen der pharmazeutischen Zusammensetzung und der spaltbaren Verbindung eine Verbindung umfasst, ausgewählt aus der Gruppe bestehend aus einer Hydrazin-Verbindung, einer Hydrazid-Verbindung, einer Hydrazon-Verbindung, einer Acetal-Verbindung, einer Ketal-Verbindung, einer Oxim-Verbindung, einer Imin-Verbindung, einer Amin-Verbindung, einer Ether-Verbindung, einer Thioether-Verbindung, einer Alkyl-Verbindung, einer Keton-Verbindung, und einer Disulfid-Verbindung.

9. System nach Anspruch 1, worin die spaltbare Verbindung eine pH-spaltbare Verbindung ist.

10. System nach Anspruch 1, worin das Ziel eine bioorthogonale funktionelle Gruppe umfasst und die Zielerkennungsgruppe eine komplementäre funktionelle Gruppe umfasst, worin die bioorthogonale funktionelle Gruppe geeignet ist, mit der komplementären funktionellen Gruppe chemisch zu reagieren, um eine kovalente Verbindung auszubilden; wahlweise worin die bioorthogonale funktionelle Gruppe ein Alkin und die komplementäre funktionelle Gruppe ein Azid umfasst, oder die bioorthogonale funktionelle Gruppe ein Azid und die komplementäre funktionelle Gruppe ein Alkin umfasst; wahlweise worin das Alkin ein Cyclooctin umfasst; wahlweise worin das Cyclooctin Dibenzocyclooctin (DBCO) umfasst.

11. System, welches eine Wirkstoff-Verabreichungseinrichtung und eine Wirkstoff-Auffüllung umfasst,
worin die Wirkstoff-Verabreichungseinrichtung einen Träger und eine Zielerkennungsgruppe umfasst und geeignet ist, an einem gewünschten Ort in einer Person implantiert zu werden;
worin die Wirkstoff-Auffüllung eine pharmazeutische Zusammensetzung und ein Ziel umfasst, worin die pharmazeutische Zusammensetzung an das Ziel über eine spaltbare Verbindung angefügt ist, die geeignet ist, in der Person gespalten zu werden;
worin die pharmazeutische Zusammensetzung unerwünschte Toxizität aufweist und die Wirkstoff-Auffüllung die Toxizität der pharmazeutischen Zusammensetzung maskiert,
worin das Ziel und die Zielerkennungsgruppe ein Zweikomponenten-Bindungspaar ausbilden;
worin die Wirkstoff-Auffüllung mobil ist, bis das Ziel auf der Wirkstoff-Auffüllung an die Zielerkennungsgruppe auf der Wirkstoff-Verabreichungseinrichtung bindet;
wobei nach Binden des Ziels an die Zielerkennungsgruppe, die Wirkstoff-Auffüllung die pharmazeutische Zusammensetzung direkt an die Wirkstoff-Verabreichungseinrichtung liefert;
wobei die pharmazeutische Zusammensetzung an dem gewünschten Ort in der Person auf eine gesteuerte Weise aus der Wirkstoff-Verabreichungseinrichtung, freigesetzt wird;
wahlweise wobei (i) die pharmazeutische Zusammensetzung an dem gewünschten Ort in der Person nach Spalten der spaltbaren Verbindung freigesetzt wird, über einen Zeitraum von Tagen, Wochen, Monaten oder Jahren; oder (ii) die pharmazeutische Zusammensetzung durch Spalten einer Verbindung zwischen der pharmazeutischen Zusammensetzung und der spaltbaren Verbindung, einer Verbindung in der spaltbaren Verbindung, oder einer Verbindung zwischen der pharmazeutischem Zusammensetzung und dem Ziel freigesetzt wird;
wahlweise wobei die Verbindung zwischen der pharmazeutischen Zusammensetzung und der spaltbaren Verbindung, der Verbindung in der spaltbaren Verbindung, oder der Verbindung zwischen der pharmazeutischen Zusammensetzung und dem Ziel durch enzymatischen Abbau, Hydrolyse oder Reduktion der Verbindung gespalten wird;
wahlweise wobei die Verbindung zwischen der pharmazeutischen Zusammensetzung und der spaltbaren Verbindung eine Verbindung umfasst, ausgewählt aus der Gruppe bestehend aus einer Hydrazin-Verbindung, einer Hydrazid-Verbindung, einer Hydrazon-Verbindung, einer Acetal-Verbindung, einer Ketal-Verbindung, einer Oxim-Verbindung, einer Imin-Verbindung, einer Amin-Verbindung, einer Ether-Verbindung, einer Thioether-Verbindung, einer Alkyl-Verbindung, einer Keton-Verbindung, und einer Disulfid-Verbindung.

12. System nach einem der Ansprüche 1 bis 11, worin die spaltbare Verbindung eine Hydrazon-Verbindung umfasst.

13. Stationäre Wirkstoff-Verabreichungseinrichtung, welche eine pharmazeutische Zusammensetzung, einer Zielerkennungsgruppe und einen Träger umfasst, worin die Zielerkennungsgruppe geeignet ist, an ein Ziel auf einer Wirkstoff-Auffüllung zu binden, worin die Wirkstoff-Auffüllung eine pharmazeutische Zusammensetzung, und ein Ziel umfasst, worin die pharmazeutische Zusammensetzung an das Ziel über eine spaltbare Verbindung angefügt ist, die geeignet ist in einer Person gespalten zu werden, worin die pharmazeutische Zusammensetzung an einem gewünschten Ort in der Person auf eine gesteuerte Weise nach Spalten der spaltbaren Verbindung freigesetzt wird, über einen Zeitraum von Tagen, Wochen, Monaten, oder Jahren, und
worin die Wirkstoff-Verabreichungseinrichtung geeignet ist, an einem gewünschten Ort in einer Person implantiert zu werden.

14. Wirkstoff-Auffüllung, welche eine pharmazeutische Zusammensetzung und ein Ziel umfasst,
worin die pharmazeutische Zusammensetzung über eine spaltbare Verbindung an das Ziel angefügt ist, die geeignet ist in einer Person gespalten zu werden,
worin die pharmazeutische Zusammensetzung unerwünschte Toxizität aufweist und die Wirkstoff-Auffüllung die Toxizität der pharmazeutischen Zusammensetzung maskiert,
worin das Ziel geeignet ist, an eine Zielerkennungsgruppe auf einer Wirkstoff-Verabreichungseinrichtung zu binden,
worin die Wirkstoff-Auffüllung mobil ist, bis das Ziel an die Zielerkennungsgruppe auf einer Wirkstoff- Verabreichungseinrichtung bindet,
wobei nach Binden des Ziels an die Zielerkennungsgruppe, die Wirkstoff-Auffüllung die pharmazeutische Zusammensetzung an die Wirkstoff-Verabreichungseinrichtung liefert, und die pharmazeutische Zusammensetzung nach dem Liefern in die Wirkstoff-Verabreichungseinrichtung demaskiert wird, und die pharmazeutische Zusammensetzung an einem gewünschten Ort in der Person auf eine gesteuerte Weise nach Spalten der spaltbaren Verbindung, über einen Zeitraum von Tagen, Wochen, Monaten, oder Jahren freigesetzt wird.

15. Kit zur Wirkstoff-Verabreichung, welches eine Wirkstoff-Verabreichungseinrichtung und eine Wirkstoff-Auffüllung umfasst,
worin die Wirkstoff-Verabreichungseinrichtung einen Träger und eine Zielerkennungsgruppe umfasst und geeignet ist, an einem gewünschten Ort in einer Person implantiert zu werden;
worin die Wirkstoff-Auffüllung eine pharmazeutische Zusammensetzung und ein Ziel umfasst, worin die pharmazeutische Zusammensetzung an das Ziel über eine spaltbare Verbindung angefügt ist, die geeignet ist, in der Person gespalten zu werden;
worin die pharmazeutische Zusammensetzung unerwünschte Toxizität aufweist und die Wirkstoff-Auffüllung die Toxizität der pharmazeutischen Zusammensetzung maskiert;
worin das Ziel und die Zielerkennungsgruppe ein Zweikomponenten-Bindungspaar ausbilden;
worin die Wirkstoff-Auffüllung mobil ist, bis das Ziel auf der Wirkstoff-Auffüllung an die Zielerkennungsgruppe auf der Wirkstoff-Verabreichungseinrichtung bindet; und
wobei nach Binden des Ziels an die Zielerkennungsgruppe, die Wirkstoff-Auffüllung die pharmazeutische Zusammensetzung an die Wirkstoff-Verabreichungseinrichtung liefert, die pharmazeutische Zusammensetzung nach dem Liefern in die Wirkstoff-Verabreichungseinrichtung demaskiert wird, und die pharmazeutische Zusammensetzung an einem gewünschten Ort in der Person auf eine gesteuerte Weise nach Spalten der spaltbaren Verbindung freigesetzt wird, über einen Zeitraum von Tagen, Wochen, Monaten, oder Jahren.

16. System nach einem der Ansprüche 1 bis 11, zur Verwendung beim (a) Erhalten oder Reduzieren der Größe eines Tumors in einer Person, die dessen bedarf oder (b) bei der Behandlung einer Augenerkrankung in einer Person, die dessen bedarf, worin
i) die Wirkstoff-Verabreichungseinrichtung an einem gewünschten Ort in der Person verabreicht wird, worin die pharmazeutische Zusammensetzung (a) einen Anti-Krebs-Wirkstoff umfasst oder (b) die Augenerkrankung behandelt;
ii) die Wirkstoff-Auffüllung hernach an die Person verabreicht wird;
iii) das Ziel auf der Wirkstoff-Auffüllung an die Zielerkennungsgruppe auf der Wirkstoff-Verabreichungseinrichtung binden kann, um dadurch die pharmazeutische Zusammensetzung direkt an die Wirkstoff-Verabreichungseinrichtung zu liefern;
iv) die pharmazeutische Zusammensetzung aus der Wirkstoff-Verabreichungseinrichtung an dem gewünschten Ort in der Person freigesetzt werden kann;
v) Schritte ii-iv) wahlweise wiederholt werden, um dadurch (a) die Größe des Tumors in der Person zu erhalten oder zu reduzieren oder (b) die Augenkrankheit in der Person zu behandeln; wahlweise worin (i) der gewünschte Ort (a) eine Tumorstelle oder eine Stelle abseits der Tumorstelle in der Person ist oder (b) das Auge der Person ist; wahlweise wobei die Auffüllung oral, bukkal, sublingual, rektal, intravenös, intraarteriell, intraossär, intramuskulär, intracerebral, intracerebroventrikulär, intrathekal, subkutan, intraperitoneal, intraokular, intranasal, transdermal, epidural, intracranial, perkutan, intravaginal, intrauterin, intravitreal, transmukosal, oder über Injektion, über aerosol-basierte Verabreichung, oder über Implantation verabreicht wird; oder (ii) der Anti-Krebs-Wirkstoff Doxorubicin umfasst; oder (iii) der Tumor einen soliden Tumor oder einen hämatologischen Tumor umfasst.

## Revendications

1. Système comprenant un dispositif d'administration de médicament et une recharge de médicament,
dans lequel le dispositif d'administration de médicament comprend un support et une fraction de reconnaissance de cible et est approprié pour une implantation dans un emplacement souhaitable à l'intérieur d'un sujet ;
dans lequel la recharge de médicament comprend une composition pharmaceutique et une cible, la composition pharmaceutique étant attachée à la cible par l'intermédiaire d'un lieur clivable capable d'être clivé à l'intérieur du sujet ;
dans lequel la cible et la fraction de reconnaissance de cible forment une paire de liaison à deux composants ;
dans lequel la recharge de médicament est mobile jusqu'à ce que la cible sur la recharge de médicament se lie à la fraction de reconnaissance de cible sur le dispositif d'administration de médicament ;
dans lequel, lors de la liaison de la cible à la fraction de reconnaissance de cible, la recharge de médicament délivre la composition pharmaceutique directement au dispositif d'administration de médicament, rechargeant ainsi le dispositif d'administration de médicament ; et
dans lequel la composition pharmaceutique est libérée à l'emplacement souhaitable à l'intérieur du sujet d'une manière contrôlée sur une échelle de temps de jours, semaines, mois ou années, lors du clivage du lieur clivable.

2. Système selon la revendication 1, dans lequel la composition pharmaceutique comprend une petite molécule ou un produit biologique.

3. Système selon la revendication 1, dans lequel la composition pharmaceutique est démasquée après délivrance dans le dispositif d'administration de médicament ; facultativement dans lequel
la composition pharmaceutique est démasquée en la séparant de la cible ; facultativement dans lequel
la cible est séparée de la composition pharmaceutique par clivage d'une liaison entre la composition pharmaceutique et le lieur clivable, une liaison à l'intérieur du lieur clivable ou une liaison entre la composition pharmaceutique et la cible ; facultativement dans lequel
la liaison entre la composition pharmaceutique et le lieur clivable, la liaison à l'intérieur du lieur clivable ou la liaison entre la composition pharmaceutique et la cible est clivée par dégradation enzymatique, hydrolyse ou réduction de la liaison ; ou facultativement dans lequel
la liaison entre la composition pharmaceutique et le lieur clivable comprend une liaison choisie dans le groupe consistant en une liaison hydrazine, une liaison hydrazide, une liaison hydrazone, une liaison acétal, une liaison cétal, une liaison oxime, une liaison imine, une liaison amine, une liaison éther, une liaison thioéther, une liaison alkyle, une liaison cétone et une liaison disulfure.

4. Système selon la revendication 1, dans lequel la composition pharmaceutique a une toxicité indésirable et dans lequel la recharge de médicament masque la toxicité de la composition pharmaceutique ; facultativement (a) dans lequel la recharge de médicament masque la toxicité de la composition pharmaceutique en empêchant la composition pharmaceutique de traverser la membrane cellulaire ; ou (b) dans lequel la recharge de médicament masque la toxicité de la composition pharmaceutique en empêchant la composition pharmaceutique de se lier à la cible biologique de la composition pharmaceutique.

5. Système selon la revendication 2, dans lequel la composition pharmaceutique comprend un médicament choisi dans le groupe consistant en un médicament anticancéreux, un médicament qui favorise la cicatrisation des plaies, un médicament qui favorise la vascularisation, un médicament qui traite ou prévient l'infection, un médicament qui prévient la resténose, un médicament qui réduit la dégénérescence maculaire, un médicament qui prévient le rejet immunologique, un médicament qui prévient la thrombose et un médicament qui traite l'inflammation ; facultativement dans lequel le médicament anticancéreux comprend la doxorubicine.

6. Système selon la revendication 1, dans lequel le support comprend un polymère, une protéine, un hydrogel synthétique, un hydrogel biologique, un organogel, une céramique, un composite, un métal, un bois ou un matériau vitreux ; facultativement dans lequel l'hydrogel est choisi dans le groupe consistant en le collagène, un alginate, un polysaccharide, l'acide hyaluronique (HA), le polyéthylène glycol (PEG), le poly(glycolide) (PGA), le poly (L-lactide) (PLA), le poly(lactide-co-glycolide) (PLGA) et l'acide poly lactique-co-glycolique ; facultativement dans lequel l'hydrogel comprend un hydrogel alginate.

7. Système selon la revendication 1, dans lequel l'emplacement souhaité est (i) un tissu à l'intérieur d'un sujet ; ou (ii) un organe à l'intérieur d'un sujet.

8. Système selon la revendication 1, facultativement dans lequel la composition pharmaceutique est libérée par clivage d'une liaison entre la composition pharmaceutique et le lieur clivable, une liaison à l'intérieur du lieur clivable, ou une liaison entre la composition pharmaceutique et la cible ;
facultativement dans lequel la liaison entre la composition pharmaceutique et le lieur clivable, la liaison à l'intérieur du lieur clivable, ou la liaison entre la composition pharmaceutique et la cible est clivée par dégradation enzymatique, hydrolyse ou réduction de la liaison ; ou
facultativement dans lequel la liaison entre la composition pharmaceutique et le lieur clivable comprend une liaison choisie dans le groupe consistant en une liaison hydrazine, une liaison hydrazide, une liaison hydrazone, une liaison acétal, une liaison cétal, une liaison oxime, une liaison imine, une liaison amine, une liaison éther, une liaison thioéther, une liaison alkyle, une liaison cétone et une liaison disulfure.

9. Système selon la revendication 1, dans lequel le lieur clivable est un lieur clivable en fonction du pH.

10. Système selon la revendication 1, dans lequel la cible comprend un groupe fonctionnel bioorthogonal et la fraction de reconnaissance de cible comprend un groupe fonctionnel complémentaire, dans lequel le groupe fonctionnel bioorthogonal est capable de réagir chimiquement avec le groupe fonctionnel complémentaire pour former une liaison covalente ; facultativement dans lequel le groupe fonctionnel bioorthogonal comprend un alcyne et le groupe fonctionnel complémentaire comprend un azide, ou le groupe fonctionnel bioorthogonal comprend un azide et le groupe fonctionnel complémentaire comprend un alcyne ; facultativement dans lequel l'alcyne comprend un cyclooctyne ; facultativement dans lequel le cyclooctyne comprend le dibenzocyclooctyne (DBCO).

11. Système comprenant un dispositif d'administration de médicament et une recharge de médicament,
dans lequel le dispositif d'administration de médicament comprend un support et une fraction de reconnaissance de cible et est approprié pour une implantation dans un emplacement souhaitable à l'intérieur d'un sujet ;
dans lequel la recharge de médicament comprend une composition pharmaceutique et une cible,
dans lequel la composition pharmaceutique est attachée à la cible par l'intermédiaire d'un lieur clivable capable d'être clivé à l'intérieur du sujet ;
dans lequel la composition pharmaceutique a une toxicité indésirable et la recharge de médicament masque la toxicité de la composition pharmaceutique,
dans lequel la cible et la fraction de reconnaissance de cible forment une paire de liaison à deux composants ;
dans lequel la recharge de médicament est mobile jusqu'à ce que la cible sur la recharge de médicament se lie à la fraction de reconnaissance de cible sur le dispositif d'administration de médicament ;
dans lequel, lors de la liaison de la cible à la fraction de reconnaissance de cible, la recharge de médicament délivre la composition pharmaceutique directement au dispositif d'administration de médicament ;
dans lequel la composition pharmaceutique est libérée d'une manière contrôlée du dispositif d'administration de médicament à l'emplacement souhaitable à l'intérieur du sujet ;
facultativement dans lequel (i) la composition pharmaceutique est libérée à l'emplacement souhaitable à l'intérieur du sujet sur une échelle de temps de jours, semaines, mois ou années lors du clivage du lieur clivable ; ou (ii) la composition pharmaceutique est libérée par clivage d'une liaison entre la composition pharmaceutique et le lieur clivable, une liaison à l'intérieur du lieur clivable, ou une liaison entre la composition pharmaceutique et la cible ;
facultativement dans lequel la liaison entre la composition pharmaceutique et le lieur clivable, la liaison à l'intérieur du lieur clivable, ou la liaison entre la composition pharmaceutique et la cible est clivée par dégradation enzymatique, hydrolyse ou réduction de la liaison ;
facultativement dans lequel la liaison entre la composition pharmaceutique et le lieur clivable comprend une liaison choisie dans le groupe consistant en une liaison hydrazine, une liaison hydrazide, une liaison hydrazone, une liaison acétal, une liaison cétal, une liaison oxime, une liaison imine, une liaison amine, une liaison éther, une liaison thioéther, une liaison alkyle, une liaison cétone et une liaison disulfure.

12. Système selon l'une quelconque des revendications 1 à 11, dans lequel le lieur clivable comprend une liaison hydrazone.

13. Dispositif stationnaire d'administration de médicament comprenant une composition pharmaceutique, une fraction de reconnaissance de cible et un support, dans lequel la fraction de reconnaissance de cible est capable de se lier à une cible sur une recharge de médicament, dans lequel la recharge de médicament comprend une composition pharmaceutique et une cible, dans lequel la composition pharmaceutique est attachée à la cible par l'intermédiaire d'un lieur clivable capable d'être clivé à l'intérieur d'un sujet, dans lequel la composition pharmaceutique est libérée à un emplacement souhaitable à l'intérieur du sujet d'une manière contrôlée sur une échelle de temps de jours, semaines, mois ou années, lors du clivage du lieur clivable, et dans lequel le dispositif d'administration de médicament est approprié pour une implantation dans un emplacement souhaité à l'intérieur d'un sujet.

14. Recharge de médicament comprenant une composition pharmaceutique et une cible,
dans laquelle la composition pharmaceutique est attachée à la cible par l'intermédiaire d'un lieur clivable capable d'être clivé à l'intérieur d'un sujet,
dans laquelle la composition pharmaceutique a une toxicité indésirable et la recharge de médicament masque la toxicité de la composition pharmaceutique,
dans laquelle la cible est capable de se lier à une fraction de reconnaissance de cible sur un dispositif d'administration de médicament,
dans lequel la recharge de médicament est mobile jusqu'à ce que la cible se lie à la fraction de reconnaissance de cible sur un dispositif d'administration de médicament,
dans lequel, lors de la liaison de la cible à la fraction de reconnaissance de cible, la recharge de médicament délivre la composition pharmaceutique au dispositif d'administration de médicament, et la composition pharmaceutique est démasquée après délivrance dans le dispositif d'administration de médicament, et la composition pharmaceutique est libérée à un emplacement souhaitable à l'intérieur du sujet d'une manière contrôlée sur une échelle de temps de jours, semaines, mois ou années, lors du clivage du lieur clivable.

15. Kit pour l'administration de médicament comprenant un dispositif d'administration de médicament et une recharge de médicament,
dans lequel le dispositif d'administration de médicament comprend un support et une fraction de reconnaissance de cible et est approprié pour une implantation dans un emplacement souhaité à l'intérieur d'un sujet ;
dans lequel la recharge de médicament comprend une composition pharmaceutique et une cible,
dans laquelle la composition pharmaceutique est attachée à la cible par l'intermédiaire d'un lieur clivable capable d'être clivé à l'intérieur du sujet ;
dans lequel la composition pharmaceutique a une toxicité indésirable et la recharge de médicament masque la toxicité de la composition pharmaceutique ;
dans lequel la cible et la fraction de reconnaissance de cible forment une paire de liaison à deux composants ;
dans lequel la recharge de médicament est mobile jusqu'à ce que la cible sur la recharge de médicament se lie à la fraction de reconnaissance de cible sur le dispositif d'administration de médicament ; et
dans lequel, lors de la liaison de la cible à la fraction de reconnaissance de cible, la recharge de médicament délivre la composition pharmaceutique au dispositif d'administration de médicament, la composition pharmaceutique est démasquée après délivrance dans le dispositif d'administration de médicament, et la composition pharmaceutique est libérée à un emplacement souhaitable à l'intérieur du sujet d'une manière contrôlée sur une échelle de temps de jours, semaines, mois ou années, lors du clivage du lieur clivable.

16. Système selon l'une quelconque des revendications 1 à 11, pour utilisation dans (a) le maintien ou la réduction de la taille d'une tumeur chez un sujet en ayant besoin ou (b) le traitement d'une maladie oculaire chez un sujet en ayant besoin, dans lequel :
i) le dispositif d'administration de médicament est administré à un emplacement souhaité à l'intérieur du sujet, la composition pharmaceutique (a) comprenant un médicament anticancéreux ou (b) traitant la maladie oculaire ;
ii) la recharge de médicament est par la suite administrée au sujet ;
iii) la cible sur la recharge de médicament est amenée à se lier à la fraction de reconnaissance de cible sur le dispositif d'administration de médicament pour délivrer ainsi la composition pharmaceutique directement au dispositif d'administration de médicament ;
iv) la composition pharmaceutique est amenée à être libérée du dispositif d'administration de médicament à l'emplacement souhaité à l'intérieur du sujet ;
v) les étapes ii à iv) sont facultativement répétées pour ainsi (a) maintenir ou réduire ainsi la taille de la tumeur à l'intérieur du sujet ou (b) traiter la maladie oculaire dans le sujet ; facultativement dans lequel (i) l'emplacement souhaité est (a) un site tumoral ou un site éloigné du site tumoral à l'intérieur du sujet ou (b) est l'œil du sujet ; facultativement dans lequel ladite recharge est administrée par voie orale, buccale, sublinguale, rectale, intraveineuse, intra-artérielle, intra-osseuse, intramusculaire, intracérébrale, intracérébroventriculaire, intrathécale, sous-cutanée, intrapéritonéale, intra-oculaire, intranasale, transdermique, épidurale, intracrânienne, percutanée, intravaginale, intra-utérine, intravitréenne, transmuqueuse ou par injection, par administration à base d'aérosol, ou par implantation ; ou (ii) le médicament anticancéreux comprend la doxorubicine ; ou (iii) la tumeur comprend une tumeur solide ou une tumeur hématologique.


### SEQUENCE LISTING

<110> BRUDNO, YEVGENY
   KEARNEY, CATHAL J.
   SILVA, EDUARDO
   AIZENBERG, MICHAEL
   KWEE, BRIAN
   DESAI, RAJIV
   JOSHI, NEEL S.
   MOONEY, DAVID J.
<120> REFILLABLE DRUG DELIVERY DEVICES AND METHODS OF USE THEREOF
<130> 117823-09303
<140> 14/878,578
   <141> 2015-10-08
<150> PCT/US2015/024540
   <151> 2015-04-06
<150> 62/085,898
   <151> 2014-12-01
<150> 61/975,443
   <151> 2014-04-04
<160> 26
<170> PatentIn version 3.5
<210> 1
   <211> 20
   <212> DNA
   <213> Artificial Sequence
<220>
   <223> Description of Artificial Sequence: Synthetic oligonucleotide
<400> 1
   tttttttttt tttttttttt 20
<210> 2
   <211> 20
   <212> DNA
   <213> Artificial Sequence
<220>
   <223> Description of Artificial Sequence: Synthetic oligonucleotide
<400> 2
   aaaaaaaaaa aaaaaaaaaa 20
<210> 3
   <211> 20
   <212> DNA
   <213> Artificial Sequence
<220>
   <223> Description of Artificial Sequence: Synthetic oligonucleotide
<220>
   <223> 3' thiol
<400> 3
   tttttttttt tttttttttt 20
<210> 4
   <211> 20
   <212> DNA
   <213> Artificial Sequence
<220>
   <223> Description of Artificial Sequence: Synthetic oligonucleotide
<220>
   <223> 3' thiol
<400> 4
   aaaaaaaaaa aaaaaaaaaa 20
<210> 5
   <211> 20
   <212> DNA
   <213> Artificial Sequence
<220>
   <223> Description of Artificial Sequence: Synthetic oligonucleotide
<220>
   <223> 5' 6-fluorescein
<400> 5
   tttttttttt tttttttttt 20
<210> 6
   <211> 20
   <212> DNA
   <213> Artificial Sequence
<220>
   <223> Description of Artificial Sequence: Synthetic oligonucleotide
<220>
   <223> 5' 6-fluorescein
<400> 6
   aaaaaaaaaa aaaaaaaaaa 20
<210> 7
   <211> 20
   <212> DNA
   <213> Artificial Sequence
<220>
   <223> Description of Artificial Sequence: Synthetic oligonucleotide
<220>
   <223> 5' Hexachlorofluorescein
<220>
   <223> 3' thiol
<400> 7
   tttttttttt tttttttttt 20
<210> 8
   <211> 20
   <212> DNA
   <213> Artificial Sequence
<220>
   <223> Description of Artificial Sequence: Synthetic oligonucleotide
<220>
   <221> misc_feature
   <222> (1)..(20)
   <223> Phosphorothioate linkage
<220>
   <223> 3' thiol
<400> 8
   tttttttttt tttttttttt 20
<210> 9
   <211> 20
   <212> DNA
   <213> Artificial Sequence
<220>
   <223> Description of Artificial Sequence: Synthetic oligonucleotide
<220>
   <221> misc_feature
   <222> (1)..(20)
   <223> Phosphorothioate linkage
<220>
   <223> 3' thiol
<400> 9
   aaaaaaaaaa aaaaaaaaaa 20
<210> 10
   <211> 22
   <212> DNA
   <213> Artificial Sequence
<220>
   <223> Description of Artificial Sequence: Synthetic oligonucleotide
<400> 10
   catggagagc gacgagagcg gc 22
<210> 11
   <211> 22
   <212> DNA
   <213> Artificial Sequence
<220>
   <223> Description of Artificial Sequence: Synthetic oligonucleotide
<400> 11
   gccgctctcg tcgctctcca tg 22
<210> 12
   <211> 18
   <212> DNA
   <213> Artificial Sequence
<220>
   <223> Description of Artificial Sequence: Synthetic oligonucleotide
<400> 12
   tgtaactgag gtaagagg 18
<210> 13
   <211> 18
   <212> DNA
   <213> Artificial Sequence
<220>
   <223> Description of Artificial Sequence: Synthetic oligonucleotide
<400> 13
   cctcttacct cagttaca 18
<210> 14
   <211> 20
   <212> DNA
   <213> Artificial Sequence
<220>
   <223> Description of Artificial Sequence: Synthetic oligonucleotide
<400> 14
   cccccccccc cccccccccc 20
<210> 15
   <211> 20
   <212> DNA
   <213> Artificial Sequence
<220>
   <223> Description of Artificial Sequence: Synthetic oligonucleotide
<400> 15
   gggggggggg gggggggggg 20
<210> 16
   <211> 24
   <212> DNA
   <213> Artificial Sequence
<220>
   <223> Description of Artificial Sequence: Synthetic oligonucleotide
<400> 16
   acacacacac acacacacac acac 24
<210> 17
   <211> 24
   <212> DNA
   <213> Artificial Sequence
<220>
   <223> Description of Artificial Sequence: Synthetic oligonucleotide
<400> 17
   gtgtgtgtgt gtgtgtgtgt gtgt 24
<210> 18
   <211> 24
   <212> DNA
   <213> Artificial Sequence
<220>
   <223> Description of Artificial Sequence: Synthetic oligonucleotide
<400> 18
   gaatgaatga atgaatgaat gaat 24
<210> 19
   <211> 32
   <212> DNA
   <213> Artificial Sequence
<220>
   <223> Description of Artificial Sequence: Synthetic oligonucleotide
<400> 19
   attcattcat tcattcattc attcattcat tc 32
<210> 20
   <211> 28
   <212> DNA
   <213> Artificial Sequence
<220>
   <223> Description of Artificial Sequence: Synthetic oligonucleotide
<400> 20
   ggattggatt gattggattg attggatt 28
<210> 21
   <211> 30
   <212> DNA
   <213> Artificial Sequence
<220>
   <223> Description of Artificial Sequence: Synthetic oligonucleotide
<400> 21
   aatccaatcc aatccaatcc aatccaatcc 30
<210> 22
   <211> 17
   <212> DNA
   <213> Artificial Sequence
<220>
   <223> Description of Artificial Sequence: Synthetic oligonucleotide
<400> 22
   gtaaaacgac ggccagt 17
<210> 23
   <211> 17
   <212> DNA
   <213> Artificial Sequence
<220>
   <223> Description of Artificial Sequence: Synthetic oligonucleotide
<400> 23
   actggccgtc gttttac 17
<210> 24
   <211> 19
   <212> DNA
   <213> Artificial Sequence
<220>
   <223> Description of Artificial Sequence: Synthetic oligonucleotide
<400> 24
   gctagttatt gctcagcgg 19
<210> 25
   <211> 19
   <212> DNA
   <213> Artificial Sequence
<220>
   <223> Description of Artificial Sequence: Synthetic oligonucleotide
<400> 25
   ccgctgagca ataactagc 19
<210> 26
   <211> 9
   <212> PRT
   <213> Artificial Sequence
<220>
   <223> Description of Artificial Sequence: Synthetic peptide
<400> 26
   Gly Gly Gly Gly Arg Gly Asp Ser Pro
   1               5